(19) Europäisches Patentamt / European Patent Office / Office européen des brevets

(11) EP 4 242 219 A1

(12) **EUROPEAN PATENT APPLICATION**
published in accordance with Art. 153(4) EPC

(43) Date of publication:
**13.09.2023 Bulletin 2023/37**

(21) Application number: **21889093.7**

(22) Date of filing: **27.10.2021**

(51) International Patent Classification (IPC):
***C07K 1/06*** *(2006.01)* ***C40B 50/14*** *(2006.01)*

(52) Cooperative Patent Classification (CPC):
**C07K 1/06; C40B 50/14;** Y02P 20/55

(86) International application number:
**PCT/JP2021/039586**

(87) International publication number:
**WO 2022/097540 (12.05.2022 Gazette 2022/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.11.2020 JP 2020184839**

(71) Applicant: **CHUGAI SEIYAKU KABUSHIKI KAISHA**
**Tokyo 115-8543 (JP)**

(72) Inventors:
- **NOMURA, Kenichi**
  **Gotemba-shi, Shizuoka 412-8513 (JP)**
- **KAGE, Mirai**
  **Gotemba-shi, Shizuoka 412-8513 (JP)**
- **TAMIYA, Minoru**
  **Kamakura-shi, Kanagawa 247-8530 (JP)**
- **KANAZAWA, Junichiro**
  **Gotemba-shi, Shizuoka 412-8513 (JP)**

(74) Representative: **Vossius & Partner**
**Patentanwälte Rechtsanwälte mbB**
**Siebertstraße 3**
**81675 München (DE)**

Remarks:
The complete document including Reference Table(s) and the Sequence Listing(s) can be downloaded from the EPO website

# (54) PEPTIDE SYNTHESIS METHOD FOR SUPPRESSING DEFECT CAUSED BY DIKETOPIPERAZINE FORMATION

(57) Solid-phase synthesis of a peptide has a problem that a desired elongation reaction is prevented from proceeding by diketopiperazine and a 6-membered diamine skeleton compound formed when a protective group at the N-terminal is removed. The present inventors have found that when in production of a peptide by a solid-phase method, a peptide in which an amino group at the N-terminal is protected with a protective group having an Fmoc skeleton is treated in a specific solvent with a base having a pKa of 23 or more in acetonitrile as a conjugate acid, and a peptide chain is then elongated, it is possible to solve the problem described above.

EP 4 242 219 A1

## Description

### Technical Field

The present invention relates to a novel peptide synthesis method capable of synthesizing a peptide with a high purity and high efficiency in peptide synthesis by a solid-phase method.

### Background Art

In peptide synthesis, the Fmoc method is a method which is widely applied because of its reliability and gentle deprotection conditions (Fmoc deprotection conditions). In recent years, studies have been actively conducted on application of peptides containing an abundance of N-alkylamino acids such as N-methylamino acids to medicaments, and such molecular species are known to have acid lability (Non Patent Literature 1 and Patent Literature 1). Thus, in these documents, the Fmoc method which enables deprotection under basic conditions, rather than the Boc method which requires acidic conditions, is adopted for deprotection at the N-terminal.

Diketopiperazine (DKP) formation in peptide synthesis is a problem that has been recognized since a long time ago. Diketopiperazine formation may occur if a protective group at the N-terminal of a dipeptide supported on a solid phase through an ester bond, so that a free amino group is exposed. Some approaches have been reported for improving the problem of diketopiperazine formation in peptide synthesis using the Fmoc method.

Specifically, a method is known in which as a protective group at a position where diketopiperazine formation is likely to occur, an Alloc group in which a deprotection reaction proceeds under neutral conditions is used instead of a base to suppress diketopiperazine formation (Non Patent Literature 2).

In addition, a method is known in which for avoiding a situation in which the N-terminal is formed by a free amino group at a position where diketopiperazine formation is likely to proceed, a dipeptide containing such a sequence is synthesized beforehand, and used to elongate a peptide chain (Non Patent Literature 3).

Further, a method is known in which by using DBU or TBAF that is a stronger base, instead of piperidine that is commonly used in deprotection of Fmoc, the time required for deprotection is made extremely short to suppress diketopiperazine formation (Non Patent Literature 4).

All of these methods are countermeasures against diketopiperazine formation in a dipeptide supported on a solid phase through an ester bond.

### Citation List

### Patent Literature

Patent Literature 1: International Publication No. WO 2018/225851 A1

### Non Patent Literature

Non Patent Literature 1: J. Peptide Res., 2005, 65, 153-166.
Non Patent Literature 2: Org. Lett., 2012, 14, 612-615.
Non Patent Literature 3: Solid Phase Peptide Synthesis (https://www.bachem.com/fileadmin/user_upload/pdf/Catalogs_Brochures/Solid_Phase_Peptide _Synthesis.pdf) published by Bachem
Non Patent Literature 4: Org. Lett., 2008, 10, 3857-3860.

### Summary of Invention

### Technical Problem

It has been known that in a dipeptide supported on a solid phase through an ester bond, the ester bond is cleaved to form diketopiperazine as described above, whereas when the peptide sequence includes a N-substituted amino acid, an amide bond stronger than an ester bond may be cleaved to form diketopiperazine, resulting in missing of a dipeptide containing a N-substituted amino acid from the peptide sequence. It has come to be known that since such missing may occur at any place where a N-substituted amino acid is present in the peptide sequence, the dipeptide that may miss is not limited to one supported on a solid phase.

Further, the present inventors have revealed that when the peptide sequence includes a N-substituted amino

acid, there is not only the problem of diketopiperazine formation, but also a problem that a desired elongation reaction does not proceed, and instead of a target product, an impurity having a 6-membered cyclic amidine skeleton (6-membered cyclic amidine skeleton compound) is formed at the N-terminal. A method for solving these problems have not been heretofore reported.

The above-described conventional techniques may be applied for suppressing not only diketopiperazine elimination which proceeds in an ester bond of a dipeptide supported on a solid phase, but also diketopiperazine elimination which proceeds an amide bond, and formation of the 6-membered cyclic amidine skeleton compound. However, there is a limitation on the range of application thereof, and these techniques cannot provide a sufficient solution.

For example, the method described in Non Patent Literature 2 requires an Alloc amino acid that is less universal than an Fmoc amino acid in terms of availability.

In the method for elongating a peptide chain using a dipeptide as described in Non Patent Literature 3, racemization of an amino acid of the dipeptide on the C-terminal side may proceed. Thus, the method described in this document is difficult to apply unless the amino acid of a dipeptide fragment on the C-terminal side is achiral (e.g. in the case of glycine) or racemization is unlikely to proceed (e.g. in the case of proline).

Further, the method described in Non Patent Literature 4 is characterized in that the time required for an Fmoc deprotection step is made very short, and this method requires an Fmoc deprotection step and a solid washing operation in a minute or less, e.g. 10 to 20 seconds. It is impossible to apply such an extremely short-time operation to scale-up synthesis for industrialization etc.

As described above, a practical methodology for suppression of diketopiperazine has never been presented. The present invention has been made in view of these circumstances, and in an aspect, an object of the present invention is to provide a peptide synthesis method capable of suppressing formation of diketopiperazine, in which (i) a protective group containing an Fmoc skeleton is used as a protective group at the N-terminal, (ii) application of sequences is not limited by racemization and (iii) scale-up synthesis for industrialization etc. is possible. In another aspect, an object of the present invention is to provide a peptide synthesis method capable of suppressing formation of a 6-membered cyclic amidine skeleton compound.

## Solution to Problem

The present inventors have found that when in production of a peptide by a solid-phase method, a peptide having a protective group containing an Fmoc skeleton is treated in a specific solvent using a base having a pKa of 23 or more in acetonitrile as a conjugate acid, and subsequently, an elongated active species such an active ester generated using an acid chloride and a condensation agent is reacted with the peptide, it is possible to suppress formation of diketopiperazine and a 6-membered cyclic amidine skeleton compound until elongation of a peptide chain from removal of the protective group. In this way, the present invention has been completed.

That is, the present invention includes the following.

[1] A method for producing a peptide by a solid-phase method, comprising the steps of:

(1) providing a first peptide having a protective group containing an Fmoc skeleton and supported on a solid-phase synthesis resin;
(2) treating the first peptide with one or more bases including at least a base whose conjugate acid has a pKa of 23 or more in acetonitrile in a solvent containing at least one selected from the group consisting of an aromatic hydrocarbon solvent, a halogen solvent, an ether solvent, an ester solvent, a ketone solvent, a carbonate solvent and a phosphoric acid ester solvent after the step (1); and
(3) condensing the first peptide with a carboxylic acid or a carboxylic acid analog in a solvent in the presence or absence of a condensation agent to obtain a third peptide after the step (2).

[2] The method according to [1], which does not comprise the step of treating the first peptide with piperidine as a single base before the step (2).
[3] The method according to [1], which does not comprise the step of treating the first peptide with a single base having a pKa of less than 23 in acetonitrile as a conjugate acid before the step (2).
[4] The method according to any one of [1] to [3], which does not comprise the step of neutralizing the residual base by adding an acid between the step (2) and the step (3).
[5] The method according to any one of [1] to [4], wherein the solvent in the step (2) is a solvent having a solid-phase resin swelling capacity of 1.5 mL/g or more, 2.0 mL/g or more, 2.5 mL/g or more, 3.0 mL/g or more, 3.5 mL/g or more, or 4.0 mL/g or more.
[6] The method according to any one of [1] to [5], wherein at least a part of the first peptide obtained from the step (2) is in the form of a carbamic acid salt.

[7] The method according to [6], wherein the carbamic acid salt is a salt with a base having a pKa of 23 or more in acetonitrile as a conjugate acid.

[8] The method according to [6] or [7], wherein the carbamic acid salt is a DBU salt, a TMG salt, a HP1 (dma) salt, a MTBD salt, a P1-tBu salt or a P2-Et salt.

[9] The method according to any one of [6] to [8], wherein in the step (2), a molar ratio of the carbamic acid salt to an amine form additionally formed with progress of decarboxylation (carbamic acid salt/amine form), which is determined from an integral ratio of protons in $^1$H-NMR, is 0.6 or more, 0.8 or more, 1.0 or more, 2.0 or more, 3.0 or more, 4.0 or more, 4.6 or more, 5.0 or more, 6.0 or more, 8.0 or more, or 10.0 or more.

[10] The method according to any one of [1] to [9], wherein the solvent in the step (2) contains at least one selected from the group consisting of an aromatic hydrocarbon solvent, a halogen solvent, an ether solvent, an ester solvent, a ketone solvent, a carbonate solvent and a phosphoric acid ester solvent at 25 v/v% or more.

[11] The method according to any one of [1] to [10], wherein the solvent in the step (2) contains at least one selected from the group consisting of an aromatic hydrocarbon solvent, a halogen solvent, an ether solvent, an ester solvent, a ketone solvent, a carbonate solvent and a phosphoric acid ester solvent at 50 v/v% or more.

[12] The method according to any one of [1] to [11], wherein the solvent in the step (2) contains at least one selected from the group consisting of an aromatic hydrocarbon solvent, a halogen solvent, an ether solvent, an ester solvent, a ketone solvent, a carbonate solvent and a phosphoric acid ester solvent at 75 v/v% or more.

[13] The method according to any one of [1] to [12], wherein the solvent in the step (2) contains one or more selected from the group consisting of an amide solvent, a urea solvent and a sulfone solvent.

[14] The method according to any one of [1] to [12], wherein the solvent in the step (2) includes at least one selected from the group consisting of an aromatic hydrocarbon solvent, a halogen solvent, an ether solvent, an ester solvent, a ketone solvent, a carbonate solvent and a phosphoric acid ester solvent.

[15] The method according to any one of [1] to [14], wherein the aromatic hydrocarbon solvent is one or more selected from the group consisting of toluene, benzene, xylene, chlorobenzene, 1,2-dichlorobenzene, bromobenzene, anisole, ethylbenzene, nitrobenzene and cumene,

the halogen solvent is one or more selected from the group consisting of dichloromethane, chloroform, 1,2-dichloroethane and carbon tetrachloride,
the ether solvent is one or more selected from the group consisting of tetrahydrofuran, diethyl ether, 2-methyltetrahydrofuran, 1,4-dioxane, 1,2-dimethoxyethane, 1,3-dioxolane, diisopropyl ether, cyclopentyl methyl ether, t-butyl methyl ether, 4-methyltetrahydropyran, diglyme, triglyme and tetraglyme,
the ester solvent is one or more selected from the group consisting of methyl acetate, ethyl acetate, butyl acetate, methyl propionate, propyl acetate, isopropyl acetate, isobutyl acetate, pentyl acetate and $\gamma$-valerolactone,
the ketone solvent is one or more selected from the group consisting of acetone, methyl ethyl ketone, methyl isobutyl ketone, cyclohexanone, cyclopentanone and diethyl ketone,
the carbonate solvent is one or more selected from the group consisting of dimethyl carbonate, diethyl carbonate and dibutyl carbonate, and
the phosphoric acid ester solvent is one or more selected from the group consisting of trimethyl phosphate, triethyl phosphate and tributyl phosphate.

[16] The method according to any one of [1] to [15], wherein the solvent in the step (2) has a donor number value of 26 or less.

[16.1] The method according to any one of [1] to [16], wherein the aromatic hydrocarbon solvent, halogen solvent, ether solvent, ester solvent, ketone solvent, carbonate solvent or phosphoric acid ester solvent in the step (2) has a donor number value of 26 or less.

[17] The method according to any one of [1] to [16], wherein the solvent in the step (2) has a donor number value of 25 or less, 24 or less, 23 or less, 22 or less, 21 or less, 20 or less, 19 or less, 18 or less, 17 or less, 16 or less, 15 or less, 14 or less, 13 or less, 12 or less, 11 or less, 10 or less, 9 or less, 8 or less, 7 or less, 6 or less, 5 or less, 4 or less, 3 or less, 2 or less, or 1 or less.

[17.1] The method according to any one of [1] to [17], wherein the aromatic hydrocarbon solvent, halogen solvent, ether solvent, ester solvent, ketone solvent, carbonate solvent or phosphoric acid ester solvent in the step (2) has a donor number value of 25 or less, 24 or less, 23 or less, 22 or less, 21 or less, 20 or less, 19 or less, 18 or less, 17 or less, 16 or less, 15 or less, 14 or less, 13 or less, 12 or less, 11 or less, 10 or less, 9 or less, 8 or less, 7 or less, 6 or less, 5 or less, 4 or less, 3 or less, 2 or less, or 1 or less.

[18] The method according to any one of [1] to [9], wherein the solvent in the step (2) is toluene or cumene.

[19] The method according to any one of [1] to [9], wherein the solvent in the step (2) is dichloromethane.

[20] The method according to any one of [1] to [9], wherein the solvent in the step (2) is tetrahydrofuran, 1,2-dimethoxyethane, 1,3-dioxolane or 2-methyltetrahydrofuran.

[21] The method according to any one of [1] to [9], wherein the solvent in the step (2) is methyl propionate or butyl acetate.

[22] The method according to any one of [1] to [9], wherein the solvent in the step (2) is methyl ethyl ketone or diethyl ketone.

[23] The method according to any one of [1] to [9], wherein the solvent in the step (2) is dimethyl carbonate.

[24] The method according to any one of [1] to [9], wherein the solvent in the step (2) is tributyl phosphate.

[25] The method according to any one of [1] to [24], wherein the base in the step (2) is at least one selected from the group consisting of an amidine, a guanidine and a phosphazene.

[26] The method according to any one of [1] to [25], wherein the base in the step (2) is at least one selected from the group consisting of DBU, MTBD, TMG, P1tBu, P2Et and HP1 (dma).

[27] The method according to any one of [1] to [26], wherein the base in the step (2) is DBU, MTBD, TMG, P1tBu, P2Et or HP1 (dma).

[28] The method according to any one of [1] to [26], wherein the base in the step (2) is (i) a combination of DBU with piperidine or (ii) a combination of DBU with MTBD, HP1 (dma), P1tBu or P2Et.

[29] The method according to any one of [1] to [28], wherein the base in the step (2) is contained in the solvent at a concentration of 1 to 8 v/v%.

[30] The method according to any one of [1] to [29], wherein the step (2) further comprises the step of bringing the solvent into contact with $CO_2$.

[31] The method according to any one of [1] to [30], wherein the step (2) further comprises the step of bubbling the solvent with $CO_2$.

[32] The method according to any one of [1] to [29], wherein the solvent in the step (2) is a solvent brought into contact with $CO_2$ in advance.

[33] The method according to any one of [1] to [29], wherein the solvent in the step (2) is a solvent bubbled with $CO_2$ in advance.

[34] The method according to any one of [1] to [33], wherein the step (2) is repeated two or more times.

[35] The method according to [34], wherein when the step (2) is repeated two or more times, the same solvent is used in the repeats of step (2).

[36] The method according to [34], wherein when the step (2) is repeated two or more times, different solvents are used in the repeats of step (2).

[37] The method according to any one of [34] to [36], wherein when the step (2) is repeated two or more times, the same base is used in the repeats of step (2).

[38] The method according to any one of [34] to [36], wherein when the step (2) is repeated two or more times, different bases are used in the repeats of step (2).

[39] The method according to any one of [34] to [38], further comprising the step of discharging a solution between the repeats of step (2) when the step (2) is repeated two or more times.

[40] The method according to any one of [1] to [39], wherein the carboxylic acid or carboxylic acid analog is an amino acid having a protective group, a second peptide having a protective group, a $C_1$-$C_8$ alkylcarboxylic acid, or a $C_6$-$C_{10}$ arylcarboxylic acid; or an active ester of an amino acid having a protective group, a second peptide having a protective group, a $C_1$-$C_8$ alkylcarboxylic acid, or a $C_6$-$C_{10}$ arylcarboxylic acid; or an acid halide of an amino acid having a protective group, a second peptide having a protective group, a $C_1$-$C_8$ alkylcarboxylic acid, or a $C_6$-$C_{10}$ arylcarboxylic acid; wherein the $C_1$-$C_8$ alkylcarboxylic acid and the $C_6$-$C_{10}$ arylcarboxylic acid are optionally substituted with one or more substituents independently selected from the group consisting of alkenyl, alkynyl, alkoxy, cycloalkyl, aryl, heteroaryl, heterocyclyl, arylalkyl, heteroarylalkyl, halogen, nitro, dialkylamino, cyano, alkoxycarbonyl and dialkylaminocarbonyl.

[41] The method according to [40], wherein the protective group is a carbamate-type protective group, a sulfonyl-type protective group or an acyl-type protective group.

[42] The method according to [41], wherein the carbamate-type protective group is a protective group containing an Fmoc skeleton, Alloc, Teoc, Boc or Cbz, the sulfonyl-type protective group is Ns, and the acyl-type protective group is Tfa.

[43] The method according to [40], wherein the carboxylic acid or carboxylic acid analog is a $C_1$-$C_8$ alkylcarboxylic acid or a $C_6$-$C_{10}$ arylcarboxylic acid, or an active ester or acid halide thereof, and the $C_1$-$C_8$ alkylcarboxylic acid and the $C_6$-$C_{10}$ arylcarboxylic acid are optionally substituted with one or more substituents independently selected from the group consisting of alkenyl, alkynyl, alkoxy, cycloalkyl and halogen.

[44] The method according to [40], wherein the carboxylic acid or carboxylic acid analog is a $C_1$-$C_8$ alkylcarboxylic acid optionally substituted with one or more substituents independently selected from the group consisting of alkenyl, alkynyl, alkoxy, cycloalkyl and halogen, or an active ester or acid halide thereof.

[45] The method according to [40], wherein the carboxylic acid or carboxylic acid analog is a $C_1$-$C_8$ alkylcarboxylic acid, or an active ester or acid halide thereof.

[46] The method according to any one of [1] to [45], wherein the first peptide contains 2 to 30, 2 to 20, 2 to 15, 14, 13, 12, 11, 10, 9, 8, 7, 6, 5, 4, 3 or 2 amino acid residues.

[47] The method according to any one of [1] to [46], wherein the first peptide is a dipeptide.

[48] The method according to any one of [1] to [47], wherein the carboxylic acid or carboxylic acid analog is an amino acid or a second peptide having a protective group; or an active ester of an amino acid or a second peptide having a protective group; or an acid halide of an amino acid or a second peptide having a protective group; wherein the first peptide and/or the second peptide having a protective group contains one or more N-substituted amino acids, and/or the amino acid having a protective group is a N-substituted amino acid.

[49] The method according to any one of [1] to [48], wherein the carboxylic acid or carboxylic acid analog is an amino acid or a second peptide having a protective group containing an Fmoc skeleton; or an active ester of an amino acid or a second peptide having a protective group containing an Fmoc skeleton; or an acid halide of an amino acid or a second peptide having a protective group containing an Fmoc skeleton; wherein the first peptide and/or the second peptide having a protective group containing an Fmoc skeleton contains one or more N-substituted amino acids, and/or the amino acid having a protective group containing an Fmoc skeleton is a N-substituted amino acid.

[50] The method according to any one of [1] to [49], wherein the amino acid at the second residue from the N-terminal of the first peptide is a N-substituted amino acid.

[51] The method according to any one of [1] to [50], wherein the condensation agent in the step (3) is in the form of a salt, and the counter anion thereof is $PF_6^-$ or $BF_4^-$.

[52] The method according to any one of [1] to [51], wherein the condensation agent in the step (3) contains at least one selected from the group consisting of PyOxim, PyAOP, PyBOP, COMU, HATU, HBTU, HCTU, TDBTU, HOTU, TATU, TBTU, TCTU and TOTU.

[53] The method according to any one of [1] to [52], wherein the condensation agent in the step (3) contains at least one selected from the group consisting of PyOxim, PyAOP, PyBOP, COMU, HATU, HBTU, HCTU, TDBTU, HOTU, TATU, TBTU, TCTU or TOTU.

[54] The method according to any one of [1] to [53], wherein the step (3) is carried out in the presence of a base.

[55] The method according to any one of [1] to [54], wherein the step (3) is carried out in the presence of a base having a pKa of 5 to 12 in water as a conjugate acid.

[56] The method according to [54] or [55], wherein the base in the step (3) is DIPEA.

[57] The method according to any one of [1] to [56], wherein the protective group containing an Fmoc skeleton is represented by the following formula (1):

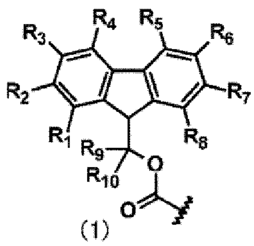

(1)

wherein

$R_1$ to $R_8$ are independently selected from the group consisting of hydrogen, $C_1$-$C_8$ alkyl, $C_1$-$C_8$ fluoroalkyl, halogen, sulfo and trimethylsilyl; and

$R_9$ to $R_{10}$ are independently hydrogen or methyl.

[58] The method according to [57], wherein the protective group containing an Fmoc skeleton is an Fmoc (2,7tb) group, an Fmoc (1Me) group, an Fmoc (2F) group, an Fmoc (2,7Br), a mio-Fmoc group, a dio-Fmoc group, a tdf-Fmoc group, an Fmoc (2TMS) group, an Fmoc (2so3h) group, a sm-Fmoc group or a rm-Fmoc group.

[59] The method according to [57], wherein the protective group containing an Fmoc skeleton is an Fmoc group.

[60] The method according to any one of [1] to [59], further comprising the step of washing a solid-phase synthesis resin between the step (2) and the step (3).

[61] The method according to [60], wherein the washing step further comprises the steps of:

i) washing a solid-phase synthesis resin using a solvent brought into contact with $CO_2$ in advance; and/or
ii) washing the solid-phase synthesis resin using a solvent that is not brought into contact with $CO_2$ in advance.

[62] The method according to [60], wherein the washing step further comprises the steps of:

i) washing a solid-phase synthesis resin using a solvent bubbled with $CO_2$ in advance; and/or
ii) washing the solid-phase synthesis resin using a solvent that is not bubbled with $CO_2$ in advance.

[63] The method according to any one of [1] to [62], wherein the solvent in the step (3) is identical to the solvent in the step (2).
[64] The method according to any one of [1] to [63], wherein the solvent in the step (3) is a solvent in which the condensation agent is soluble.
[65] The method according to any one of [1] to [64], wherein the solvent in the step (3) contains at least one selected from the group consisting of an aromatic hydrocarbon solvent, a halogen solvent, an ether- solvent, an amide solvent, a sulfoxide solvent, a sulfone solvent, a urea solvent, an ester solvent, a ketone solvent, a carbonate solvent and a phosphoric acid ester solvent.
[66] The method according to any one of [1] to [64], wherein the solvent in the step (3) contains at least one selected from the group consisting of an aromatic hydrocarbon solvent, a halogen solvent, an ether solvent, an amide solvent, a sulfoxide solvent, a sulfone solvent, a urea solvent, an ester solvent, a ketone solvent, a carbonate solvent and a phosphoric acid ester solvent at 25 v/v%.
[67] The method according to any one of [1] to [64], wherein the solvent in the step (3) contains at least one selected from the group consisting of an aromatic hydrocarbon solvent, a halogen solvent, an ether solvent, an amide solvent, a sulfoxide solvent, a sulfone solvent, a urea solvent, an ester solvent, a ketone solvent, a carbonate solvent and a phosphoric acid ester solvent at 50 v/v%.
[68] The method according to any one of [1] to [64], wherein the solvent in the step (3) contains at least one selected from the group consisting of an aromatic hydrocarbon solvent, a halogen solvent, an ether solvent, an amide solvent, a sulfoxide solvent, a sulfone solvent, a urea solvent, an ester solvent, a ketone solvent, a carbonate solvent and a phosphoric acid ester solvent at 75 v/v%.
[69] The method according to any one of [1] to [68], wherein the solvent in the step (3) includes at least one selected from the group consisting of an aromatic hydrocarbon solvent, a halogen solvent, an ether solvent, an amide solvent, a sulfoxide solvent, a sulfone solvent, a urea solvent, an ester solvent, a ketone solvent, a carbonate solvent and a phosphoric acid ester solvent.
[70] The method according to any one of [1] to [69], wherein the solvent in the step (3) is an aromatic hydrocarbon solvent, a halogen solvent, an ether solvent, an amide solvent, a sulfoxide solvent, a sulfone solvent, a urea solvent, an ester solvent, a ketone solvent, a carbonate solvent or a phosphoric acid ester solvent.
[71] The method according to any one of [65] to [70], wherein the aromatic hydrocarbon solvent is one or more selected from the group consisting of benzene, toluene, xylene, chlorobenzene, 1,2-dichlorobenzene, bromobenzene, anisole, ethylbenzene, nitrobenzene and cumene,

the halogen solvent is one or more selected from the group consisting of dichloromethane, chloroform, 1,2-dichloroethane and carbon tetrachloride,
the ether solvent is one or more selected from the group consisting of diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, t-butyl methyl ether, diglyme, triglyme and tetraglyme,
the amide solvent is one or more selected from the group consisting of DMF, NMP, DMA, NEP, NBP and formamide,
the sulfoxide solvent is one or more selected from the group consisting of DMSO and methyl phenyl sulfoxide,
the sulfone solvent is one or more selected from the group consisting of diphenyl sulfone, dimethyl sulfone, sulfolane, 3-methylsulfolane, ethyl methyl sulfone and ethyl isopropyl sulfone,
the urea solvent is one or more selected from the group consisting of DMI and DMPU,
the ester solvent is one or more selected from the group consisting of methyl acetate, ethyl acetate, methyl propionate, butyl acetate, propyl acetate, isopropyl acetate, isobutyl acetate, pentyl acetate and $\gamma$-valerolactone,
the ketone solvent is one or more selected from the group consisting of acetone, methyl ethyl ketone, methyl isobutyl ketone, cyclohexanone, diethyl ketone and cyclopentanone,
the carbonate solvent is one or more selected from the group consisting of dimethyl carbonate, diethyl carbonate and dibutyl carbonate, and
the phosphoric acid ester solvent is one or more selected from the group consisting of trimethyl phosphate, triethyl phosphate and tributyl phosphate.

[72] The method according to any one of [1] to [71], wherein the carboxylic acid for use in the step (3) is an amino acid or a second peptide having a protective group containing an Fmoc skeleton, and the protective group containing an Fmoc skeleton in the amino acid or second peptide is identical to or different from the protective group containing an Fmoc skeleton in the first peptide for use in the step (1).

[73] The method according to any one of [1] to [72], wherein the carboxylic acid analog for use in the step (3) is an acid halide of an amino acid having a protective group, a second peptide having a protective group, a $C_1$-$C_8$ alkylcarboxylic acid, or a $C_6$-$C_{10}$ arylcarboxylic acid, and the carboxyl group of the amino acid having a protective group containing an Fmoc skeleton, the carboxyl group at the C-terminal of the second peptide having a protective group containing an Fmoc skeleton, the carboxyl group of the $C_1$-$C_8$ alkylcarboxylic acid or the carboxyl group or the $C_6$-$C_{10}$ arylcarboxylic acid.

[74] A method for reducing the amount of a diketopiperazine impurity and/or a 6-membered cyclic amidine skeleton compound impurity formed in production of a peptide by a solid-phase method, comprising the steps of:

(1) providing a first peptide having a protective group containing an Fmoc skeleton and supported on a solid-phase; and

(2) treating the first peptide with one or more bases including at least a base having a pKa of 23 or more in acetonitrile as a conjugate acid in a solvent containing at least one selected from the group consisting of an aromatic hydrocarbon solvent, a halogen solvent, an ether solvent, an ester solvent, a ketone solvent, a carbonate solvent and a phosphoric acid ester solvent after the step (1).

**Advantageous Effects of Invention**

In peptide synthesis using a solid-phase method, the present invention ensures that even if there is an amino acid sequence in which under conventional conditions, a desired elongation reaction does not sufficiently proceed because diketopiperazine is formed and/or a 6-membered cyclic amidine skeleton compound is formed, formation of such impurities can be significantly reduced, so that a peptide chain can be efficiently elongated to obtain a peptide having a desired amino acid sequence. Since the method of the present invention does not require the use of a special protective group such as Alloc having low universality, and does not place a limitation in terms of reaction operation such that an extremely short reaction time is addressed, the method can be practical synthesis method which has high versatility and can be scaled up.

**Brief Description of Drawings**

[Figure 1] Figure 1 shows a $^1$H-NMR spectrum between 3.6 ppm and 6.8 ppm before and after 1,8-diazabicyclo[5.4.0]-7-undecene (DBU) is applied to Fmoc-Phe-$NMe_2$ (compound 1-3-1) in N,N-dimethylformamide-d7 (DMF-d7), along with characteristic $^1$H signals of compound 1-3-1, compound 1-3-2, compound 1-3-3a and dibenzofulvene observed in this range.

In Figure 1, a) shows a $^1$H-NMR spectrum for a solution obtained by applying 1,8-diazabicyclo[5.4.0]-7-undecene (DBU) to compound 1-3-1 in N,N-dimethylformamide-d7 (DMF-d7).

In Figure 1, b) shows a $^1$H-NMR spectrum for a solution of compound 1-3-1 in N,N-dimethylformamide-d7 (DMF-d7).

[Figure 2] Figure 2 is a diagram of a $^1$H-NMR spectrum at around 5.4 to 3.5 ppm which shows that compound 1-3-2 and compound 1-3-3a formed by applying 1,8-diazabicyclo[5.4.0]-7-undecene (DBU) to compound 1-3-1 in N,N-dimethylformamide-d7 (DMF-d7) undergo chemical exchange and the ratio thereof is 82 : 18.

In Figure 2, a)-1 shows a $^1$H-NMR spectrum for a solution obtained by applying 1,8-diazabicyclo[5.4.0]-7-undecene (DBU) to compound 1-3-1 in N,N-dimethylformamide-d7 (DMF-d7).

In Figure 2, a)-2 shows a 1D-ROESY spectrum obtained by selectively exciting a $^1$H signal at 4.82 ppm for a solution obtained by applying 1,8-diazabicyclo[5.4.0]-7-undecene (DBU) to compound 1-3-1 in N,N-dimethylformamide-d7 (DMF-d7).

In Figure 2, a)-3 shows a 1D-ROESY spectrum obtained by selectively exciting a $^1$H signal at 3.95 ppm for a solution obtained by applying 1,8-diazabicyclo[5.4.0]-7-undecene (DBU) to compound 1-3-1 in N,N-dimethylformamide-d7 (DMF-d7).

In Figure 2, b) shows $^1$H-NMR spectrum showing an integral ratio between a $^1$H signal at 4.85 ppm and a $^1$H signal at 3.95 ppm for a solution obtained by applying 1,8-diazabicyclo[5.4.0]-7-undecene (DBU) to compound 1-3-1 in N,N-dimethylformamide-d7 (DMF-d7).

[Figure 3] Figure 3 shows a $^1$H-NMR spectrum at around 5.2 to 3.5 ppm which shows that compound 1-3-2 is formed by applying 1,8-diazabicyclo[5.4.0]-7-undecene (DBU) to compound 1-3-1 in N,N-dimethylformamide-d7 (DMF-d7).

In Figure 3, a)-1 shows a $^1$H-NMR spectrum of a standard product of compound 1-3-2 in N,N-dimethylformamide-d7 (DMF-d7).

In Figure 3, a)-2 shows a $^1$H-NMR spectrum for a solution obtained by applying 1,8-diazabicyclo[5.4.0]-7-undecene (DBU) to compound 1-3-1 in N,N-dimethylformamide-d7 (DMF-d7).

In Figure 3, a)-3 shows a $^1H$-NMR spectrum for a solution obtained by applying 1,8-diazabicyclo[5.4.0]-7-undecene (DBU) to compound 1-3-1 in N,N-dimethylformamide-d7 (DMF-d7) and adding 3 μL of a standard product of compound 1-3-2.

In Figure 3, a)-4 shows a 1D-NOESY spectrum obtained by selectively exciting a $^1H$ signal at 4.84 ppm for a solution obtained by applying 1,8-diazabicyclo[5.4.0]-7-undecene (DBU) to compound 1-3-1 in N,N-dimethylformamide-d7 (DMF-d7) and adding 3 μL of a standard product of compound 1-3-2.

In Figure 3, a)-5 shows a 1D-NOESY spectrum obtained by selectively exciting a $^1H$ signal at 3.95 ppm for a solution obtained by applying 1,8-diazabicyclo[5.4.0]-7-undecene (DBU) to compound 1-3-1 in N,N-dimethylformamide-d7 (DMF-d7) and adding 3 μL of a standard product of compound 1-3-2.

In Figure 3, b) is a diagram in which the integral value of a proton signal at 4.84 ppm and 3.95 ppm in a $^1H$-NMR spectrum for a solution obtained by applying 1,8-diazabicyclo[5.4.0]-7-undecene (DBU) to compound 1-3-1 in N,N-dimethylformamide-d7 (DMF-d7) and adding 3 μL of a standard product of compound 1-3-2 is determined and the abundance ratio between compound 1-3-3 and compound 1-3-2 is calculated on the basis of the integral value.

[Figure 4] Figure 4 shows $^1H$-NMR and 1D-NOESY spectra at around 5.1 to 3.7 ppm which show that compound 1-3-3a is formed by bubbling compound 1-3-2 with $^{13}CO_2$ and then applying 1,8-diazabicyclo[5.4.0]-7-undecene (DBU) in N,N-dimethylformamide-d7 (DMF-d7). In Figure 4, a) shows a $^1H$-NMR spectrum of a standard product of compound 1-3-2 in N,N-dimethylformamide-d7 (DMF-d7).

In Figure 4, b) shows a $^1H$-NMR spectrum for a solution obtained by bubbling a solution of compound 1-3-2 in N,N-dimethylformamide-d7 (DMF-d7) with $^{13}CO_2$ for 1 minute.

In Figure 4, c) shows a 1D-NOESY spectrum obtained by selectively exciting $^1H$ signal at 4.77 ppm for a solution obtained by bubbling a solution of compound 1-3-2 in N,N-dimethylformamide-d7 (DMF-d7) with $^{13}CO_2$ for 1 minute.

In Figure 4, d) shows a 1D-NOESY spectrum obtained by selectively exciting $^1H$ signal at 3.96 ppm for a solution obtained by bubbling a solution of compound 1-3-2 in N,N-dimethylformamide-d7 (DMF-d7) with $^{13}CO_2$ for 1 minute.

In Figure 4, e) shows a $^1H$-NMR spectrum for a solution obtained by bubbling a solution of compound 1-3-2 in N,N-dimethylformamide-d7 (DMF-d7) with $^{13}CO_2$ for 1 minute and then applying 1,8-diazabicyclo[5.4.0]-7-undecene (DBU).

In Figure 4, f) is a diagram of a $^1H$-NMR spectrum showing an integral ratio between a $^1H$ signal at 4.77 ppm and a $^1H$ signal at 3.96 ppm after a solution of compound 1-3-2 in N,N-dimethylformamide-d7 (DMF-d7) is bubbled with $^{13}CO_2$ for 1 minute.

In Figure 4, e) is a diagram of an enlarged $^1H$ signal at 4.83 ppm in $^1H$-NMR spectrum for a solution obtained by bubbling a solution of compound 1-3-2 in N,N-dimethylformamide-d7 (DMF-d7) with $^{13}CO_2$ for 1 minute and then applying 1,8-diazabicyclo[5.4.0]-7-undecene (DBU). Only for this diagram, conversion using Sin-bell as a window function is applied for improvement of resolution.

[Figure 5] Figure 5 shows a $^{13}C$-NMR spectrum at around 164 to 124 ppm which show that compound 1-3-3a is formed by bubbling compound 1-3-2 with $^{13}CO_2$ and then applying 1,8-diazabicyclo[5.4.0]-7-undecene (DBU) in N,N-dimethylformamide-d7 (DMF-d7).

In Figure 5, a) shows a $^{13}C$-NMR spectrum for a solution obtained by bubbling compound 1-3-2 with $^{13}CO_2$ in N,N-dimethylformamide-d7 (DMF-d7).

In Figure 5, b) shows a $^{13}C$-NMR spectrum for a solution obtained by bubbling a solution of compound 1-3-2 with $^{13}CO_2$ in N,N-dimethylformamide-d7 (DMF-d7) and then applying 1,8-diazabicyclo[5.4.0]-7-undecene (DBU).

[Figure 6] Figure 6 shows a $^1H$-NMR spectrum at around 6.9 to 3.6 ppm which shows that compound 1-3-3a is present in a solution (solution B, Sol B) obtained by applying 1,8-diazabicyclo[5.4.0]-7-undecene (DBU) to compound 1-3-1 in N,N-dimethylformamide-d7 (DMF-d7).

In Figure 6, a) shows a $^1H$-NMR spectrum for a solution (solution B) obtained by applying 1,8-diazabicyclo[5.4.0]-7-undecene (DBU) to compound 1-3-1 in N,N-dimethylformamide-d7 (DMF-d7).

In Figure 6, b) shows a $^1H$-NMR spectrum for a solution obtained by mixing a solution (solution B) obtained by applying 1,8-diazabicyclo[5.4.0]-7-undecene (DBU) to compound 1-3-1 in N,N-dimethylformamide-d7 (DMF-d7) and a solution (solution A) obtained by bubbling a solution of compound 1-3-2 in N,N-dimethylformamide-d7 (DMF-d7) with $^{13}CO_2$ for 1 minute and then applying 1,8-diazabicyclo[5.4.0]-7-undecene (DBU).

In Figure 6, c) shows a $^1H$-NMR spectrum for a solution (solution A) obtained by bubbling a solution of compound 1-3-2 in N,N-dimethylformamide-d7 (DMF-d7) with $^{13}CO_2$ for 1 minute and then applying 1,8-diazabicyclo[5.4.0]-7-undecene (DBU).

[Figure 7] Figure 7 shows NH-HSQC ($^{15}N$[F1]: around 97-85 ppm and $^1H$[F2]: around 5.7 to 4.7 ppm), NH-HMBC ($^{15}N$[F1]: around 101 to 83 ppm and $^1H$[F2]: around 3.04 to 2.64 ppm) and CH-HMBC ($^{13}C$[F1]: around 165 to 158 ppm and $^1H$[F2]: around 5.7 to 4.5 ppm) spectra showing that compound 1-3-3a is present in a solution obtained by mixing a solution (solution B) obtained by applying 1,8-diazabicyclo[5.4.0]-7-undecene (DBU) to compound 1-3-1 in N,N-dimethylformamide-d7 (DMF-d7) and a solution (solution A) obtained by bubbling a solution of compound 1-3-2 in N,N-dimethylformamide-d7 (DMF-d7) with $^{13}CO_2$ for 1 minute and then applying 1,8-diazabicyclo[5.4.0]-7-undecene (DBU).

In Figure 7, a) shows a NH-HSQC spectrum for a solution obtained by mixing a solution (solution B) obtained by applying 1,8-diazabicyclo[5.4.0]-7-undecene (DBU) to compound 1-3-1 in N,N-dimethylformamide-d7 (DMF-d7) and a solution (solution A) obtained by bubbling a solution of compound 1-3-2 in N,N-dimethylformamide-d7 (DMF-d7) with $^{13}CO_2$ for 1 minute and then applying 1,8-diazabicyclo[5.4.0]-7-undecene (DBU).

In Figure 7, b) shows a NH-HMBC spectrum for a solution obtained by mixing a solution (solution B) obtained by applying 1,8-diazabicyclo[5.4.0]-7-undecene (DBU) to compound 1-3-1 in N,N-dimethylformamide-d7 (DMF-d7) and a solution (solution A) obtained by bubbling a solution of compound 1-3-2 in N,N-dimethylformamide-d7 (DMF-d7) with $^{13}CO_2$ for 1 minute and then applying 1,8-diazabicyclo[5.4.0]-7-undecene (DBU).

In Figure 7, c) shows a CH-HMBC spectrum for a solution obtained by mixing a solution (solution B) obtained by applying 1,8-diazabicyclo[5.4.0]-7-undecene (DBU) to compound 1-3-1 in N,N-dimethylformamide-d7 (DMF-d7) and a solution (solution A) obtained by bubbling a solution of compound 1-3-2 in N,N-dimethylformamide-d7 (DMF-d7) with $^{13}CO_2$ for 1 minute and then applying 1,8-diazabicyclo[5.4.0]-7-undecene (DBU).

[Figure 8] Figure 8 is a diagram showing that all of $^1H$, $^{13}C$ and $^{15}N$ signals of a carbamate part of compound 1-3-3a present in a solution obtained by mixing a solution (solution B) obtained by applying 1,8-diazabicyclo[5.4.0]-7-undecene (DBU) to compound 1-3-1 in N,N-dimethylformamide-d7 (DMF-d7) and a solution (solution A) obtained by bubbling a solution of compound 1-3-2 in N,N-dimethylformamide-d7 (DMF-d7) with $^{13}CO_2$ for 1 minute and then applying 1,8-diazabicyclo[5.4.0]-7-undecene (DBU).

## Description of Embodiments

(Terminology)

Examples the "halogen atom" in the present specification include F, Cl, Br and I.

In the present specification, the "alkyl" is a monovalent group which is induced by the removal of any one hydrogen atom from aliphatic hydrocarbon and has a subset of a hydrocarbyl or hydrocarbon group structure containing hydrogen and carbon atoms without containing a heteroatom (which refers to an atom other than carbon and hydrogen atoms) or an unsaturated carbon-carbon bond in the skeleton. The alkyl includes not only a linear form but also a branched form. The alkyl is specifically alkyl having 1 to 20 carbon atoms ($C_1$-$C_{20}$; hereinafter, "$C_p$-$C_q$" means that the number of carbon atoms is p to q), preferably $C_1$-$C_{10}$ alkyl, more $C_1$-$C_6$ alkyl. Examples of the alkyl specifically include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

In the present specification, the "alkenyl" is a monovalent group having at least one double bond (two adjacent $SP^2$ carbon atoms). Depending on the conformation of the double bond and a substituent (if present), the geometric morphology of the double bond can assume entgegen (E) or zusammen (Z) and cis or trans conformations. The alkenyl includes not only a linear form but also a branched form. The alkenyl is preferably $C_2$-$C_{10}$ alkenyl, more preferably $C_2$-$C_6$ alkenyl. Examples thereof specifically include vinyl, allyl, 1-propenyl, 2-propenyl, 1-butenyl, 2-butenyl (which includes cis and trans), 3-butenyl, pentenyl, 3-methyl-2-butenyl, and hexenyl.

In the present specification, the "alkynyl" is a monovalent group having at least one triple bond (two adjacent SP carbon atoms). The alkynyl includes not only a linear form but also a branched form. The alkynyl is preferably $C_2$-$C_{10}$ alkynyl, more preferably $C_2$-$C_6$ alkynyl. Examples thereof specifically include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

In the present specification, the "cycloalkyl" means a saturated or partially saturated cyclic monovalent aliphatic hydrocarbon group and includes a monocyclic ring, a bicyclo ring, and a spiro ring. The cycloalkyl is preferably $C_3$-$C_8$ cycloalkyl. Examples thereof specifically include cyclopropyl, cyclobutyl, cyclopentyl, cyclohexyl, cycloheptyl, cyclooctyl, bicyclo[2.2.1]heptyl, and spiro[3.3]heptyl.

In the present specification, the "aryl" means a monovalent aromatic hydrocarbon ring and is preferably $C_6$-$C_{10}$ aryl. Examples of the aryl specifically include phenyl and naphthyl (e.g., 1-naphthyl and 2-naphthyl).

In the present specification, the "heterocyclyl" means a nonaromatic cyclic monovalent group containing a carbon atom as well as 1 to 5 heteroatoms. The heterocyclyl may have a double and/or triple bond in the ring. A carbon atom in the ring may form carbonyl through oxidation, and the ring may be a monocyclic ring or a condensed ring. The number of atoms constituting the ring is preferably 4 to 10 (4- to 10-membered heterocyclyl), more preferably 4 to 7 (4- to 7-membered heterocyclyl). Examples of the heterocyclyl specifically include azetidinyl, oxiranyl, oxetanyl, dihydrofuryl, tetrahydrofuryl, dihydropyranyl, tetrahydropyranyl, tetrahydropyridyl, tetrahydropyrimidyl, morpholinyl, thiomorpholinyl,

pyrrolidinyl, piperidinyl, piperazinyl, pyrazolidinyl, imidazolinyl, imidazolidinyl, oxazolidinyl, isoxazolidinyl, thiazolidinyl, isothiazolidinyl, 1,2-thiazinane, thiadiazolidinyl, oxazolidone, benzodioxanyl, benzoxazolyl, dioxolanyl, dioxanyl, tetrahydropyrrolo[1,2-c]imidazole, thietanyl, 3,6-diazabicyclo[3.1.1]heptanyl, 2,5-diazabicyclo[2.2.1]heptanyl, 3-oxa-8-azabicyclo[3.2.1]octanyl, sultam, and 2-oxaspiro[3.3]heptyl.

In the present specification, the "heteroaryl" means an aromatic cyclic monovalent group containing a carbon atom as well as 1 to 5 heteroatoms. The ring may be a monocyclic ring or a condensed ring with another ring and may be partially saturated. The number of atoms constituting the ring is preferably 5 to 10 (5- to 10-membered heteroaryl), more preferably 5 to 7 (5- to 7-membered heteroaryl). Examples of the heteroaryl specifically include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

In the present specification, the "alkoxy" means an oxy group bonded to the "alkyl" defined above and is preferably $C_1$-$C_6$ alkoxy. Examples of the alkoxy specifically include methoxy, ethoxy, 1-propoxy, 2-propoxy, n-butoxy, i-butoxy, s-butoxy, t-butoxy, pentyloxy, and 3-methylbutoxy.

In the present specification, the "alkenyloxy" means an oxy group bonded to the "alkenyl" defined above and is preferably $C_2$-$C_6$ alkenyloxy. Examples of the alkenyloxy specifically include vinyloxy, allyloxy, 1-propenyloxy, 2-propenyloxy, 1-butenyloxy, 2-butenyloxy (which includes cis and trans), 3-butenyloxy, pentenyloxy, and hexenyloxy.

In the present specification, the "cycloalkoxy" means an oxy group bonded to the "cycloalkyl" defined above and is preferably $C_3$-$C_8$ cycloalkoxy. Examples of the cycloalkoxy specifically include cyclopropoxy, cyclobutoxy, and cyclopentyloxy.

In the present specification, the "aryloxy" means an oxy group bonded to the "aryl" defined above and is preferably $C_6$-$C_{10}$ aryloxy. Examples of the aryloxy specifically include phenoxy, 1-naphthyloxy, and 2-naphthyloxy.

In the present specification, the "heteroaryloxy" means an oxy group bonded to the "heteroaryl" defined above and is preferably 5- to 10-membered heteroaryloxy.

In the present specification, the "amino" means $-NH_2$ in the narrow sense and means - NRR' in the broad sense. In this context, R and R' are each independently selected from hydrogen, alkyl, alkenyl, alkynyl, cycloalkyl, heterocyclyl, aryl, and heteroaryl, or R and R' form a ring together with the nitrogen atom bonded thereto. Examples of the amino preferably include $-NH_2$, mono-$C_1$-$C_6$ alkylamino, di-$C_1$-$C_6$ alkylamino, and 4- to 8-membered cyclic amino.

In the present specification, the "monoalkylamino" means a group of the "amino" defined above in which R is hydrogen, and R' is the "alkyl" defined above, and is preferably mono-$C_1$-$C_6$ alkylamino. Examples of the monoalkylamino specifically include methylamino, ethylamino, n-propylamino, i-propylamino, n-butylamino, s-butylamino, and t-butylamino.

In the present specification, the "dialkylamino" means a group of the "amino" defined above in which R and R' are each independently the "alkyl" defined above, and is preferably di-$C_1$-$C_6$ alkylamino. Examples of the dialkylamino specifically include dimethylamino and diethylamino.

In the present specification, the "cyclic amino" means a group of the "amino" defined above in which R and R' form a ring together with the nitrogen atom bonded thereto, and is preferably 4- to 8-membered cyclic amino. Examples of the cyclic amino specifically include 1-azetidyl, 1-pyrrolidyl, 1-piperidyl, 1-piperazyl, 4-morpholinyl, 3-oxazolidyl, 1,1-dioxidothiomorpholinyl-4-yl, and 3-oxa-8-azabicyclo[3.2.1]octan-8-yl.

In the present specification, the "haloalkyl" means a group in which one or more hydrogen atoms of the "alkyl" defined above are replaced with halogen, and is preferably $C_1$-$C_8$ haloalkyl, more preferably $C_1$-$C_6$ haloalkyl.

In the present specification, the "fluoroalkyl" means a group in which one or more fluorine atoms of the "alkyl" defined above are replaced with halogen, with $C_1$-$C_8$ haloalkyl being preferred. Examples of the haloalkyl specifically include monofluoromethyl, difluoromethyl, trifluoromethyl, 2,2-difluoroethyl, 2,2,2-trifluoroethyl, 3,3-difluoropropyl, 4,4-difluorobutyl, 5,5-difluoropentyl, and 3,3,4,4,5,5,6,6,7,7,8,8,8-tridecafluorooctyl.

In the present specification, the "aralkyl (arylalkyl)" means a group in which at least one hydrogen atom of the "alkyl" defined above is replaced with the "aryl" defined above, and is preferably $C_7$-$C_{14}$ aralkyl, more preferably $C_7$-$C_{10}$ aralkyl. Examples of the aralkyl specifically include benzyl, phenethyl, and 3-phenylpropyl.

In the present specification, the "aralkyloxy" means an oxy group bonded to the "aralkyl" defined above and is preferably $C_7$-$C_{14}$ aralkyloxy, more preferably $C_7$-$C_{10}$ aralkyloxy. Examples of the aralkyloxy specifically include benzyloxy, phenethyloxy, and 3-phenylpropoxy.

In the present specification, the "peptide chain" refers to a peptide chain of 1 or more natural amino acids and/or non-natural amino acids linked through an amide bond and/or an ester bond. The peptide chain is preferably a peptide chain comprising 1 to 15 amino acid residues, more preferably a peptide chain consisting of 5 to 12 amino acid residues.

As used herein, the term "peptide compound" is not particularly limited as long as it is a peptide compound in which natural amino acids and/or non-natural amino acids are linked through an amide bond or an ester bond, and the peptide compound is preferably one having 5 to 30 residues, more preferably one having 8 to 15 residues, still more

preferably one having 9 to 13 residues. The peptide compound synthesized in the present invention contains preferably at least three N-substituted amino acids, more preferably at least 5 N-substituted amino acids, in one peptide. The N-substituted amino acids may be present continuously or discontinuously in the N-substituted cyclic peptide compound. The peptide compound according to the present invention may be linear or cyclic, and is preferably a cyclic peptide compound. In the present specification, the "peptide residue" is sometimes referred to as a "peptide".

The "cyclic peptide compound" in the present invention is a cyclic peptide compound which can be obtained by cyclizing a group on the N-terminal side and a group on the C-terminal side in a linear peptide compound. The cyclization may take any form, such as cyclization through a carbon-nitrogen bond such as an amide bond, cyclization through a carbon-oxygen bond such as an ester bond or ether bond, cyclization through a carbon-sulfur bond such as a thioether bond, cyclization through a carbon-carbon bond, or cyclization by heterocyclic construction. Among these, cyclization through covalent bonds such as amide bonds or carbon-carbon bonds is preferred, and cyclization through an amide bond of a carboxylic acid group on the side chain and an amino group at the n-terminal on the main chain is more preferred. The positions of the carboxylic acid group, the amino group, and the like used for cyclization may be on the main chain or the side chain, and are not particularly limited as long as the positions allow the groups to be cyclized.

In the present specification, the term "one or more" means a number of 1 or 2 or larger. When the term "one or more" is used in a context related to a substituent for a certain group, this term means a number from 1 to the maximum number of substituents accepted by the group. Examples of the term "one or more" specifically include 1, 2, 3, 4, 5, 6, 7, 8, 9, 10, or larger numbers.

In the present specification, the "solid-phase synthesis resin" is not particularly limited as long as it can be used for synthesis of a peptide compound by a solid-phase method. Examples of the solid-phase synthesis resin specifically include those that can be removed under acidic conditions, such as CTC resin, Wang resin, SASRIN resin, tritylchloride resin (Trt resin), 4-methyltrityl chloride resin (Mtt resin), and 4-methoxytrityl chloride resin (Mmt). The resin can be appropriately selected in accordance with a functional group on the side of an amino acid used. For example, when a carboxylic acid (a main chain carboxylic acid or a side chain carboxylic acid typified by Asp or Glu) or a hydroxy group on an aromatic ring (a phenol group typified by Tyr) is used as the functional group on the amino acid side, it is preferred to use trityl chloride resin (Trt resin) or 2-chlorotrityl chloride resin (CTC resin) as the resin. When an aliphatic hydroxy group (an aliphatic alcohol group typified by Ser or Thr) is used as the functional group on the amino acid side, it is preferred to use trityl chloride resin (Trt resin), 2-chlorotrityl chloride resin (CTC resin) or 4-methyltrityl chloride resin (Mtt resin) as the resin. In the present specification, the resin may be referred to as resin.

The type of polymer constituting the resin is not particularly limited. For the resin composed of polystyrene, either resin of 100 to 200 mesh or resin of 200 to 400 mesh may be used. The crosslinking degree is not particularly limited, and resin cross-linked with 1% DVB (divinylbenzene) is preferred. Examples of the type of polymer constituting the resin include Tentagel and Chemmatrix.

If in production of a compound described in the present specification, a defined group undergoes undesired chemical conversion under conditions of the implementation method, for example, means such as protection or deprotection of a functional group can be used to produce the compound. Here, for operations of selection and desorption of a protective group, for example, methods described in "Greene’s, "Protective Groups in Organic Synthesis" (5th edition, John Wiley & Sons 2014)" can be mentioned, and may be appropriately used according to reaction conditions. It is also possible to change the order of reaction steps such as introduction of substituents if necessary.

When the modifier "optionally substituted" is given in the present specification, examples of the relevant substituent include alkyl, alkoxy, fluoroalkyl, fluoroalkoxy, oxo, aminocarbonyl, alkylsulfonyl, alkylsulfonylamino, cycloalkyl, aryl, heteroaryl, heterocyclyl, arylalkyl, heteroarylalkyl, halogen, nitro, amino, monoalkylamino, dialkylamino,cyano, carboxyl, alkoxycarbonyl, and formyl.

A substituent may be added to each of the groups. Such a substituent is not limited and can be one or two or more substituents each independently freely selected from arbitrary substituents including a halogen atom, an oxygen atom, a sulfur atom, a nitrogen atom, a boron atom, a silicon atom, and a phosphorus atom. Specifically, examples thereof include optionally substituted alkyl, alkenyl, alkynyl, aryl, heteroaryl, aralkyl, and cycloalkyl.

The compound described in the present specification can be a salt thereof, or a solvate thereof. Examples of the salt described in the present specification thereof include: hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salt, and potassium salt; alkaline earth metal salts such as magnesium salt and calcium salt; and ammonium salts such as ammonium salt, alkylammonium salt, dialkylammonium salt, trialkylammonium salt, and tetraalkylammonium salt. These salts are produced by, for example, bringing the relevant compound into contact with an acid or a base. The solvate of the compound described in the present specification refers to a phenomenon in which solute molecules strongly attract solvent molecules to form a molecular group in the solution. The solvate is a hydrate when the solvent is water. The compound described in the present specification may be a solvate with a single solvent selected from organic solvents such as alcohols (e.g. methanol, ethanol, 1-propanol and 2-propanol), dimethylformamide and diglyme, water and the like, or a solvate with a plurality of

solvents.

The term "amino acid" in the present specification includes a natural amino acid and a non-natural amino acid (sometimes referred to as an amino acid derivative). In the present specification, the "natural amino acid" refers to Gly, Ala, Ser, Thr, Val, Leu, Ile, Phe, Tyr, Trp, His, Glu, Asp, Gln, Asn, Cys, Met, Lys, Arg, or Pro. Non-natural amino acids (amino acid derivatives) are not particularly limited, and examples thereof include a β-amino acid, a D-type amino acid, an N-substituted amino acid, an α,α-disubstituted amino acid, an amino acid having a side chain different from that of natural amino acids, and a hydroxycarboxylic acid. As the amino acids herein, amino acids having any conformation are acceptable. The selection of a side chain of an amino acid is not particularly limited, and the side chain is freely selected from, in addition to a hydrogen atom, an alkyl group, an alkenyl group, an alkynyl group, an aryl group, a heteroaryl group, an aralkyl group, a heteroaralkyl group, a cycloalkyl group, a spiro-bonded cycloalkyl group, and the like. A substituent may be added to each of the groups. The substituent is also not limited, and one or two or more substituents may be freely selected independently from arbitrary substituents including, for example, a halogen atom, an O atom, a S atom, a N atom, a B atom, a Si atom, or a P atom. That is, examples of the side chain include an alkyl group, an alkoxy group, an alkenyl group, an alkynyl group, an aryl group, a heteroaryl group, an aralkyl group, or a cycloalkyl group which may be substituted, oxo, aminocarbonyl, and a halogen atom. In a non-limiting aspect, the amino acid in the present specification may be a compound having a carboxy group and an amino group in the same molecule (even in this case, the amino acid also includes imino acids such as proline and hydroxyproline).

In the present specification, examples of the substituent containing a halogen atom include an alkyl group, a cycloalkyl group, an alkenyl group, an alkynyl group, an aryl group, a heteroaryl group, and an aralkyl group having halogen as a substituent and more specifically include fluoroalkyl, difluoroalkyl, and trifluoroalkyl.

Examples of substituents containing an oxygen atom include hydroxy (-OH), oxy (-OR), carbonyl (-C=O-R), carboxy ($-CO_2H$), oxycarbonyl (-C=O-OR), carbonyloxy (-O-C=O-R), thiocarbonyl (-C=O-SR), a carbonylthio (-S-C=O-R), aminocarbonyl (-C=O-NHR), carbonylamino (-NH-C=O-R), oxycarbonylamino (-NH-C=O-OR), sulfonylamino ($-NH-SO_2-R$), aminosulfonyl ($-SO_2-NHR$), sulfamoylamino ($-NH-SO_2-NHR$), thiocarboxyl (-C=O-SH), and carboxylcarbonyl ($-C=O-CO_2H$).

Examples of the oxy (-OR) include alkoxy, cycloalkoxy, alkenyloxy, alkynyloxy, aryloxy, heteroaryloxy, and aralkyloxy. The alkoxy is preferably $C_1$-$C_4$ alkoxy, $C_1$-$C_2$ alkoxy, particularly preferably methoxy or ethoxy.

Examples of the carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of the oxycarbonyl (-C=O-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of the carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of the thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of the carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of the aminocarbonyl (-C=O-NHR) include alkylaminocarbonyl (e.g. $C_1$-$C_6$ or $C_1$-$C_4$ alkylaminocarbonyl, particularly, ethylaminocarbonyl and methylaminocarbonyl), cycloalkylaminocarbonyl, alkenylaminocarbonyl, alkynylaminocarbonyl, arylaminocarbonyl, heteroarylaminocarbonyl, and aralkylaminocarbonyl. Examples thereof additionally include groups in which the H atom bonded to the N atom in -C=O-NHR is further replaced with alkyl, cycloalkyl, alkenyl, alkynyl, aryl, heteroaryl, or aralkyl.

Examples of the carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Examples thereof additionally include groups in which the H atom bonded to the N atom in -NH-C=O-R is further replaced with alkyl, cycloalkyl, alkenyl, alkynyl, aryl, heteroaryl, or aralkyl.

Examples of the oxycarbonylamino (-NH-C=O-OR) include alkoxycarbonylamino, cycloalkoxycarbonylamino, alkenyloxycarbonylamino, alkynyl oxycarbonylamino, aryloxycarbonylamino, heteroaryloxycarbonylamino, and aralkyloxycarbonylamino. Examples thereof additionally include groups in which the H atom bonded to the N atom in -NH-C=O-OR is further replaced with alkyl, cycloalkyl, alkenyl, alkynyl, aryl, heteroaryl, or aralkyl.

Examples of the sulfonylamino ($-NH-SO_2-R$) include alkylsulfonylamino, cycloalkylsulfonylamino, alkenylsulfonylamino, alkynylsulfonylamino, arylsulfonylamino, heteroarylsulfonylamino, and aralkylsulfonylamino. Examples thereof additionally include groups in which the H atom bonded to the N atom in $-NH-SO_2-R$ is further replaced with alkyl, cycloalkyl, alkenyl, alkynyl, aryl, heteroaryl, or aralkyl.

Examples of the aminosulfonyl ($-SO_2-NHR$) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Examples thereof additionally include groups in which the H atom bonded to the N atom in $-SO_2-NHR$ is further replaced with alkyl, cycloalkyl, alkenyl, alkynyl, aryl, heteroaryl, or aralkyl.

Examples of the sulfamoylamino (-NH-$SO_2$-NHR) include alkylsulfamoylamino, cycloalkylsulfamoylamino, alkenyl sulfamoyl amino, alkynyl sulfamoyl amino, arylsulfamoylamino, heteroaryl sulfamoyl amino, and aralkylsulfamoylamino. The two H atoms bonded to the N atoms in -NH-$SO_2$-NHR may be substituted by substituents each independently selected from the group consisting of alkyl, cycloalkyl, alkenyl, alkynyl, aryl, heteroaryl, and aralkyl, and these two substituents may form a ring.

Examples of the substituent containing a S atom include groups such as thiol (-SH), thio(-S-R), sulfinyl (-S=O-R), sulfonyl (-$SO_2$-R), and sulfo(-$SO_3$H).

Examples of the thio (-S-R) that can be selected include alkylthio, cycloalkylthio, alkenylthio, alkynylthio, arylthio, heteroarylthio, and aralkylthio.

Examples of the sulfonyl (-$SO_2$-R) include alkylsulfonyl, cycloalkylsulfonyl, alkenylsulfonyl, alkynylsulfonyl, arylsulfonyl, heteroarylsulfonyl, and aralkylsulfonyl.

Examples of the substituent containing a nitrogen atom include groups such as azide (-$N_3$; also referred to as an "azide group"), cyano (-CN), primary amino (-$NH_2$), secondary amino (-NH-R; also referred to as mono-substituted amino), tertiary amino (-NR(R'); also referred to as di-substituted amino), amidino (-C(=NH)-$NH_2$), substituted amidino (-C(=NR)-NR'R"), guanidino (-NH-C(=NH)-$NH_2$), substituted guanidino (-NR-C(=NR‴)-NR'R"), aminocarbonylamino (-NR-CO-NR'R"), pyridyl, piperidino, morpholino, and azetidinyl.

Examples of the secondary amino (-NH-R: mono-substituted amino) include alkylamino, cycloalkylamino, alkenylamino, alkynylamino, arylamino, heteroarylamino, and aralkylamino.

Examples of the tertiary amino (-NR(R'): di-substituted amino) include an amino group having arbitrary two substituents each independently selected from alkyl, cycloalkyl, alkenyl, alkynyl, aryl, heteroaryl, and aralkyl, for example, alkyl(aralkyl)amino. These arbitrary two substituents may form a ring. Examples thereof specifically include dialkylamino, particularly, $C_1$-$C_6$ dialkylamino, $C_1$-$C_4$ dialkylamino, dimethylamino, and diethylamino. In the present specification, the "$C_p$-$C_q$ dialkylamino group" refers to a group in which an amino group is substituted by two $C_p$-$C_q$ alkyl groups. The $C_p$-$C_q$ alkyl groups may be the same or different.

Examples of the substituted amidino (-C(=NR)-NR'R") include groups in which three substituents R, R', and R" on the N atoms are each independently selected from alkyl, cycloalkyl, alkenyl, alkynyl, aryl, heteroaryl, and aralkyl, for example, alkyl(aralkyl)(aryl)amidino.

Examples of the substituted guanidino (-NR-C(=NR‴)-NR'R") include groups in which R,R', R", and R‴ are each independently selected from alkyl, cycloalkyl, alkenyl, alkynyl, aryl, heteroaryl, and aralkyl, and groups in which these substituents form a ring.

Examples of the aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, and groups in which these substituents form a ring.

In the present specification, the "amino acid residue" constituting the peptide compound are sometimes referred to simply as an "amino acid".

The term "N-substituted amino acid" in the present specification means an amino acid in which the main chain amino group is N-substituted, among the amino acids defined above. Examples of the N-substituted amino acid specifically include those in which the main chain amino group of the amino acid is a NHR group and R is any group other than hydrogen, for example, an optionally substituted alkyl group, alkenyl group, alkynyl group, aryl group, heteroaryl group, aralkyl group or cycloalkyl group, and cyclic amino acids in which a carbon atom bonded to a N atom and a carbon atom at position $\alpha$ form a ring as in proline). Examples thereof include prolines that form a 5-membered ring, azetidines that form a 4-membered ring, and piperidines that form a 6-membered ring. The substituent group for each optionally substituted group is not particularly limited, and examples thereof include halogen groups, ether groups, and hydroxyl groups.

The "amino acid" in the present specification includes all corresponding isotopes to each. The isotope of "amino acid" is one in which at least one atom is substituted with an atom having the same atomic number (the same number of protons) and a different mass number (different sum of numbers of protons and neutrons). Examples of the isotope included in the "amino acid" herein include a hydrogen atom, a carbon atom, a nitrogen atom, an oxygen atom, a phosphorus atom, a sulfur atom, a fluorine atom, a chlorine atom, and the like, and they include $^{2}H$, $^{3}H$; $^{13}C$, $^{14}C$; $^{15}N$; $^{17}O$, $^{18}O$; $^{32}P$; $^{35}S$; $^{18}F$; $^{36}Cl$ and the like, respectively.

The term "proline" in the present specification means proline, as well as a group of compounds in which one or more arbitrary substituents are bonded on any carbon atom forming a 5-membered ring of proline. When a plurality of substituents are present on the 5-membered ring, the substituents may together form a ring, and the ring may be any aromatic ring. Examples of the "proline" include proline, trans-4-hydroxy-L-proline, cis-4-hydroxy-L-proline, trans-4-fluoro-L-proline, cis-4-fluoro-L-proline, and 2-methyl-L-proline. When the proline has a hydroxy group, the hydroxy group may be protected with any protective group, or any substituent and an oxygen atom of the hydroxy group may form an ether bond or an ester bond. Further, any one of carbon atoms forming a 5-membered ring may be replaced by an oxygen atom or a sulfur atom. Examples of such a compound specifically include L-thioproline. Further, the proline may

have an unsaturated bond in the 5-membered ring formed. Examples of such a compound specifically include 3,4-dehydro-L-proline. They are not necessarily in a L-form, and may be in a D-form.

In the present specification, the term "azetidine" means a cyclic amino acid based on a compound in which one nitrogen atom and three carbon atoms form a 4-membered ring, and a carboxyl group is bonded to a carbon atom next to the nitrogen atom. One or more arbitrary substituents may be bonded on any carbon atom forming a 4-membered ring. When a plurality of substituents are present on the 4-membered ring, the substituents may together form a ring. Examples of the "azetidine" specifically include (S)-azetidine-2-carboxylic acid, and (R)-azetidine-2-carboxylic acid. The "azetidine" also includes compounds in which a carboxyl group is bonded to a carbon atom that is next but one to the nitrogen atom forming the 4-membered ring. Examples of such a compound include azetidine-3-carboxylic acid.

In the present specification, the term "piperidine" means a cyclic amino acid based on a compound in which one nitrogen atom and five carbon atoms form a 6-membered ring, and a carboxyl group is bonded to a carbon atom next to the nitrogen atom. One or more arbitrary substituents may be bonded on any carbon atom forming a 6-membered ring. When a plurality of substituents are present on the 6-membered ring, the substituents may together form a ring, and the ring may be any aromatic ring. Examples of the "piperidine" specifically include (R)-piperidine-2-carboxylic acid, (S)-piperidine-2-carboxylic acid, (R)-1,2,3,4-tetrahydroisoquinoline-3-carboxylic acid, and (S)-1,2,3,4-tetrahydroisoquinoline-3-carboxylic acid.

In the present specification, examples of the aromatic hydrocarbon solvent include benzene, toluene, xylene, chlorobenzene, 1,2-dichlorobenzene, bromobenzene, anisole, ethylbenzene, nitrobenzene, and cumene.

In the present specification, examples of the halogen solvent include dichloromethane, chloroform, 1,2-dichloroethane, and carbon tetrachloride.

In the present specification, examples of the ether solvent include diethyl ether, tetrahydrofuran, 2-methyltetrahydrofuran, 1,4-dioxane, 1,3-dioxane, 1,2-dimethoxyethane, diisopropyl ether, cyclopentyl methyl ether, t-butyl methyl ether, 4-methyltetrahydropyran, diglyme, triglyme, and tetraglyme.

In the present specification, examples of the amide solvent include N,N-dimethylformamide (DMF), N-methylpyrrolidone (NMP), N,N-dimethylacetamide (DMA), Methylpyrrolidone (NEP), N-butylpyrrolidone (NBP), and formamide.

In the present specification, examples of the sulfoxide solvent include dimethyl sulfoxide (DMSO), and methyl phenyl sulfoxide.

In the present specification, examples of the sulfone solvent include diphenyl sulfone, dimethyl sulfone, diethyl sulfone, sulfolane, 3-methylsulfolane, ethyl methyl sulfone, and ethyl isopropyl sulfone.

In the present specification, examples of the urea solvent include 1,3-dimethyl-2-imidazolidinone (DMI), and 1,3-dimethyl-3,4,5,6-tetrahydro-2(1H)-pyrimidinone (DMPU).

In the present specification, examples of the ester solvent include methyl acetate, ethyl acetate, butyl acetate, methyl propionate, butyl acetate, propyl acetate, isopropyl acetate, isobutyl acetate, pentyl acetate, and $\gamma$-valerolactone.

In the present specification, examples of the ketone solvent include acetone, methyl ethyl ketone, methyl isobutyl ketone, cyclohexanone, cyclopentanone, and diethyl ketone.

Examples of the carbonate solvent include dimethyl carbonate, diethyl carbonate, and dibutyl carbonate.

Examples of the phosphoric acid ester solvent include trimethyl phosphate, triethyl phosphate, and tributyl phosphate.

In the present specification, the term "amidine" means a base represented by the following formula B1:

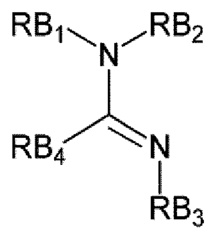

(B1)

wherein

$RB_1$ and $RB_4$ are each independently $C_1$-$C_4$ alkyl, or $RB_1$ and $RB_4$ form a 5- to 8-membered ring together with a nitrogen atom bonded to $RB_1$ and a carbon atom bonded to $RB_4$.

$RB_2$ and $RB_3$ are each independently $C_1$-$C_4$ alkyl, or $RB_2$ and $RB_3$ form a 5- to 8-membered ring together with a nitrogen atom bonded to $RB_2$ and a nitrogen atom bonded to $RB_3$ and a carbon atom bonded to the nitrogen atom.

When $RB_1$ to $RB_4$ are $C_1$-$C_4$ alkyl, the $C_1$-$C_4$ alkyl is preferably methyl or ethyl.

When $RB_1$ and $RB_4$ form a 5- to 8-membered ring, the 5- to 8-membered ring is preferably a pyrrolidine ring,

a piperidine ring, an azepane ring or the like.

When $RB_2$ and $RB_3$ form a 5- to 8-membered ring, the 5- to 8-membered ring is preferably a 1,4,5,6-tetrahydropyrimidine ring or the like.

Examples of the amidine specifically include DBU: 1,8-diazabicyclo[5.4.0]-7-undecene and DBN: 1,5-diazabicyclo[4.3.0]-5-nonene.

In the present specification, the term "guanidine" means a base represented by the following formula B2:

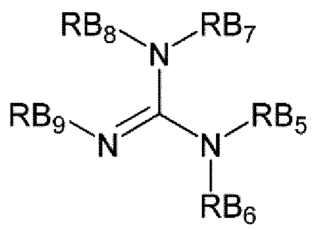

(B2)

wherein

- $RB_6$ is hydrogen or $C_1$-$C_4$ alkyl,
- $RB_5$ and $RB_7$ are each independently $C_1$-$C_4$ alkyl, or form a 5- to 8-membered ring together with nitrogen atoms bonded to $RB_5$ and $RB_7$ and carbon atoms bonded to the nitrogen atoms, and
- $RB_8$ is $C_1$-$C_4$ alkyl, or $RB_9$ is hydrogen, $C_1$-$C_4$ alkyl or phenyl, or $RB_8$ and $RB_9$ form a 5-to 8-membered ring together with nitrogen atoms bonded to $RB_8$ and $RB_9$ and carbon atoms bonded to the nitrogen atoms,
- where, when $RB_9$ is phenyl, the two B2 formulae may have naphthalene formed by condensation of the benzene rings of the phenyl group.

When $RB_5$ to $RB_8$ are $C_1$-$C_4$ alkyl, the $C_1$-$C_4$ alkyl is preferably methyl, and when $RB_9$ is $C_1$-$C_4$ alkyl, the $C_1$-$C_4$ alkyl is preferably t-butyl.

When $RB_5$ and $RB_7$ form a 5- to 8-membered ring, the 5- to 8-membered ring is preferably an imidazolidine ring, a hexahydropyrimidine ring, 1,3-diazepane ring or the like.

When $RB_8$ and $RB_9$ form a 5- to 8-membered ring, the 5- to 8-membered ring is preferably a 1,4,5,6-tetrahydropyrimidine ring or the like.

Examples of the guanidine specifically include TMG: 1,1,3,3-tetramethylguanidine, TBD: 1,5,7-triazabicyclo[4.4.0]deca-5-ene, and MTBD: 7-methyl-1,5,7-triazabicyclo[4.4.0]deca-5-ene.

In the present specification, the term "phosphazene" means a base represented by the following formula B3 or the following formula B4:

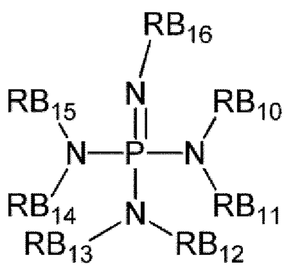

(B3)

wherein

- $RB_{10}$ is $C_1$-$C_4$ alkyl, or $RB_{10}$ and $RB_{11}$ form a 5- to 8-membered ring together with a nitrogen atom bonded to $RB_{10}$ and $RB_{11}$,
- $RB_{11}$ is $C_1$-$C_4$ alkyl unless $RB_{10}$ and $RB_{11}$ form a 5- to 8-membered ring, or $RB_{11}$ and $RB_{12}$ form a 5- to 8-membered ring together with nitrogen atoms bonded to $RB_{10}$ and $RB_{11}$ and a phosphorus atom bonded to the nitrogen atoms,
- $RB_{12}$ is $C_1$-$C_4$ alkyl unless $RB_{11}$ and $RB_{12}$ form a 5- to 8-membered ring, or $RB_{12}$ and $RB_{13}$ form a 5- to 8-membered ring together with a nitrogen atom bonded to $RB_{12}$ and $RB_{13}$,
- $RB_{13}$ is $C_1$-$C_4$ alkyl unless $RB_{12}$ and $RB_{13}$ form a 5- to 8-membered ring, or $RB_{13}$ and $RB_{14}$ form a 5- to 8-membered ring together with nitrogen atoms bonded to $RB_{13}$ and $RB_{14}$ and a phosphorus atom bonded to the nitrogen atoms,
- $RB_{14}$ is $C_1$-$C_4$ alkyl unless $RB_{13}$ and $RB_{14}$ form a 5- to 8-membered ring, or $RB_{14}$ and $RB_{15}$ form a 5- to 8-membered

ring together with a nitrogen atom bonded to $RB_{14}$ and $RB_{15}$,
$RB_{15}$ is $C_1$-$C_4$ alkyl unless $RB_{14}$ and $RB_{15}$ form a 5- to 8-membered ring, and
$RB_{16}$ is hydrogen, $C_1$-$C_8$ alkyl or $C_6$-$C_{10}$ aryl.

When $RB_{10}$ to $RB_{15}$ are $C_1$-$C_4$ alkyl, the $C_1$-$C_4$ alkyl is preferably methyl or ethyl, and when $RB_{16}$ is $C_1$-$C_8$ alkyl, the $C_1$-$C_8$ alkyl is preferably t-butyl or t-octyl.

When $RB_{10}$ and $RB_{11}$, $RB_{12}$ and $RB_{13}$, and/or $RB_{14}$ and $RB_{15}$ form a 5- to 8-membered ring, the 5- to 8-membered ring is preferably a pyrrolidine ring, a piperidine ring, an azepane ring or the like.

$RB_{11}$ and $RB_{12}$, and/or $RB_{13}$ and $RB_{14}$ form a 5- to 8-membered ring, the 5- to 8-membered ring is preferably a 5- to 8-membered saturated ring which does not contain hetero atoms other than nitrogen atoms bonded to $RB_{11}$, $RB_{12}$, $RB_{13}$ and $RB_{14}$ and a phosphorus atom bonded to the nitrogen atoms.

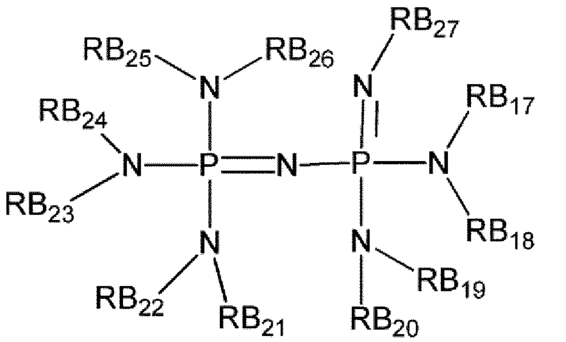

(B4)

wherein

$RB_{17}$ is independently $C_1$-$C_4$ alkyl, or $RB_{17}$ and $RB_{18}$ form a 5- to 8-membered ring together with a nitrogen atom bonded to $RB_{17}$ and $RB_{18}$,
$RB_{18}$ is $C_1$-$C_4$ alkyl unless $RB_{17}$ and $RB_{18}$ form a 5- to 8-membered ring, or $RB_{18}$ and $RB_{19}$ form a 5- to 8-membered ring together with nitrogen atoms bonded to $RB_{18}$ and $RB_{19}$ and a phosphorus atom bonded to the nitrogen atoms,
$RB_{19}$ is $C_1$-$C_4$ alkyl unless $RB_{18}$ and $RB_{19}$ form a 5- to 8-membered ring, or $RB_{19}$ and $RB_{20}$ form a 5- to 8-membered ring together with a nitrogen atom bonded to $RB_{19}$ and $RB_{20}$,
$RB_{20}$ is $C_1$-$C_4$ alkyl unless $RB_{19}$ and $RB_{20}$ form a 5- to 8-membered ring,
$RB_{21}$ is $C_1$-$C_4$ alkyl, or $RB_{21}$ and $RB_{22}$ form a 5- to 8-membered ring together with a nitrogen atom bonded to $RB_{21}$ and $RB_{22}$,
$RB_{22}$ is $C_1$-$C_4$ alkyl unless $RB_{21}$ and $RB_{22}$ form a 5- to 8-membered ring, or $RB_{22}$ and $RB_{23}$ form a 5- to 8-membered ring together with nitrogen atoms bonded to $RB_{22}$ and $RB_{23}$ and a phosphorus atom bonded to the nitrogen atoms,
$RB_{23}$ is $C_1$-$C_4$ alkyl unless $RB_{22}$ and $RB_{23}$ form a 5- to 8-membered ring, or $RB_{23}$ and $RB_{24}$ form a 5- to 8-membered ring together with a nitrogen atom bonded to $RB_{23}$ and $RB_{24}$,
$RB_{24}$ is $C_1$-$C_4$ alkyl unless $RB_{23}$ and $RB_{24}$ form a 5- to 8-membered ring, or $RB_{24}$ and $RB_{25}$ form a 5- to 8-membered ring together with nitrogen atoms bonded to $RB_{24}$ and $RB_{25}$ and a phosphorus atom bonded to the nitrogen atoms,
$RB_{25}$ is $C_1$-$C_4$ alkyl unless $RB_{24}$ and $RB_{25}$ form a 5- to 8-membered ring, or $RB_{25}$ and $RB_{26}$ form a 5- to 8-membered ring together with a nitrogen atom bonded to $RB_{25}$ and $RB_{26}$,
$RB_{26}$ is $C_1$-$C_4$ alkyl unless $RB_{25}$ and $RB_{26}$ form a 5- to 8-membered ring,
$RB_{27}$ is $C_1$-$C_4$ alkyl or $C_6$-$C_{10}$ aryl.

When $RB_{17}$ to $RB_{26}$ are $C_1$-$C_4$ alkyl, the $C_1$-$C_4$ alkyl is preferably methyl or ethyl, and when $RB_{27}$ is $C_1$-$C_4$ alkyl, the $C_1$-$C_4$ alkyl is preferably ethyl or t-butyl.

When $RB_{17}$ and $RB_{18}$, $RB_{19}$ and $RB_{20}$, $RB_{21}$ and $RB_{22}$, $RB_{23}$ and $RB_{24}$, and/or $RB_{25}$ and $RB_{26}$ form a 5- to 8-membered ring, the 5- to 8-membered ring is preferably a pyrrolidine ring, a piperidine ring, an azepane ring or the like.

When both $RB_{17}$ and $RB_{18}$ are $C_1$-$C_4$ alkyl, it is preferred that both $RB_{19}$ and $RB_{20}$ be $C_1$-$C_4$ alkyl, and it is preferred that $RB_{17}$ and $RB_{18}$ form a 5- to 8-membered ring and $RB_{19}$ and $RB_{20}$ also form a 5- to 8-membered ring.

When both $RB_{21}$ and $RB_{22}$ are $C_1$-$C_4$ alkyl, it is preferred that all of $RB_{23}$, $RB_{24}$, $RB_{25}$ and $RB_{26}$ be $C_1$-$C_4$ alkyl, and it is preferred that $RB_{21}$ and $RB_{22}$ form a 5- to 8-membered ring and $RB_{23}$ and $RB_{24}$, and $RB_{25}$ and $RB_{26}$ also form 5- to 8-membered rings, respectively.

$RB_{18}$ and $RB_{19}$, and/or $RB_{22}$ and $RB_{23}$ form a 5- to 8-membered ring, the 5- to 8-membered ring is preferably a 5- to 8-membered saturated ring which does not contain hetero atoms other than nitrogen atoms bonded to $RB_{11}$, $RB_{12}$, $RB_{13}$ and $RB_{14}$ and a phosphorus atom bonded to the nitrogen atoms.

Specific examples of the phosphazene include P2tBu: 1-tert-butyl-2,2,4,4,4-pentakis(dimethylamino)-2$\lambda^5$,4$\lambda^5$-catenadi(phosphazene), P2Et: tetramethyl(tris(dimethylamino)phosphoranylidene)phosphoric triamide-ethylimine, HP1 (dma): imino-tris(dimethylamino)phospholane, BTPP: tert-butylimino-tri(pyrrolidino)phosphorane, P1tBu: tert-butylimino-tris(dimethylamino)phosphorane, and BEMP: 2-tert-butylamino-2-diethylamino-1,3-dimethylperhydro-1,3,2-diazaphospholine.

In the present specification, the term "and/or" is meant to include every combination of the terms "and" and "or" appropriately combined. Specifically, for example, the term "A, B and/or C" includes the following seven variations: (i) A, (ii) B, (iii) C, (iv) A and B, (v) A and C, (vi) B and C, and (vii) A, B and C.

(Production method)

The present invention relates to a method for producing a peptide by a solid-phase method, comprising the following steps (1) to (3):

(1) providing a first peptide having a protective group containing an Fmoc skeleton and supported on a solid-phase synthesis resin;
(2) treating the first peptide with one or more bases including at least a base having a pKa of 23 or more in acetonitrile as a conjugate acid in a solvent containing at least one selected from the group consisting of an aromatic hydrocarbon solvent, a halogen solvent, an ether solvent, an ester solvent, a ketone solvent, a carbonate solvent and a phosphoric acid ester solvent after the step (1); and
(3) condensing the first peptide with a carboxylic acid or a carboxylic acid analog in a solvent in the presence or absence of a condensation agent to obtain a third peptide after the step (2).

Step (1)

Step (1) in the present invention is a step of providing a first peptide having a protective group containing an Fmoc skeleton and supported on a solid-phase synthesis resin;

The "first peptide having a protective group containing an Fmoc skeleton and supported on a solid-phase synthesis resin" in step (1) is typically supported on a solid-phase synthesis resin through a functional group (e.g. carboxyl group) in an amino acid residue at the C-terminal thereof, but may be supported on a solid-phase synthesis resin through a functional group (e.g. carboxyl group) in an amino acid residue other than one at the C-terminal. The amino group of an amino acid residue at the N-terminal of the "first peptide having a protective group containing an Fmoc skeleton and supported on a solid-phase synthesis resin" is protected with a protective group containing an Fmoc skeleton.

As the solid-phase synthesis resin on which the first peptide is supported, any solid-phase synthesis resin known in the relevant art can be used, and in general, the resin is linked to a predetermined amino acid residue of the first peptide (e.g. amino acid residue at the C-terminal). It is particularly preferred that the resin have a resin bonding group graded at "H (< 5% TFA in DCM)" as acid sensitivity described in Solid-Phase Synthesis Handbook (issued by Merck, published on 1 May, 2002), and the resin can be appropriately selected according to a functional group on the side of an amino acid used.

For example, when a carboxylic acid (a main chain carboxylic acid or a side chain carboxylic acid typified by Asp or Glu) or a hydroxy group on an aromatic ring (a phenol group typified by Tyr) is used as the functional group on the amino acid side, it is preferred to use trityl chloride resin (Trt resin) or 2-chlorotrityl chloride resin (Clt resin) as the resin. When an aliphatic hydroxy group (an aliphatic alcohol group typified by Ser or Thr) is used as the functional group on the amino acid side, it is preferred to use trityl chloride resin (Trt resin), 2-chlorotrityl chloride resin (Clt resin) or 4-methyltrityl chloride resin (Mtt resin) as the resin.

In the present invention, the "protective group containing an Fmoc skeleton" means a group in which any substituent is introduced at any position on an Fmoc group or a skeleton constituting the Fmoc group. Examples of such a protective group containing an Fmoc skeleton specifically include protective groups represented by the following formula (1):

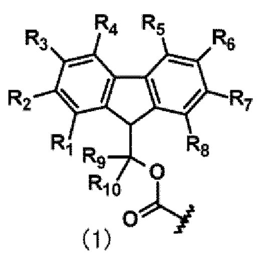

(1)

wherein

$R_1$ to $R_8$ are independently selected from the group consisting of hydrogen, $C_1$-$C_8$ alkyl, $C_1$-$C_8$ fluoroalkyl, halogen, sulfo and trimethylsilyl; and
$R_9$ to $R_{10}$ are independently hydrogen or methyl.

Examples of the protective group containing an Fmoc skeleton more specifically include a 9-fluorenylmethyloxycarbonyl (Fmoc) group, a 2,7-di-tert-butyl-Fmoc (Fmoc (2,7tb)) group, a 1-methyl-Fmoc (Fmoc (1Me)) group, a 2-fluoro-Fmoc (Fmoc (2F)) group, a 2,7-dibromo-Fmoc (Fmoc (2,7Br)) group, a 2-monoisooctyl-Fmoc (mio-Fmoc) group, a 2,7-diisooctyl-Fmoc (dio-Fmoc) group, a 2,7-(3,3,4,4,5,5,6,6,7,7,8,8,8-tridecafluorooctyl)-Fmoc (tdf-Fmoc) group, a 2,7-bis(trimethylsilyl)-Fmoc (Fmoc (2TMS)) group, a (2-sulfo-9H-fluoren-9-yl)methoxycarbonyl group (Fmoc (2so3h)), a [(1S)-1-(9H-fluoren-9-yl)ethoxy]carbonyl group (sm-Fmoc), and a [(1R)-1-(9H-fluoren-9-yl)ethoxt]carbonyl group (rm-Fmoc). Such a protective group containing an Fmoc skeleton is preferably an Fmoc group. Such a protective group containing an Fmoc skeleton can be introduced by a known method using a commercially available reagent or the like.

In the present invention, the types and the number of amino acid residues constituting the first peptide are not limited, and a peptide having any amino acid sequence including 2 or more amino acid residues, for example, 2 to 30, 2 to 20, 2 to 15, 14, 13, 12, 11, 10, 9, 8, 7, 6, 5, 4, 3 or 2 amino acid residues can be used as the first peptide. For the amino acids, natural amino acids and/or non-natural amino acids can be used. It is preferred that the first peptide contain one or more N-substituted amino acids.

The first peptide having a protective group containing an Fmoc skeleton and supported on a solid-phase synthesis resin, which is provided in step (1), can be obtained from a commercial supplier, or produced by applying a method known in the relevant art, for example, a method shown in WO 2013/100132 or WO 2018/225864.

In certain aspect, the first peptide is a dipeptide. In this case, the amino acid residue at the C-terminal of the dipeptide is typically supported on a solid-phase synthesis resin through an ester bond, and as shown in the following scheme, a free amino group resulting from removal of a protective group for the amino acid residue at the N-terminal may react with a carbonyl group in the ester bond, leading to formation of undesirable DKP. By using the method of the present invention, a decrease in yield of a target peptide due to DKP elimination can be suppressed.

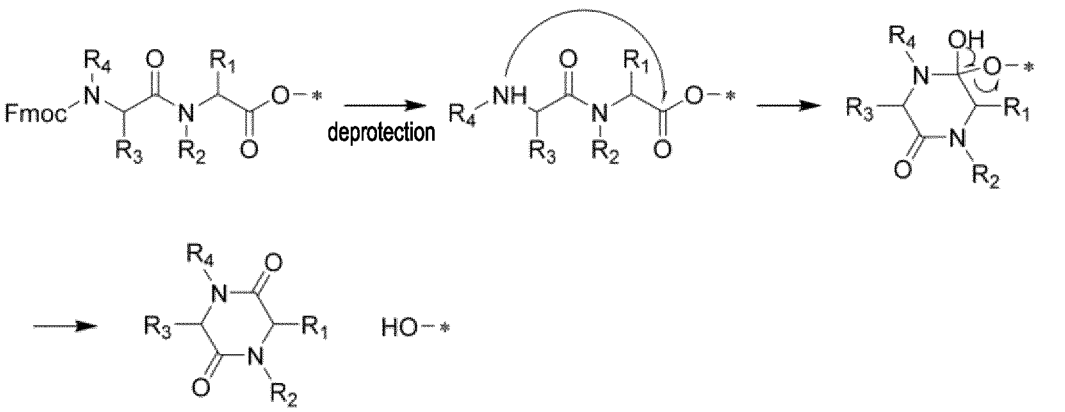


DKP

wherein $R_1$ to $R_4$ each represent any atom or group, and * represents a position of bonding to a solid-phase synthesis resin.

In certain aspects, the first peptide is a peptide in which the amino acid at the second residue from the N-terminal thereof is a "N-substituted amino acid". In this case, as shown in the following scheme, a free amino group resulting from removal of a protective group for the amino acid residue at the N-terminal may react with a carbonyl group in a peptide bond between the amino acids at the second residue and the third residue from the N-terminal, so that DKP, a DKP elimination compound, or an impurity having an amidine skeleton (6-membered cyclic amidine skeleton compound), which is undesirable, may be formed. By using the method of the present invention, formation of the DKP, DKP elimination compound and 6-membered cyclic amidine skeleton compound can be suppressed to prevent a decrease in yield of a target peptide.

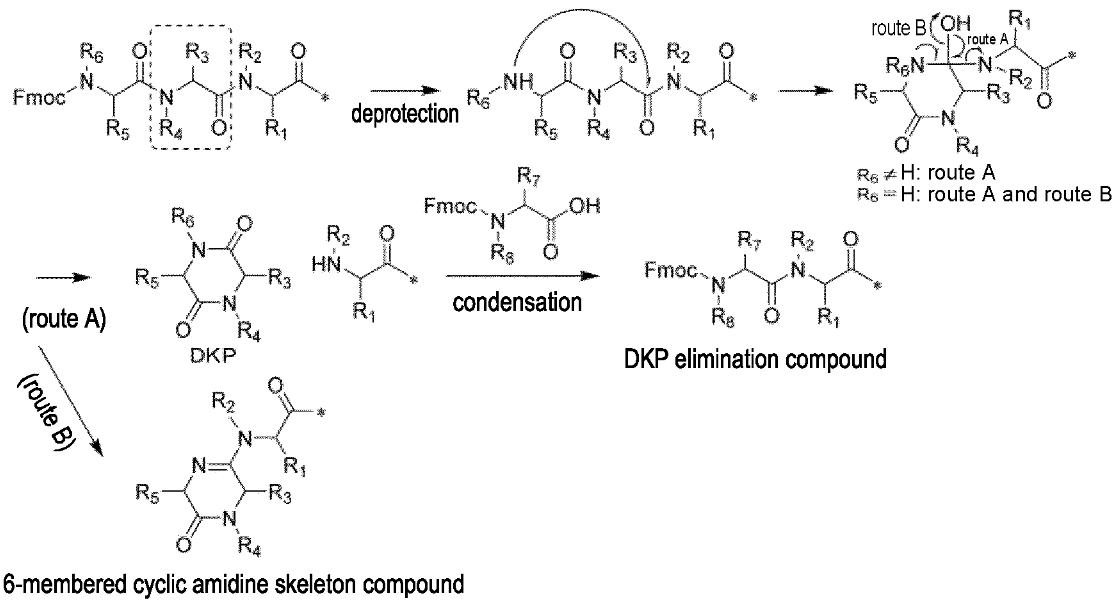


wherein $R_4$ represents any atom or group other than hydrogen, $R_1$ to $R_3$ and $R_5$ to $R_8$ each represent any atom or group, and * represents a position of bonding to an adjacent amino acid residue or solid-phase synthesis resin, where it is possible to pass through any of route A and route B in the scheme when $R_6$ is hydrogen, and it is possible to pass through only route A in the scheme when $R_6$ is not hydrogen.

As the "N-substituted amino acid" in the present invention, N-alkylamino acids obtained by N-alkylation such as N-methylation, and cyclic amino acids such as prolines and azetidines are preferably exemplified.

Step (2)

Step (2) in the present invention is a step of treating the first peptide provided in step (1] with one or more bases including at least a base having a pKa of 23 or more in acetonitrile as a conjugate acid in a solvent containing at least one selected from the group consisting of an aromatic hydrocarbon solvent, a halogen solvent, an ether solvent, an ester solvent, a ketone solvent, a carbonate solvent and a phosphoric acid ester solvent.

Step (2) in the present invention is a step involved in removal of a protective group for the amino acid residue at the N-terminal. Without being bound by a specific theory, the DKP elimination compound and the 6-membered cyclic amidine skeleton compound may be formed through a 6-membered ring intermediate as described above. The result of performing calculation of energy until DKP elimination through a 6-membered ring intermediate shows that the higher the hydrogen receiving capacity of a solvent, the greater the stabilization by coordination, and the donor number (DN) value as an index of the unshared electron pair donating property of the solvent may be an important parameter. When the solvent is a solvent having a DN value of 26 or less, or contains such a solvent, the reaction through a 6-membered ring intermediate may tend to be suppressed, leading to reduced formation of a DKP elimination compound and a 6-membered cyclic amidine skeleton compound. Therefore, the solvent for use in step (2) is preferably one having a donor number (DN) value of 26 or less. In certain aspects, the DN value can be 26 or less, 25 or less, 24 or less, 23 or less, 22 or less, 21 or less, 20 or less, 19 or less, 18 or less, 17 or less, 16 or less, 15 or less, 14 or less, 13 or less, 12 or less, 11 or less, 10 or less, 9 or less, 8 or less, 7 or less, 6 or less, 5 or less, 4 or less, 3 or less, 2 or less, or 1 or less. As an index of the DN value of each solvent, a value described in "Eur. Chem. Bull., 2015, 4(2), 92-97" can be adopted. In addition, values in a table described in "Japanese Patent Laid-Open No. 2010-50089", a table described in "Encyclopedic Dictionary of Chemistry 1989, 1584-1585" or the like can be adopted. In addition, the DN value can be measured by a method described in "Journal of the American Chemical Society 1972, 94(5), 1431-1434" or "Chemistry A European Journal 2012, 18(35), 10969-10982". Further, the DN value of a mixed solvent can be measured by a method described in "Chemistryselect 2021, 6(4), 600-608". Specific DN values are as follows: toluene: 0.1; DCM: 1; cumene: 6; methyl propionate: 11; sulfolane: 14.8; diethyl ketone: 15; butyl acetate: 15; propyl acetate: 16; diethyl carbonate: 16; acetone: 17; dimethyl carbonate: 17.2; methyl ethyl ketone: 17.4; 2-methyltetrahydrofuran: 18; diethyl ether: 19.2; tetrahydrofuran: 20; 1,2-dimethoxyethane: 20; 1,3-dioxolane: 21.2; trimethyl phosphate: 23; tributyl phosphate: 23.7; DMF: 26.6; NMP: 27.3; DMA: 27.8; and DMI: 27.8.

In certain aspects, at least a part of the first peptide obtained in step (2) is in the form of a carbamic acid salt. Without being bound by a specific theory, in step (2), dibenzofulvene is formed without progress of decarboxylation in

at least a part of the first peptide in removal of a protective group containing an Fmoc skeleton, the amino group at the N-terminal thus forms a carbamate ion (-NRC(=O)O$^-$, where R is hydrogen or any substituent of an amino group) with COO$^-$ derived from the protective group, and the carbamate ion forms a carbamic acid salt with a protonated base in the system (below is a scheme showing formation of a carbamic acid salt when Fmoc is used as a protective group containing an Fmoc skeleton and DBU is used as a base).

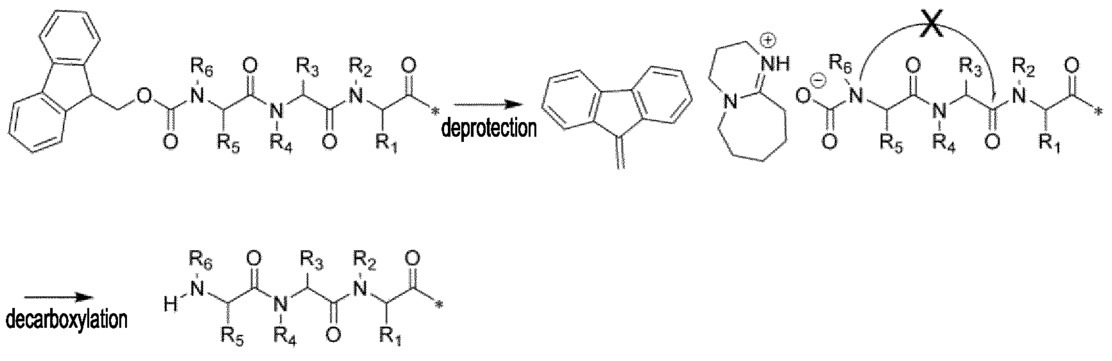


When a carbamic acid salt is formed, an intramolecular nucleophilic reaction of a terminal amino group can be suppressed, so that even under basic deprotection conditions, formation of a 6-membered ring intermediate is suppressed to reduce formation of a DKP elimination compound and a 6-membered cyclic amidine skeleton compound as side products.

In the method of the present invention in which a base having a pKa of 23 or more in acetonitrile as a conjugate acid is used in step (2), the first peptide may be present as a carbamic acid salt which enables significant reduction of formation of a DKP elimination compound and a 6-membered cyclic amidine skeleton compound. For example, in the method of the present invention, a carbamic acid salt may be formed in an amount such that the molar ratio of the carbamic acid salt to an amine form additionally formed with progress of decarboxylation (carbamic acid salt/amine) is 0.6 or more, 0.8 or more, 1.0 or more, 2.0 or more, 3.0 or more, 4.0 or more, 4.6 or more, 5.0 or more, 6.0 or more, 8.0 or more, or 10.0 or more. Such a molar ratio can be determined from, for example, an integral ratio of protons in $^1$H-NMR.

The carbamic acid salt is a salt with any base having a pKa of 23 or more in acetonitrile as a conjugate acid. Examples of such a salt specifically include DBU salts, TMG salts, HP1 (dma) salts, MTBD salts, P1-tBu salts, and P2-Et salts.

In certain aspects, the solvent for use in the step (2) may contain at least one of an aromatic hydrocarbon solvent, a halogen solvent, an ether solvent, an ester solvent, a ketone solvent, a carbonate solvent and a phosphoric acid ester solvent, and may be a single solvent or a mixed solvent. In the case of a mixed solvent, it is preferred that the solvent contain an aromatic hydrocarbon solvent, a halogen solvent, an ether solvent, an ester solvent, a ketone solvent, a carbonate solvent and a phosphoric acid ester solvent in a total amount of 25 v/v% or more, 50 v/v% or more, or 75 v/v% or more with respect to the total amount of the solvent, and it is more preferred that the solvent contain at least one selected from the group consisting of an aromatic hydrocarbon solvent, a halogen solvent, an ether solvent, an ester solvent, a ketone solvent, a carbonate solvent and a phosphoric acid ester solvent at 25 v/v% or more, 50 v/v% or more, or 75 v/v% or more.

When an aromatic hydrocarbon solvent is used in step (2), examples of the solvent specifically include benzene, toluene, xylene, chlorobenzene, 1,2-dichlorobenzene, bromobenzene, anisole, ethylbenzene, nitrobenzene, and cumene, with toluene and cumene being preferred.

When a halogen solvent is used in step (2), examples of the solvent specifically include dichloromethane, chloroform, 1,2-dichloroethane, and carbon tetrachloride, with dichloromethane being preferred.

When an ether solvent is used in step (2), examples of the solvent specifically include diethyl ether, tetrahydrofuran, 2-methyltetrahydrofuran, 1,4-dioxane, 1,3-dioxane, 1,2-dimethoxyethane (DME), diisopropyl ether, cyclopentyl methyl ether (CPME), t-butyl methyl ether, 4-methyltetrahydropyran, diglyme, triglyme, and tetraglyme, with tetrahydrofuran, 2-methyltetrahydrofuran, 1,2-dimethoxyethane and 1,3-dioxolane being preferred.

When an ester solvent is used in step (2), examples of the solvent specifically include methyl acetate, ethyl acetate, butyl acetate, methyl propionate, butyl acetate, propyl acetate, isopropyl acetate, isobutyl acetate, pentyl acetate, and γ-valerolactone, with butyl acetate and methyl propionate being preferred.

When a ketone solvent is used in step (2), examples of the solvent specifically include acetone, methyl ethyl ketone (MEK), methyl isobutyl ketone, cyclohexanone, cyclopentanone, and diethyl ketone (3-pentanone), with methyl ethyl ketone and diethyl ketone being preferred.

When a carbonate solvent is used in step (2), examples of the solvent specifically include dimethyl carbonate,

diethyl carbonate, and dibutyl carbonate, with dimethyl carbonate being preferred.

When a phosphoric acid ester solvent is used in step (2), examples of the solvent specifically include trimethyl phosphate, triethyl phosphate, and tributyl phosphate, with tributyl phosphate being preferred.

When the solvent for use in step (2) is a mixed solvent, the mixed solvent may include at least one or two or more solvents selected from an aromatic hydrocarbon solvent, a halogen solvent, an ether solvent, an ester solvent, a ketone solvent, a carbonate solvent and a phosphoric acid ester solvent, or may contain one or more solvents selected from an amide solvent, a urea solvent or a sulfone solvent in addition to an aromatic hydrocarbon solvent, a halogen solvent, an ether solvent, an ester solvent, a ketone solvent, a carbonate solvent and a phosphoric acid ester solvent.

When an amide solvent is used in step (2), examples of the solvent specifically include N,N-dimethylformamide (DMF), N-methylpyrrolidone (NMP), N,N-dimethylacetamide (DMA), M-ethylpyrrolidone (NEP), N-butylpyrrolidone (NBP), and formamide.

When a urea solvent is used in step (2), examples of the solvent specifically include 1,3-dimethyl-2-imidazolidinone (DMI), and 1,3-dimethyl-3,4,5,6-tetrahydro-2(1H)-pyrimidinone (DMPU).

When a sulfone solvent is used in step (2), examples of the solvent specifically include diphenyl sulfone, dimethyl sulfone, diethyl sulfone, sulfolane, 3-methylsulfolane, ethyl methyl sulfone, and ethyl isopropyl sulfone.

When the solvent for use in step (2) is a single solvent, the solvent is preferably toluene, cumene, 1,2-dichlorobenzene, benzene, anisole, dichloromethane, chloroform, 1,2-dichloroethane, tetrahydrofuran, 2-methyltetrahydrofuran, cyclopentyl methyl ether, 4-methyltetrahydropyran, 1,4-dioxane, 1,2-dimethoxyethane, 1,3-dioxolane, ethyl acetate, isopropyl acetate, butyl acetate, methyl propionate, acetone, methyl ethyl ketone, diethyl ketone, dimethyl carbonate, diethyl carbonate, trimethyl phosphate, or tributyl phosphate.

In certain aspects, the solid-phase resin swelling capacity of the solvent for use in step (2) is preferably 1.5 mL/g or more, 2.0 mL/g or more, 2.5 mL/g or more, 3.0 mL/g or more, 3.5 mL/g or more, or 4.0 mL/g or more. As an index of the solid-phase resin swelling capacity of each solvent, a value described in "Tetrahedron Lett., 1998, 39, 8951-8954", "Org. Process Res Dev. 2018, 22, 494-503" or "RSC Adv., 2020, 10, 42457-42492" can be adopted. The solid-phase resin swelling capacity (mL/g) is specifically as follows: DMI: 8.5; DMPU: 8.0; NMP: 6.4; THF: 6.0; DMA: 5.8; $CHCl_3$: 5.6; CPME: 5.6; $CH_2Cl_2$: 5.4; 2-methyltetrahydrofuran: 5.4; DMF: 5.2; butyl acetate: 5.2; DME: 4.8; MEK: 4.4; toluene: 4.0; AcOEt: 4.2; xylene: 3.0; Et2O: 2.8; dimethyl carbonate: 2.8; MTBE: 2.4; $CH_3CN$: 2.0; tributyl phosphate: 1.6; MeOH: 1.6; and $H_2O$: 1.6. In addition, estimations in a table described in "Green Chem., 2017, 19, 952-962", table 2 in "Green Chem., 2020, 22, 996-1018" or the like can be adopted.

In step (2), one or more bases including at least a base having a pKa of 23 or more in acetonitrile as a conjugate acid is used. When only one base is used, the base is a base having a pKa of 23 or more in acetonitrile as a conjugate acid. On the other hand, when a plurality of bases are used, at least one of the bases is a base having a pKa of 23 in acetonitrile as a conjugate acid, and the other bases may be bases having a pKa od 23 or more, or less than 23 in acetonitrile as a conjugate acid.

The base having a pKa of 23 or more in acetonitrile as a conjugate acid for use in step (2) may be one having a pKa of 34 or less, preferably 30 or less, more preferably 28 or less.

The pKa is an index of the degree of basicity, and larger the pKa, the higher the basicity. As the pKa of the organic base in acetonitrile as a conjugate acid ($CH_3CN$) in the present invention, a value shown in "New J. Chem. 2009, 33, 588"[1)], "Sigma-Aldrich, Phosphazene Bases: https://www.sigmaaldrich.com/chemistry/chemical-synthesis/technology-spotlights/phosphazenes.html"[2)], or "Eur. J. Org. Chem. 2019, 6735-6748", or the like may be adopted.

Examples of the base having a pKa of 23 or more in acetonitrile as a conjugate acid include amidines, guanidines and phosphazenes, and for example, the bases listed below all have a pKa of 23 or more.

DBU: pKa[1)] = 24.3
DBN: pKa[1)] = 23.8
TMG: pKa[1)] = 23.3
TBD: pKa[1)] = 26.0
MTBD: pKa[1)] = 25.5
P2tBu: pKa[1)] = 33.5
P2Et: pKa[1)] = 32.9
HP1 (dma): pKa[1)] = 25.9
BTPP: pKa[2)] = 28.4
P1tBu: pKa[1)] = 27.0
BEMP: pKa[2)] = 27.6

When a plurality of bases having a pKa of 23 or more in acetonitrile as a conjugate acid are used in this step, for example, two or more, two bases selected from, for example, the amidine, guanidine and phosphazene can be used in combination. As specific combinations of such bases, combinations of DBU and MTBD, DBU and HP1 (dma), DBU

and P1tBu, DBU and P2Et, and the like are preferably exemplified.

When a base having a pKa of 23 or more and a base having a pKa of less than 23 in acetonitrile as a conjugate acid are used in combination in this step, the base having a pKa of 23 or more in acetonitrile as a conjugate acid can be selected from, for example, the amidine, guanidine and phosphazene. It is preferred that the base having a pKa of less than 23 in acetonitrile as a conjugate acid be selected from piperidine, piperazine and morpholine. As a specific combination of such bases, a combination of DBU and piperidine is preferably exemplified.

In certain aspects, step (2) can be repeated two or more times before step (3). In this case, in the repeats of step (2), the same solvent and/or the same base may be used, or different solvents and/or different bases may be used. In the present invention, the term "the same solvent" means that the solvents are identical in type and mixing ratio, and the term "different solvents" means that the solvents are different in one or both of type and mixing ratio. In the present invention, the term "the same base" means that the bases are identical in type and concentration of the base in the solvent, and the term "different bases" means that the bases are different in one or both of type and concentration of the base in the solvent.

In certain aspects, the method of the present invention may comprise the step of discharging a solution between the repeats of step (2) when step (2) is repeated two or more times. This enables replacement of a solvent and/or a base used in the immediately preceding repeat of step (2) by a solvent and/or a base to be used in the next repeat of step (2).

In certain aspects, the method of the present invention does not comprise the step of treating the first peptide with a single base having a pKa of less than 23 in acetonitrile as a conjugate acid, for example, piperidine before step (2). Specifically, the present invention may exclude an aspect in which the first peptide having a protective group containing an Fmoc skeleton and provided in step (1) is treated in any solvent (e.g. piperidine) using a base having a pKa of less than 23 in acetonitrile as a conjugate acid (e.g. piperidine), and subsequently, step (2) in the present invention is then carried out (e.g. treatment is performed with DBU or a combination of DBU and piperidine in a solvent containing toluene).

In certain aspects, the base for use in step (2) may be contained in a solvent at any concentration which enables the reaction of this step to proceed. Specifically, the concentration is in the range of, for example, 0.25 to 20 v/v%, 0.5 to 20 v/v%, 1 to 20 v/v%, 1 to 15 v/v%, 1 to 10 v/v%, 1 to 9 v/v%, 1 to 8 v/v%, 1 to 7 v/v%, 1 to 6 v/v%, 1 to 5 v/v%, 1 to 4 v/v%, 1 to 3 v/v%, or 1 to 2 v/v%, preferably 1 to 8 v/v%.

In certain aspects, step (2) can further comprise the step of bringing the solvent into contact with $CO_2$. In certain aspects, the solvent for use in step (2) can be a solvent brought into contact with $CO_2$ before the step. The solvent can be brought into contact with $CO_2$ by, for example, bubbling the solvent with $CO_2$. By bringing the solvent into contact with $CO_2$ during step (2) or before step (2), $CO_2$ can be incorporated into the reaction system. Without being bound by a specific theory, in step (2), the amino group at the N-terminal forms a carbamic acid salt with a base protonated with $COO^-$ derived from the protective group in at least a part of the first peptide, and by incorporating $CO_2$ into the reaction system, the amine form in the reaction system can be more easily converted into a carbamic acid salt. Therefore, by incorporating $CO_2$ into the reaction system, the amount of the amine form during the reaction can be further reduced to further suppress an intramolecular nucleophilic reaction of a terminal amino group, so that even under basic deprotection conditions, formation of a 6-membered ring intermediate is further suppressed to further reduce formation of a DKP elimination compound and a 6-membered cyclic amidine skeleton compound as side products.

In certain aspects, the reaction of step (2) can be carried out at -100°C to a temperature near a boiling point of the solvent, preferably 0 to 50°C. The reaction of step (2) can be carried out for 1 minute to 1 week, preferably 1 minute to 3 hours, 3 minutes to 3 hours or 5 minutes to 3 hours, more preferably 1 minute to 20 minutes, 3 minutes to 20 minutes or 5 minutes to 20 minutes.

Step (3)

Step (3) in the present invention is a step of condensing the first peptide with a carboxylic acid or a carboxylic acid analog in a solvent in the presence or absence of a condensation agent to obtain a third peptide after step (2). In this step, the amino group at the N-terminal of the first peptide is condensed with the carboxyl group of the carboxylic acid to synthesize a third peptide.

In certain aspects, the carboxylic acid for use in step (3) can be an amino acid having a protective group, a second peptide having a protective group, a $C_1$-$C_8$ alkylcarboxylic acid, or a $C_6$-$C_{10}$ arylcarboxylic acid.

In certain aspects, the carboxylic acid analog for use in step (3) can be an active ester of carboxylic acid, or an acid halide of carboxylic acid.

By using an active ester of an amino acid having a protective group, a second peptide having a protective group, a $C_1$-$C_8$ alkylcarboxylic acid, or a $C_6$-$C_{10}$ arylcarboxylic acid, the condensation reaction of step (3) can be carried out without a condensation agent. Examples of the active ester specifically include those in which a carbonyl group in the above-mentioned compound, particularly a carbonyl group contained in an amino acid residue at the C-terminal of the second peptide when the compound is the second peptide, is bonded to a group capable of accelerating a reaction

with amine, for example, an O-(benzotriazol-1-yl) group (OBt), an O-(7-azabenzotriazol-1-yl) group (OAt), a N-hydroxysuccinimide group (OSu), a pentafluorophenoxy group (OPfp), S-$R_{11}$ (wherein $R_{11}$ represents a hydrogen atom, an alkyl group optionally having a substituent, an aryl group optionally having a substituent, an aralkyl group optionally having a substituent, a cycloalkyl group optionally having a substituent, a heteroaryl group optionally having a substituent, an alkenyl group optionally having a substituent, or an alkylene group optionally having a substituent), or the like.

By using an acid halide of an amino acid having a protective group, a second peptide having a protective group, a $C_1$-$C_8$ alkylcarboxylic acid, or a $C_6$-$C_{10}$ arylcarboxylic acid, the condensation reaction of step (3) can be carried out without a condensation agent. Examples of the acid halide specifically include those in which a carboxyl group in the above-mentioned compound, particularly a carboxyl group contained in an amino acid residue at the C-terminal of the second peptide when the compound is the second peptide, is converted into a chlorocarbonyl group, a fluorocarbonyl group, a bromocarbonyl group, an iodocarbonyl group or the like.

When the carboxylic acid or carboxylic acid analog for use in step (3), for example, the amino acid or the second peptide has a protective group, the protective can be a carbamate-type protective group, a sulfonyl-type protective group, or an acyl-type protective group.

In the present specification, the term "carbamate-type protective group" means a protective group constituting a carbamate structure, and includes, for example, protective groups having an Fmoc skeleton, Alloc, Teoc, Boc, Cbz, and a 2,2,2-trichloroethoxycarbonyl group (Troc). The term "sulfonyl-type protective group" means a protective group having a sulfonyl structure, and includes, for example, a methanesulfonyl (Ms) group, a p-toluenesulfonyl (Ts) group, a 2-nitrobenzenesulfonyl (Ns) group, a trifluoromethanesulfonyl (Tf) group, and a (2-trimethylsilyl)-ethanesulfonyl (SES) group. The term "acyl-type protective group" means a protective group having an acyl structure, and includes, for example, an acetyl group, a pivaloyl group, a benzoyl group, a trifluoroacetyl (Tfa) group.

In certain aspects, when the carboxylic acid or carboxylic acid analog for use in step (3) is an amino acid having a protective group containing an Fmoc skeleton, any natural or non-natural amino acid protected with a protective group containing an Fmoc can be used for the amino acid having a protective group containing an Fmoc skeleton.

In certain aspects, when the carboxylic acid or carboxylic acid analog for use in step (3) is a second peptide having a protective group containing an Fmoc skeleton, the types and the number of amino acid residues constituting the second peptide having a protective group containing an Fmoc skeleton are not limited as long as the amino acid at the N-terminal of the peptide is protected with a protective group containing an Fmoc skeleton, and a peptide having any amino acid sequence including two or more natural and/or non-natural amino acid residues. It is preferred that the second peptide contain one or more N-substituted amino acids.

In certain aspects, when the carboxylic acid or carboxylic acid analog for use in step (3) is a $C_1$-$C_8$ alkylcarboxylic acid, a $C_6$-$C_{10}$ arylcarboxylic acid, or an active ester or acid halide thereof, such a compound is optionally substituted with one or more substituents independently selected from the group consisting of alkenyl, alkynyl, alkoxy, cycloalkyl, aryl, heteroaryl, heterocyclyl, arylalkyl, heteroarylalkyl, halogen, nitro, dialkylamino,cyano, alkoxycarbonyl and dialkylaminocarbonyl. The $C_1$-$C_8$ alkylcarboxylic acid, or the active ester or acid halide thereof is preferably a $C_1$-$C_4$ alkylcarboxylic acid, or an active ester or acid halide thereof, and examples thereof specifically include acetic acid, propanoic acid, butanoic acid, pentanoic acid, and active esters or acid halides thereof. Examples of the $C_6$-$C_{10}$ arylcarboxylic acid, or the active ester or acid halide thereof specifically include benzoic acid, naphthoic acid, and active esters or acid halides thereof.

In step (3), it is possible to use an amino acid having a protective group containing an Fmoc skeleton at 1 to 20 equivalents, preferably 2 to 10 equivalents, to the first peptide, or a second peptide having a protective group containing an Fmoc skeleton.

In certain aspects, it is preferred that the first peptide, and/or the second peptide having a protective group containing an Fmoc skeleton contain at least one N-substituted amino acid. The amino acid having a protective group containing an Fmoc skeleton is preferably a N-substituted amino acid.

In certain aspects, the protective group containing an Fmoc skeleton in the amino acid or second peptide for use in step (3) may be identical to or different from the protective group containing an Fmoc skeleton in the first peptide for use in step (1).

In certain aspects, the solvent for use in the step (3) may contain at least one of an aromatic hydrocarbon solvent, a halogen solvent, an ether solvent, an amide solvent, a sulfoxide solvent, a sulfone solvent, a urea solvent, an ester solvent, a ketone solvent, a carbonate solvent and a phosphoric acid ester solvent, and may be a single solvent or a mixed solvent. When the solvent for use in the step (3) is a mixed solvent, it is preferred that the solvent contain at least one of an aromatic hydrocarbon solvent, a halogen solvent, an ether solvent, an amide solvent, a sulfoxide solvent, a sulfone solvent, a urea solvent, an ester solvent, a ketone solvent, a carbonate solvent and a phosphoric acid ester solvent at 25 v/v% or more, 50 v/v% or more, or 75 v/v% or more.

When an aromatic hydrocarbon solvent is used in step (3), examples of the solvent specifically include benzene, toluene, xylene, chlorobenzene, 1,2-dichlorobenzene, bromobenzene, anisole, ethylbenzene, nitrobenzene, and cumene, with toluene and cumene being preferred.

When a halogen solvent is used in step (3), examples of the solvent specifically include dichloromethane, chloroform, 1,2-dichloroethane, and carbon tetrachloride, with dichloromethane being preferred.

When an ether solvent is used in step (3), examples of the solvent specifically include diethyl ether, tetrahydrofuran, 2-methyltetrahydrofuran, 1,4-dioxane, 1,3-dioxane, 1,2-dimethoxyethane, diisopropyl ether, cyclopentyl methyl ether, t-butyl methyl ether, 4-methyltetrahydropyran, diglyme, triglyme, and tetraglyme, with tetrahydrofuran, 2-methyltetrahydrofuran, 1,2-dimethoxyethane and 1,3-dioxolane being preferred.

When an amide solvent is used in step (3), examples of the solvent specifically include N,N-dimethylformamide (DMF), N-methylpyrrolidone (NMP), N,N-dimethylacetamide (DMA), M-ethylpyrrolidone (NEP), N-butylpyrrolidone (NBP), and formamide, with DMF and NMP being preferred.

When a sulfoxide solvent is used in step (3), examples of the solvent specifically include dimethyl sulfoxide (DMSO), and methyl phenyl sulfoxide, with DMSO being preferred.

When a sulfone solvent is used in step (3), examples of the solvent specifically include diphenyl sulfone, dimethyl sulfone, diethyl sulfone, sulfolane, 3-methylsulfolane, ethyl methyl sulfone, and ethyl isopropyl sulfone.

When a urea solvent is used in step (3), examples of the solvent specifically include 1,3-dimethyl-2-imidazolidinone (DMI), and 1,3-dimethyl-3,4,5,6-tetrahydro-2(1H)-pyrimidinone (DMPU), with DMI being preferred.

When an ester solvent is used in step (3), examples of the solvent specifically include methyl acetate, ethyl acetate, butyl acetate, methyl propionate, butyl acetate, propyl acetate, isopropyl acetate, isobutyl acetate, pentyl acetate, and $\gamma$-valerolactone, with ethyl acetate, butyl acetate and methyl propionate being preferred.

When a ketone solvent is used in step (3), examples of the solvent specifically include acetone, methyl ethyl ketone, methyl isobutyl ketone, cyclohexanone, cyclopentanone, and diethyl ketone, with methyl ethyl ketone and diethyl ketone being preferred.

When a carbonate solvent is used in step (3), examples of the solvent specifically include dimethyl carbonate, diethyl carbonate, and dibutyl carbonate, with dimethyl carbonate being preferred.

When a phosphoric acid ester solvent is used in step (3), examples of the solvent specifically include trimethyl phosphate, triethyl phosphate, and tributyl phosphate, with tributyl phosphate being preferred.

When the solvent for use in the step (3) is a mixed solvent, the mixed solvent may include two or more solvents selected from an aromatic hydrocarbon solvent, a halogen solvent, an ether solvent, an amide solvent, a sulfoxide solvent, a sulfone solvent, a urea solvent, an ester solvent, a ketone solvent, a carbonate solvent and a phosphoric acid ester solvent, and may contain any other solvents.

When the solvent for use in step (3) is a single solvent, the solvent is preferably toluene, cumene, 1,2-dichlorobenzene, benzene, anisole, dichloromethane, chloroform, 1,2-dichloroethane, tetrahydrofuran, 2-methyltetrahydrofuran, cyclopentyl methyl ether, 4-methyltetrahydropyran, 1,4-dioxane, 1,2-dimethoxyethane, 1,3-dioxolane, DMF, NMP, DSMO, DMI, DMPU, ethyl acetate, isopropyl acetate, butyl acetate, methyl propionate, acetone, methyl ethyl ketone, diethyl ketone, dimethyl carbonate, diethyl carbonate, trimethyl phosphate, tributyl phosphate, sulfolane, or 3-methylsulfolane.

In certain aspects, the solvent for use in step (3) can be identical to the solvent for use in step (2).

As the solvent for use in step (3), one in which a condensation agent is soluble is preferably used. In the present invention, the base used in step (2) may remain, and the remaining base may cause formation of side products in which an amino acid or peptide is excessively elongated. When a solvent in which a condensation agent is soluble is used in step (3), formation of such an excessive elongation compound can be suppressed.

In certain aspects, the condensation agent for use in step (3) is in the form of a salt, and the counter anion thereof is $PF_6^-$ or $BF_4^-$. Examples of such a condensation agent specifically include PyOxim ([ethylcyano(hydroxyimino)acetato-$O^2$]tri-1-pyrrolidinylphosphonium hexafluorophosphate), PyAOP ((7-azabenzotriazol-1-yloxy)trispyrrolidinophosphonium hexafluorophosphate), PyBOP (benzotriazol-1-yloxy)trispyrrolidinophosphonium hexafluorophosphate), COMU ((1-cyano-2-ethoxy-2-oxoethylideneaminoxy)dimethylaminomorpholinocarbenium hexafluorophosphate), HATU (O-(7-aza-1H-benzotriazol-1-yl)-N,N,N',N'-tetramethyluronium hexafluorophosphate), HBTU (1-[bis(dimethylamino)methylene]-1H-benzotriazolium 3-oxide hexafluorophosphate, HCTU (O-(6-chlorobenzotriazol-1-yl)-N,N,N',N'-tetramethyluronium hexafluorophosphate), TDBTU (O-(3,4-dihydro-4-oxo-1,2,3-benzotriazin-3-yl)-N,N,N',N'-tetramethyluronium tetrafluoroborate), HOTU (O-[(ethoxycarbonyl)cyanomethyleneamino]-N,N,N',N'-tetramethyluronium hexafluorophosphate), TATU (1-[bis(dimethylamino)methylene]-1H-1,2,3-triazolo[4,5-b]pyridinium 3-oxide tetrafluoroborate), TBTU (1-[bis(dimethylamino)methylene]-1H-benzotriazolium 3-oxide tetrafluoroborate), TCTU (O-(6-chloro-1H-benzotriazol-1-yl)-N,N,N',N'-tetramethyluronium tetrafluoroborate), and TOTU (O-[ethoxycarbonyl)cyanomethyleneamino]-N,N,N',N'-tetramethyluronium tetrafluoroborate).

It is preferred that the condensation agent for use in step (3) contain at least one selected from the group consisting of PyOxim, PyAOP, PyBOP, COMU, HATU, HBTU, HCTU, TDBTU, HOTU, TATU, TBTU, TCTU and TOTU, and it is more preferred to use any one of these compounds.

In step (3), it is possible to use a condensation agent at 1 to 20 equivalents, preferably 2 to 10 equivalents, to the first peptide.

In certain aspects, the condensation reaction of step (3) can be carried out in the presence of a base. The type of base is not limited as long as it contributes to progress of the condensation reaction, and a base having a pKa of 5 to 12 in water as a conjugate acid is preferred. Examples of such a base specifically include DIPEA, triethylamine, 2,6-lutidine, 2,4,6-trimethylpyridine, and pyridine. In step (3), it is possible to use a base at 1 to 20 equivalents, preferably 2 to 10 equivalents, to the first peptide. As the pKa of the base in water as a conjugate acid, a value calculated in accordance with, for example, Advanced Chemistry Development (ACD/Labs) Software V11.02 ((c)1994-2020 ACD/Labs), or the like can be used.

In certain aspects, the present invention does not include the step of neutralizing the residual base by adding an acid between step (2) and step (3). When a peptide chain is elongated by a solid-phase method, a remaining base may be neutralized with an acid between the deprotection step and the elongation step because remaining of a base used in the deprotection step before the elongation step is generally considered to be undesirable. Unexpectedly, however, it has been found that in the present invention, such neutralization rather leads to formation of a DKP form, and the neutralization does not contribute to progress of the reaction with efficiency. Without being bound by a specific theory, it is thought that by adding an acid, a carbamic acid salt formed is decomposed and converted into an amine form, leading to an increase in the amount of a DKP elimination compound and a 6-membered cyclic amidine skeleton compound formed as side products through a 6-membered ring intermediate.

In certain aspects, the present invention may further include the step of washing the solid-phase synthesis resin between step (2) and step (3). For washing the resin, any solvent can be used, and it is preferred to use a solvent used in step (2). The resin can be washed two or more times with the same solvent or different solvents.

In certain aspects, in the step of washing the solid-phase synthesis resin, a solvent brought into contact with $CO_2$ before the step (e.g. a solvent bubbled with $CO_2$ gas) may be used, a solvent which has not been brought into contact with $CO_2$ may be used, or a solvent brought into contact with $CO_2$ may be used in combination with a solvent which has not been brought into contact with $CO_2$. When the washing is performed two or more times, it is preferred to wash the resin one or more times with a solvent brought into contact with $CO_2$ in advance, and then wash the resin one or more times with a solvent which has not been brought into contact with $CO_2$ in advance.

In certain aspects, the reaction of step (3) can be carried out at -100°C to a temperature near a boiling point of the solvent, preferably 0 to 60°C. The reaction of step (3) can be carried out for 1 minute to 1 week, preferably 30 minute to 20 hours.

In certain aspects, the peptide produced by the method of the present invention contains a third peptide produced through the steps (1) to (3). In this case, the peptide produced by the method of the present invention can further contain any number of arbitrary amino acids in addition to the third peptide.

In certain aspects, the peptide produced by the method of the present invention can contain a third peptide produced through the steps (1) to (3).

In certain aspects, the peptide produced by the method of the present invention can contain at least one, or two or more, three or more, four or more or five or more N-substituted amino acids. The N-substituted amino acid can be a N-alkylamino acid, preferably a N-methylamino acid.

In certain aspects, the method of the present invention may comprise the step of isolating a peptide from the solid-phase synthesis resin after the step of elongating a peptide chain. For the isolation step, a method known in the art, or a method described in Examples can be used.

In certain aspects, the present invention also relates to a method for reducing the amount of a diketopiperazine impurity and/or a 6-membered cyclic amidine skeleton compound impurity formed in production of a peptide by a solid-phase method comprising the step (1) and the step (2).

All references cited herein are incorporated herein by reference.

## Examples

The present invention will be described in further detail by way of Examples, Comparative examples and Reference Examples, but the present invention is not limited to Examples. All starting materials and reagents were obtained from commercial suppliers, or synthesized using known methods.

In Examples, the following abbreviations were used.

AA: ammonium acetate
Alloc: allyloxycarbonyl group
Boc: tert-butoxycarbonyl group
Cbz: benzyloxycarbonyl group
COMU: (1-cyano-2-ethoxy-2-oxoethylideneaminoxy)dimethylaminomorpholinocarbenium hexafluorophosphate
DBU: 1,8-diazabicyclo[5.4.0]-7-undecene
DCM: dichloromethane

DCE: 1,2-dichloroethane
DMA: dimethylacetamide
DME: 1,2-dimethoxyethane
DMF: N,N-dimethylformamide
DMI: 1,3 -dimethyl-2-imidazolidine
DKP: diketopiperazine
DIC: N,N'-diisopropylcarbodiimide
DIPEA: N,N'-diisopropylethylamine
DMSO: dimethyl sulfoxide
EDCI·HCl: 1-ethyl-3-(3-dimethylaminopropyl)carbodiimide hydrochloride
EtOAc: ethyl acetate
FA: formic acid
Fmoc: 9-fluorenylmethyloxycarbonyl group
HOAt: 1-hydroxy-7-azabenzotriazole
HOBt: 1-hydroxybenzotriazole
HOOBt: 3,4-dihydro-3-hydroxy-4-oxo-1,2,3-benzotriazine
HATU: O-(7-aza-1H-benzotriazol-1-yl)-N,N,N',N'-tetramethyluronium hexafluorophosphate
HBTU: O-(1H-benzotriazol-1-yl)-N,N,N',N'-tetramethyluronium hexafluorophosphate
HCTU: O-(1H-6-chloro-1-yl)-1,1,3,3-tetramethyluronium hexafluorophosphate
HOTU: O-[(ethoxycarbonyl)cyanomethyleneamino]-N,N,N',N'-tetramethyluronium hexafluorophosphate
HP1 (dma): imino-tris(dimethylamino)phosphorane
MEK: methyl ethyl ketone
MTBD: 7-methyl-1,5,7-triazabicyclo[4.4.0]deca-5-ene
NMP: N-methyl-2-pyrrolidone
Ns: 2-nitrobenzenesulfonyl group
Oxyma: ethyl cyano(hydroxyimino)acetate
pip: piperidine
P1tBu: t-butylimino-tris(dimethylamino)phosphorane
P2Et: triamide tetramethyl(tris(dimethylamino)phosphoranylidene)phosphate-ethylimie
PyAOP: (7-azabenzotriazol-1-yloxy)tripyrrolidinophosphonium hexafluorophosphate
PyBOP: benzotriazol-1-yloxy-tris-pyrrolidino-phosphonium hexafluorophosphate
PyOxim: [ethylcyano(hydroxyimino)acetat-O2]tri-1-pyrrolidinylphosphonium hexafluorophosphate
TBME: t-butyl methyl ether
Teoc: 2-trimethylsilylethoxycarbonyl group
TES: triethylsilane
Tfa: trifluoroacetyl group
TFA: trifluoroacetic acid
TFE: 2,2,2-trifluoroethanol
THF: tetrahydrofuran
THP: tetrahydropyranyl
TMG: 1,1,3,3-tetramethylguanidine
TATU: (1-[bis(dimethylamino)methylene]-1H-1,2,3-triazolo[4,5-b]pyridinium 3-oxide tetrafluoroborate
TBTU: 1-[bis(dimethylamino)methylene]-1H-benzotriazolium 3-oxide tetrafluoroborate
TCTU: O-(6-chlorobenzotriazol-1-yl)-N,N,N',N'-tetramethyluronium tetrafluoroborate
TDBTU: O-(3,4-dihydro-4-oxo-1,2,3-benzotriazin-3-yl)-N,N,N',N'-tetramethyluronium tetrafluoroborate
TOTU: O-[(ethoxycarbonyl)cyanomethyleneamino]-N,N,N',N'-tetramethyluronium tetrafluoroborate

The analysis conditions of LCMS are shown in Table 1.

[Table 1]

| Analysis conditions | Apparatus | Column (1.D. × length) (mm) | Mobile phase | Gradient (A/B) | Flow rate (mL/min) | Column temperature (°C) | Wavelength |
|---|---|---|---|---|---|---|---|
| SQDAA05 | Acquity UPLC/SQD or Acquity UPLC/SQD2 | Ascent is Express C18 (2. 1x50) | A) 10 mM $AcONH_4$, $H_2O$<br>B) Me0H | 95/5 (initial)<br>=> 0/100 (1.0 min)<br>=> 0/100 (0.4 min) | 1.0 | 35 | 210-400nm PDA total |
| SQDAA50 | Acquity UPLC/SQD or Acquity UPLC/SQD2 | Ascent is Express C18 (2. 1x50) | A) 10mM $AcONH_4$, $H_2O$<br>B) MeOH | 50/50 (initial)<br>=> 0/100 (0.7 min)<br>=> 0/100 (0.7 min) | 1.0 | 35 | 210-400nm PDA total |
| SQDFA05 | Acquity UPLC/SQD or Acquity UPLC/SQD2 | Ascent is Express 018 (2. 1x50) | A)0.1% FA, $H_2O$<br>B)0.1% FA, MeCN | 95/5 (initial)<br>=> 0/100 (1.0 min)<br>=> 0/100 (0.4 min) | 1.0 | 35 | 210-400nm PDA total |
| SQDFA50 | Acquity UPLC/SQD or Acquity UPLC/SQD2 | Ascent is Express C18 (2. 1x50) | A)0.1% FA, $H_2O$<br>B)0.1% FA, MeCN | 50/50 (initial)<br>=> 0/100 (0.7 min)<br>=> 0/100(0.7 min) | 1.0 | 35 | 210-400nm PDA total |
| SQDFA05long | Acquity UPLC/SQD2 | Ascent is Express C18 (2. 1x50) | A)0.1% FA. $H_2O$<br>B)0.1% FA, MeCN | 95/5 (initial)<br>=> 0/100 (4.5 min)<br>=> 0/100 (0.5 min) | 1.0 (0-4.5min)<br>0.1 (4.5-5min) | 35 | 210-400nm PDA total |
| SQDAA05long | Acquity UPLC/SQD2 | Ascent is Express C18 (2. 1x50) | A) 10mM $Ac0NH_4$, $H_2O$<br>B) MeOH | 95/5 (initial)<br>=> 0/100 (4.5 min)<br>=> 0/100(0.5 min) | 1.0 (0-4.5min)<br>0.1 (4.5-5mi n) | 35 | 210-400nm PDA total |

Example 1: Preparation of amino acid, resin-supported peptide and the like used in Example

Example 1-1: Fmoc-amino acid used for peptide synthesis by an automated peptide synthesizer

In peptide synthesis described in the present specification, Fmoc-amino acids shown Tables 2 to 4 were used for synthesis by an automated peptide synthesizer.

The Fmoc-amino acids shown in Table 2 were synthesized in accordance with methods described in WO 2018/225864 and WO 2021/090856.

The Fmoc-amino acids shown in Table 3 were purchased from commercial suppliers.

The Fmoc-amino acids shown in Table 4 were synthesized in accordance with the schemes shown below.

[Table 2]

| Compound No. | Abbreviation | Structural formula | Name |
|---|---|---|---|
| aa1-1 | Fmoc-Ser (iPen)-OH | 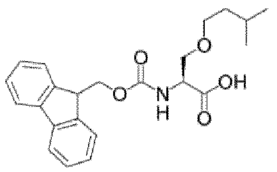 | (2S)-2-(9H-fluoren-9-ylmethoxycarbonylamino)-3-(3-methylbutoxy)propanoic acid |
| aa1-2 | Fmoc-Ser(nPr)-OH | 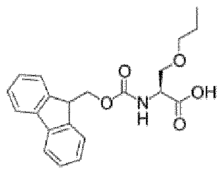 | (2S)-2-(9H-fluoren-9-ylmethoxycarbonylamino)-3-propoxypropanoic acid |
| aa1-3 | Tfa-Aib-OH | 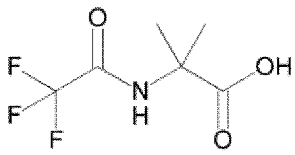 | 2-methyl-2-[(2,2,2-trifluoroacetyl) amino]propanoic acid |

[Table 3]

| Compound No. | Abbreviation | Structural formula | Name | CAS No. |
|---|---|---|---|---|
| aa2-1 | Fmoc-Aib-OH | 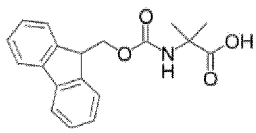 | **2-(9H-fluoren-9-ylmethoxycarbonylamino)-2-methylpropanoic acid** | 94744-50-0 |
| aa2-2 | Fmoc-Aze (2)-OH | 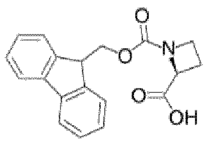 | **(2S)-1-(9H-fluoren-9-ylmethoxycarbonyl)acetidin-2-carboxylic acid** | 136552-06-2 |
| aa2-3 | Fmoc-cLeu-OH | 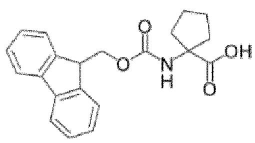 | **1-(9H-fluoren-9-ylmethoxycarbonylamino) cyclopentan-1-carboxylic acid** | 117322-30-2 |

(continued)

| Compound No. | Abbreviation | Structural formula | Name | CAS No. |
|---|---|---|---|---|
| aa2-4 | Fmoc-Ile-OH | 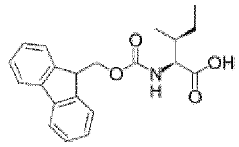 | **(2S,3S)-2-(9H-fluoren-9-ylmethoxycarbonylamino)-3-methylpentanoic acid** | 71989-23-6 |
| aa2-5 | Fmoc-MeAla-OH | 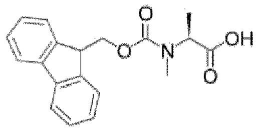 | **(2S)-2-[9H-fluoren-9-ylmethoxycarbonyl(methyl) amino]propanoic acid** | 84000-07-7 |
| aa2-6 | Fmoc-MeCha-OH | 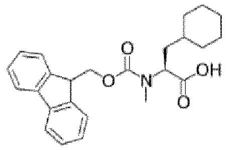 | **(2S)-3-cyclohexyl-2-[9H-fluoren-9-ylmethoxycarbonyl(methyl) amino]propanoic acid** | 148983-03-3 |
| aa2-7 | Fmoc-MeGly-OH | 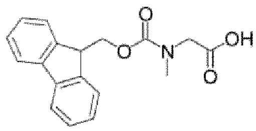 | **2-[9H-fluoren-9-ylmethoxycarbonyl(methyl) amino]acetic acid** | 77128-70-2 |
| aa2-8 | Fmoc-MeIle-OH | 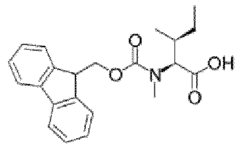 | **(2S,3S)-2-[9H-fluoren-9-ylmethoxycarbonyl(methyl) amino]-3-methylpentanoic acid** | 138775-22-1 |
| aa2-9 | Fmoc-MeLeu-OH | 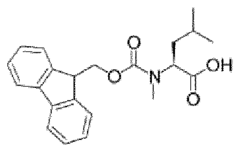 | **(2S)-2-[9H-fluoren-9-ylmethoxycarbonyl(methyl) amino]-4-methylpentanoic acid** | 103478-62-2 |
| aa2-10 | Fmoc-MeVal-OH | 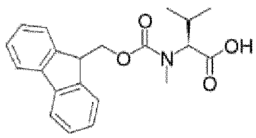 | **(2S)-2-[9H-fluoren-9-ylmethoxycarbonyl(methyl) amino] -3-methylbutanoic acid** | 84000-11-3 |
| aa2-11 | Fmoc-Nle-OH | 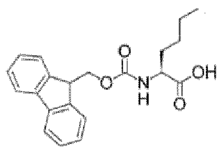 | **(2S)-2-(9H-fluoren-9-ylmethoxycarbonylamino) hexanoic acid** | 77284-32-3 |
| aa2-12 | Fmoc-Phe-OH | 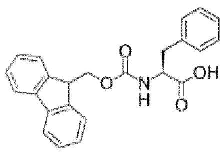 | **(2S)-2-(9H-fluoren-9-ylmethoxycarbonylamino) -3-phenylpropanoic acid** | 35661-40-6 |
| aa2-13 | Fmoc-Pro-OH | 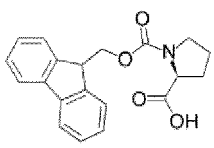 | **(2S)-1-(9H-fluoren-9-ylmethoxycarbonyl)pyrrolidi ne-2-carboxylic acid** | 71989-31-6 |

(continued)

| Compound No. | Abbreviation | Structural formula | Name | CAS No. |
|---|---|---|---|---|
| aa2-14 | Fmoc-Thr (tBu)-OH | 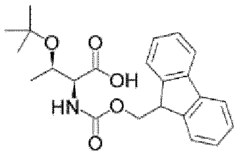 | **(2S,3R)-2-(9H-fluoren-9-ylmethoxycarbonylamino)-3-(2-methyl-2-propanyloxy)butanoic acid** | 71989-35-0 |
| aa2-15 | Fmoc-Cys (StBu)-OH | 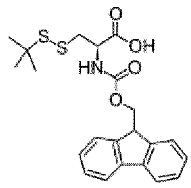 | **(2S)-2-(9H-fluoren-9-ylmethoxycarbonylamino)-3-(2-methyl-2-propanyldisulphanyl) propanoic acid** | 73724-43-3 |
| as2-16 | Boc-MeAla-OH | 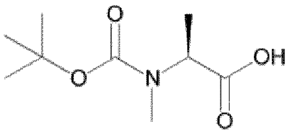 | **2-((tert-butoxycarbonyl)(methyl) amino)propanoic acid** | 16948-16-6 |
| aa2-17 | Fmoc-Ala-OH | 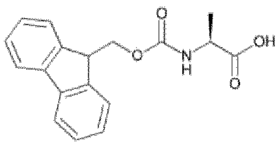 | **(2S)-2-(9H-fluoren-9-ylmethoxycarbonylamino) propanoic acid** | 35661-39-3 |

[Table 4]

| Compound No. | Abbreviation | Structural formula | Name |
|---|---|---|---|
| aa3-1 | Fmoc-MeAla (3-Pyr)-OH | 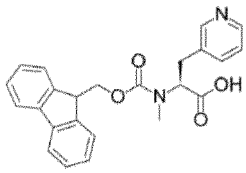 | **(2S)-2-[9H-fluoren-9-ylmethoxycarbonyl (methyl)amino] -3-pyridin-3-ylpropanoic acid** |
| aa3-2 | Teoc-Phe(4-CF3)-OH | 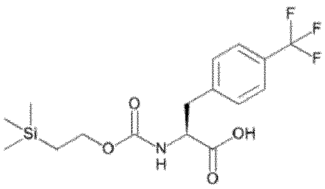 | **(2S)-3-[4-(trifluoromethyl)phenyl]-2-(2-trimethylsilylethoxycarbonylamino) propanoic acid** |
| aa3-3 | Alloc-Phe-OH | 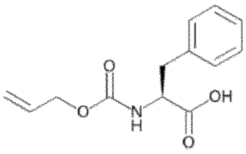 | **(2S)-2-(allyloxycarbonylamino)-3-phenylpropanoic acid** |
| aa3-4 | Ns-Aib-OH | 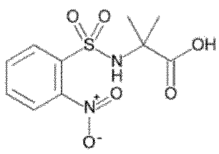 | **2-methyl-2-[(2-nitrophenyl)sulfonylamino] propanoic acid** |

Synthesis of compound aa3-1: (2S)-2-[9H-fluoren-9-ylmethoxycarbonyl(methyl)amino]-3-pyridin-3-ylpropanoic acid (Fmoc-MeAla (3-Pyr)-OH)

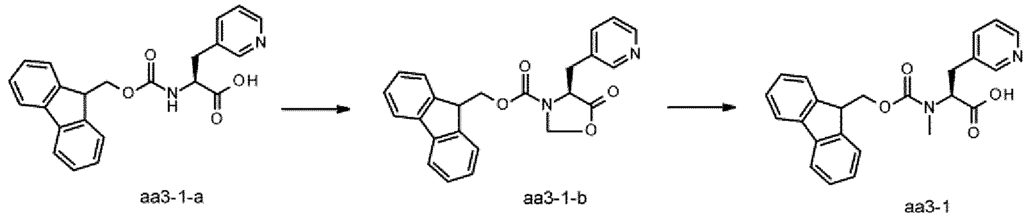

aa3-1-a aa3-1-b aa3-1

Paraformaldehyde (696 mg, 23.17 mmol) and trifluoroacetic acid (TFA) (5.36 mL, 69.5 mmol) were added to a solution of commercially available (2S)-2-[9H-fluoren-9-ylmethoxycarbonylamino]-3-pyridin-3-ylpropanoic acid (Fmoc-Ala(3-Pyr)-OH, compound aa3-1-a) (3 g, 7.72 mmol) in toluene (10.5 mL) in a nitrogen atmosphere, and the mixture was stirred at 40°C for 2 hours. The reaction solution was concentrated under reduced pressure, diluted with dichloromethane (DCM), then washed with a saturated aqueous sodium bicarbonate solution, dried over anhydrous magnesium sulfate, and then filtered. The obtained solution was concentrated under reduced pressure to obtain compound aa3-1-b (2.95 g, 95%) as a crude product. The compound was used for the next reaction without being further purified.

LCMS(ESI) m/z = 401 (M+H)+
Retention time: 0.64 minutes (analysis condition SQDFA05)

Triethylsilane (TES) (10.59 mL, 66.3 mmol) and trifluoroacetic acid (TFA) (15.32 mL, 199 mmol) were added to a solution of compound aa3-1-b (2.95 g) in 1,2-dichloroethane (DCE) (15.5 mL) at room temperature in a nitrogen atmosphere, and the mixture was stirred at 70°C for 5 hours. The reaction solution was cooled to room temperature, the solvent was distilled off under reduced pressure, the thus-obtained crude product was dissolved in ethyl acetate, t-butyl methyl ether/n-hexane = 9/1 (40 mL) was added, and the mixture was stirred. This mixture was cooled in a refrigerator for 30 minutes, the resulting solid was then filtered, washed three times with t-butyl methyl ether/n-hexane = 9/1 (30 mL), and then dried under reduced pressure. The obtained crude product was suspended in DCM (50 mL), an aqueous sodium dihydrogenphosphate solution at 3 mol/L (45 mL, including sodium chloride at 1.5 mol/L) was added, the mixture was stirred, the aqueous layer was removed, and the organic layer was then washed twice with an aqueous sodium dihydrogenphosphate solution at 3 mol/L (45 mL, including sodium chloride at 1.5 mol/L). The organic layer was dried over anhydrous magnesium sulfate, the solvent was then distilled off under reduced pressure, and the thus-obtained residue was purified by reverse-phase column chromatography (water/acetonitrile) to obtain compound aa3-1: (2S)-2-[9H-fluoren-9-ylmethoxycarbonyl(methyl)amino]-3-pyridin-3-ylpropanoic acid (Fmoc-MeAla (3-Pyr)-OH) (2.67 g, 90%).

LCMS(ESI) m/z = 403 (M+H)+
Retention time: 0.53 minutes (analysis condition SQDFA05)

Synthesis of compound aa3-2: (2S)-3-[4-trifluoromethyl)phenyl]-2-(2-trimethylsilylethoxycarbonylamino)propanoic acid (Teoc-Phe(4-CF3)-OH)

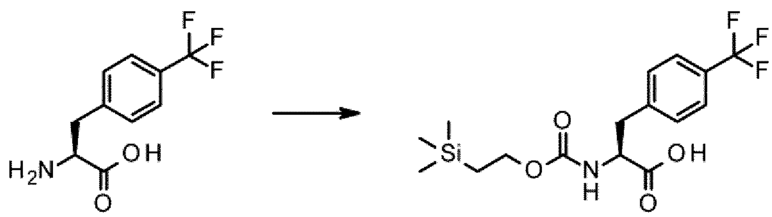

aa3-2-a aa3-2

Commercially available (S)-2-amino-3-(4-(trifluoromethyl)phenyl)propanoic acid (H-Phe (4-CF3)-OH, compound aa3-2-a) (2.00 g, 8.58 mmol) was dissolved in water (12.0 mL) in a nitrogen atmosphere, a mixed solution of triethylamine (2.38 mL, 17.15 mmol) and 1,4-dioxane (10.0 mL) was then added, and the mixture was stirred at room temperature for 10 minutes. Thereafter, 2,5-dioxopyrrolidin-1-yl(2-(trimethylsilyl)ethyl)carbonate (2.45 g, 9.43 mmol) was added, and the mixture was stirred at room temperature for 1 hour. To the reaction solution was added 2-methyltetrahydrofuran (5 mL), and the mixture was washed with a 10% aqueous sodium carbonate solution (10 mL). The obtained organic layer was washed with a saturated aqueous potassium bisulfate solution (10 mL), then washed with a 10% aqueous sodium chloride solution, dried over anhydrous magnesium sulfate, and then filtered. The obtained solution was concentrated under reduced pressure to obtain compound aa3-2: 2S)-3-[4-trifluoromethyl)phenyl]-2-(2-trimethylsilylethoxycarbonylamino)propanoic acid (Teoc-Phe(4-CF3)-OH). (2.88 g, 89%)

LCMS(ESI) m/z = 376 (M-H)-
Retention time: 0.87 minutes (analysis condition SQDFA05)

Synthesis of compound aa3-3: (2S)-2-(allyloxycarbonylamino)-3-phenylpropanoic acid (Alloc-Phe-OH)

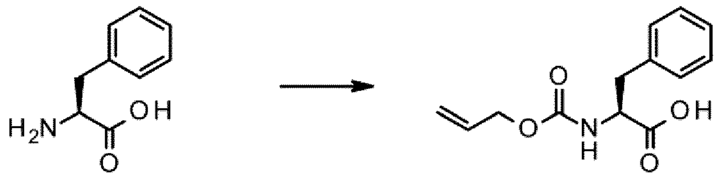

aa3-3-a aa3-3

A 50% aqueous 1,4-dioxane solution (20 mL) was added to and mixed with L-phenylalanine (1.00 g, 6.05 mmol), and sodium bicarbonate (1.27 g, 15.1 mmol, 2.5 equivalents) was then added. Ally chloroformate (0.97 mL, 9.1 mmol, 1.5 equivalents) was added dropwise while the mixed solution was cooled in an ice bath. The reaction mixture was stirred at room temperature for 2 hours, and then diluted with dichloromethane (10 mL, 10 v/w), phosphoric acid (0.53 mL, 1.5 equivalents) was added to acidify the aqueous layer, and the organic layer and the aqueous layer were separated. The aqueous layer was further washed with dichloromethane (5 mL, 5 v/w), the obtained organic layer was then washed with a half saturated aqueous sodium chloride solution (15 mL, 15 v/w), dried over anhydrous sodium sulfate, and then filtered, and the solution was concentrated under reduced pressure. The obtained crude product was purified by reverse-phase column chromatography (0.1% formic acid-containing acetonitrile/0.1% formic acid-containing distilled water), and the collected fractions were purified by reverse-phase column chromatography (additive-free acetonitrile/distilled water) to obtain compound 3-3: (2S)-2-(allyloxycarbonylamino)-3-phenylpropanoic acid (Alloc-Phe-OH) (0.52 g, 35%).

LCMS(ESI) m/z = 248 (M-H)-
Retention time: 0.58 minutes (analysis condition SQDFA05)

Synthesis of compound aa3-4: 2-methyl-2-[(2-nitrophenyl)sulfonylamino]propanoic acid (Ns-Aib-OH)

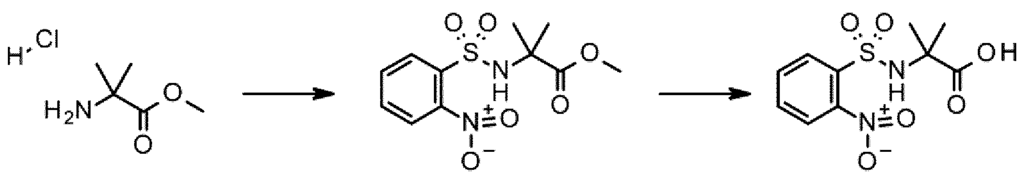

aa3-4-a aa3-4-b aa3-4

A solution of 2-nitrobenzenesulfonyl chloride (6.93 g, 31.2 mmol) in DCM (30.0 mL) was added to a solution of commercially available 2-amino-2-methylpropanoic acid methyl ester hydrochloride (4.0 g, 26.0 mmol) and DIPEA (10.92 mL, 62.5 mmol) in DCM (22.1 mL) with cooling in an ice bath, and the mixture was stirred at 0°C for 5 minutes, and then stirred at room temperature for 2 hours. The reaction mixture was concentrated under reduced pressure to

obtain crude product aa3-4-b.

LCMS(ESI) m/z = 301 (M-H)-
Retention time: 0.64 minutes (analysis condition SQDFA05)

A 5 N aqueous sodium hydroxide solution (26 mL) was added to a solution of the obtained crude product aa3-4-b (3.63 g) in THF/methanol (1/1, 52 mL) with cooling in an ice bath, and the mixture was stirred at 0°C for 5 minutes, and then stirred at room temperature for 5 hours. The reaction mixture was concentrated under reduced pressure, the obtained residue was then dissolved in 4 N hydrochloric acid (30 mL), and the solvent was distilled off. The mixture was extracted twice with ethyl acetate, the organic layer was then dried over anhydrous sodium sulfate, and then filtered, and the solution was concentrated under reduced pressure. The obtained crude product was purified twice by reverse-phase column chromatography (0.1% formic acid-containing acetonitrile/0.1% formic acid-containing distilled water) to obtain compound aa3-4: 2-methyl-2-[(2-nitrophenyl)sulfonylamino]propanoic acid (Ns-Aib-OH) (2.46 g, 71%).

LCMS(ESI) m/z = 287 (M-H)-
Retention time: 0.53 minutes (analysis condition SQDFA05)

Example 1-2: Preparation of resin-supported amino acid and peptide and the like used in Example

Example 1-2-1: Synthesis of (3S)-3-(9H-fluoren-9-ylmethoxycarbonylamino)-4-oxo-4-pyrrolidin-1-ylbutanoic acid-2-chlorotrityl resin (Fmoc-Asp (O-Trt (2-Cl)-resin)-pyrro, compound 1-2-1)

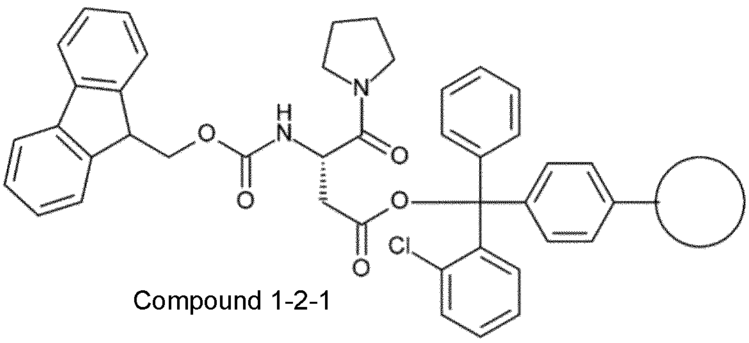

Compound 1-2-1

In the present specification, a polymer or resin part may be drawn as ○ when the polymer or resin is bonded to the compound. For the purpose of clarifying the reaction point of the resin part, the chemical structure of the reaction part may be drawn with the compound connected to ○. For example, in the above structure (Fmoc-Asp (O-Trt (2-Cl)-resin)-pyrro (compound 1-2-1)), the 2-chlorotriethyl group of the resin is bonded to the carboxylic acid on the side chain of Asp through an ester bond. The term "pyrro" means pyrrolidine, and in the above structure, the carboxylic acid group at the C-terminal forms an amide bond with pyrrolidine.

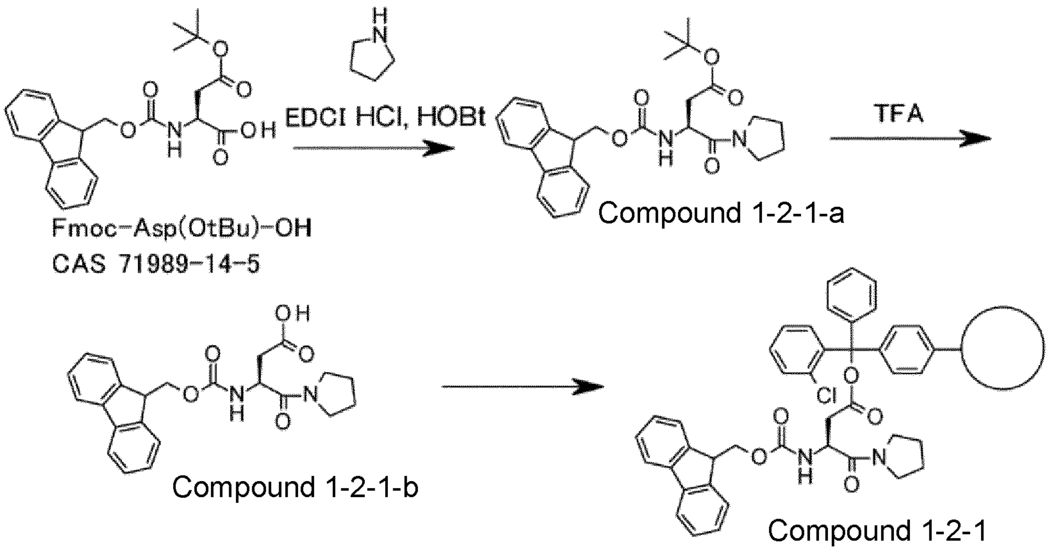


Fmoc-Asp(OtBu)-OH
CAS 71989-14-5

Compound 1-2-1-a

Compound 1-2-1-b

Compound 1-2-1

EDCI·HCl (67.1 g, 350 mmol), HOBt (43.3 g, 321 mmol) and Fmoc-Asp (OtBu)-OH (120 g, 2929 mmol) were added in this order to DMF (600 mL) at 0°C in a nitrogen atmosphere, and the mixture was stirred at 0°C for 1 hour. To the reaction solution, ethyl acetate (10 v) and aqueous hydrochloric acid at 0.5 mol/L (2 V) were added at 0°C, and the organic layer was separated. The obtained organic layer was washed with aqueous hydrochloric acid at 0.5 mol/L, water, saturated aqueous sodium bicarbonate solution/water (1/1 (v/v)), and saturated aqueous sodium chloride solution/water (1/1 (v/v)) in this order, and dried over anhydrous sodium sulfate, and the solvent was then distilled off under reduced pressure to obtain compound 1-2-1-a as a crude product. (137.1 g, quant.)

LCMS(ESI) m/z = 465 (M+H)+
Retention time: 1.05 minutes (analysis condition SQDAA05)

TFA (271 mL) was slowly added to a solution of compound 1-2-1-a (137 g, 395 mmol) in DCM (137 mL) under cooling with ice in such a manner that the internal temperature did not exceed 10°C. The mixture was stirred at room temperature for 1 hour, diisopropyl ether (3.4 L) was then added in four divided parts, and the precipitated solid was taken by filtration, and dried to obtain compound 1-2-1-b ((3s)-3-(9H-fluoren-9-ylmethoxycarbonylamino)-4-oxo-4-pyrrolidin-1-ylbutanoic acid, Fmoc-Asp-pyrro). (108.4 g, 90%)

LCMS(ESI) m/z = 409 (M+H)+
Retention time: 0.83 minutes (analysis condition SQDAA05)

The reaction for supporting the Fmoc-amino acid on the resin was carried out in accordance with a method described in WO 2013/100132 or WO 2018/225864. A reaction vessel with a filter was charged with 2-chlorotrityl chloride resin (1.60 mmol/g, 100 to 200 mesh, 1% DVB, 48.7 g) and dehydrated dichloromethane (500 mL), and the mixture was shaken at room temperature for 20 minutes. Nitrogen pressure was applied to remove the dichloromethane, a mixed solution obtained by adding dehydrated methanol (12.63 mL) and diisopropylethylamine (DIPEA) (32.6 mL) to compound 1-2-1-b (15.91 g) and dehydrated dichloromethane (350 mL) was then added in the reaction vessel, and the mixture was shaken for 60 minutes. Nitrogen pressure was applied to remove the reaction solution, a mixed solution obtained by adding dehydrated methanol (97.3 mL) and diisopropylethylamine (DIPEA) (32.6 mL) to dehydrated dichloromethane (350 mL) was then added in the reaction vessel, and the mixture was shaken for 1 hour and 30 minutes. Nitrogen pressure was applied to remove the reaction solution, dichloromethane (350 mL) was then put in the reaction vessel, the mixture was shaken for 5 minutes, and nitrogen pressure was then applied to remove the reaction solution. The washing of the resin with dichloromethane was repeated five times, and the obtained resin was dried overnight under reduced pressure to obtain (3S)-3-(9H-fluoren-9-ylmethoxycarbonylamino)-4-oxo-4-pyrrolidin-1-ylbutanoic acid-2-chlorotrityl resin (Fmoc-Asp (O-Trt (2-Cl)-resin)-pyrro, compound 1-2-1, 59.79 g).

For determination of the loading rate, the obtained compound 1-2-1 (12.6 mg) was put in the reaction vessel, DMF (2 mL) was added, and the mixture was shaken at room temperature for 1 hour. Thereafter, DBU (40 μL) was added, and the mixture was shaken at 30°C for 30 minutes. Thereafter, DMF (8 mL) was added to the reaction mixed solution, and 1 mL of the resulting solution was diluted with DMF (11.5 mL). The absorbance of the obtained dilution solution (294 nm) was measured (using Shimadzu, UV-1600 PC (cell length: 1.0 cm)), and the loading rate of compound

1-2-1 was calculated to be 0.464 mmol/g.

Another lot with a different loading rate, which had been similarly synthesized, was also used for peptide synthesis, studies and the like.

Example 1-2-2: Synthesis of (S)-3-(((9H-fluoren-9-yl)methoxy)carbonyl)amino)-4-oxo-4-(piperidin-1-yl)butanoic acid-2-chlorotrityl resin (Fmoc-Asp (O-Trt (2-Cl)-resin)-pip, compound 1-2-2)

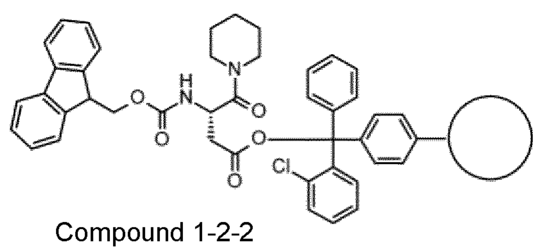

Compound 1-2-2

(S)-3-(((9H-fluoren-9-yl)methoxy)carbonyl)amino)-4-oxo-4-(piperidin-1-yl)butanoic acid-2-chlorotrityl resin (compound 1-2-2, Fmoc-Asp (O-Trt (2-Cl)-resin)-pip) was synthesized by a method described in a document (document: International Publication No. WO 2013/100132 A1). The term "pip" means piperidine, and in the above structure, the carboxylic acid group at the C-terminal forms an amide bond with piperidine.

Example 1-2-3: Synthesis of (3R)-3-(9H-fluoren-9-ylmethoxycarbonylamino)butanoic acid-2-chlorotrityl resin (Fmoc-D-3-Abu-O-Trt (2-Cl)-resin, compound 1-2-3)

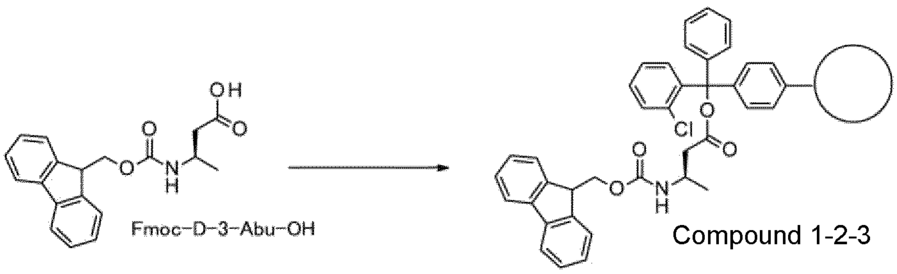


Compound 1-2-3

Using (3R)-3-(9H-fluoren-9-ylmethoxycarbonylamino)butanoic acid (Fmoc-D-3-Abu-OH) (7.1 g, 21.82 mmol) and 2-chlorotrityl chloride resin (1.6 mmol/g, 100 to 200 mesh, 1% DVB, 27.25 g, 43.6 mmol) purchased from a commercial supplier, the same procedure as in synthesis of compound 1-2-1 was carried out to obtain compound 1-2-3: (3R)-3-(9H-fluoren-9-ylmethoxycarbonylamino)butanoic acid-2-chlorotrityl resin (Fmoc-D-3-Abu-O-Trt (2-Cl)-resin). (33.44 g, loading rate: 0.598 mmol/g)

Another lot with a different loading rate, which had been similarly synthesized, was also used for peptide synthesis in this Example.

Example 1-2-4: Synthesis of (2S)-2-[9H-fluoren-9-ylmethoxycarbonyl(methyl)amino]propanoic acid-2-chlorotrityl resin (Fmoc-MeAla-O-Trt (2-Cl)-resin, compound 1-2-4)

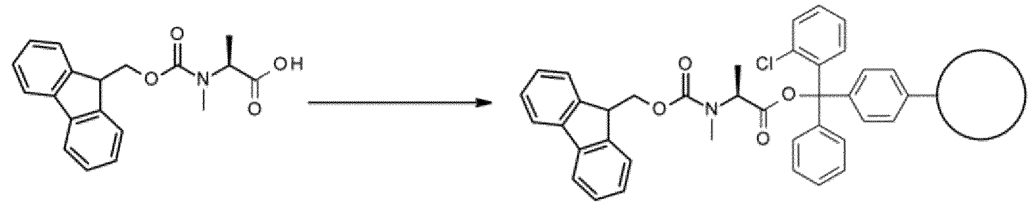

Fmoc-MeAla-OH

Compound 1-2-4

Using (2S)-2-[9H-fluoren-9-ylmethoxycarbonyl(methyl)amino]propanoic acid (Fmoc-MeAla-OH) (1.0 g, 3.07 mmol) and 2-chlorotrityl chloride resin (1.25 mmol/g, 100 to 200 mesh, 1% DVB, 4.92 g, 6.15 mmol) purchased from a commercial supplier, the same procedure as in synthesis of compound 1-2-1 was carried out to obtain compound 1-2-4: (2S)-2-[9H-fluoren-9-ylmethoxycarbonyl(methyl)amino]propanoic acid-2-chlorotrityl resin (Fmoc-MeAla-O-Trt (2-Cl)-resin). (5.42 g, loading rate: 0.514 mmol/g)

Example 1-2-5: Preparation of solid phase-supported peptide compounds (compounds 1-2-5 to 1-2-12) used in Example

Preparation of compounds 1-2-5 to 1-2-12 used in this Example was performed by an Fmoc method using an automated peptide synthesizer (Multipep RS; manufactured by Intavis AG). Detailed procedures of operations were consistent with those in the manual attached to the automated peptide synthesizer.

An Fmoc-protected amino acid constituting a target peptide (0.6 mol/L) and HOAt, Oxyma or HOOBt as a carboxylic acid activating agent (0.375 mol/L) were dissolved in NMP to prepare solution 1. In the case where the Fmoc-protected amino acid or HOOBt was hardly soluble, solution 1 was prepared with DMSO added to 20%. N,N'-diisopropylcarbodiimide (DIC) (10 v/v%) was mixed with N,N-dimethylformamide (DMF) to prepare solution 2.

2-Chlorotrityl resin bonded to a carboxylic acid part on the side chain of aspartic acid protected at the N-terminal with an Fmoc group or a carboxylic acid part on the main chain of an amino acid protected at the N-terminal with an Fmoc group (compounds 1-2-1 to 1-2-3) (100 mg per column), which had been prepared in Examples 1-2-1 to 1-2-3, was added in a solid-phase reaction vessel, and the reaction vessel was set in an automated peptide synthesizer. To this resin (100 mg) was added dichloromethane (DCM) (1.0 mL), and the mixture was left standing for about 30 minutes to swell the resin. Solution 1 and solution 2 were set in an automated peptide synthesizer, and automatic synthesis by the automated peptide synthesizer was started. The solution was discharged from a frit, and subsequently, the resin was washed twice with DMF (0.7 mL per column).

Fmoc deprotection step

A solution of 1,8-diazabicyclo[5.4.0]-7-undecene (DBU) in DMF (2 v/v%, 0.7 mL per column) was added, and deprotection of an Fmoc group was performed at room temperature. The mixture was reacted for 4.5 minutes in deprotection at the first residue, and reacted for 10 minutes in deprotection at the second and subsequent residues, followed by discharge of the solution from the frit. Subsequently, the resin was washed twice with DMF (0.7 mL per column), once with a solution of DIPEA (0.14 mol/L) and HOAt (0.14 mol/L) in DMF (0.7 mL per column), and twice with DMF (0.7 mL per column).

Elongation step

Solution 1 (0.3 mL per column) was mixed with solution 2 (0.36 mL per column) by a mixing vial of the automated peptide synthesizer, and the mixture was then added to the resin. The solid-phase reaction vessel was heated to 40-60°C in the case of a difficult-to-elongate sequence, and the mixture was reacted for 2.5 hours to 14 hours in the case of a difficult-to-elongate sequence to carry out a condensation reaction of the amino group on resin with the Fmoc-protected amino acid, followed by discharge of the solution from the frit. In the case where the elongation efficiency was low, this condensation reaction of the Fmoc-protected amino acid was further repeated once or twice. Subsequently, the resin was washed three times with DMF (0.7 mL per column).

This condensation reaction of the Fmoc amino acid subsequent to the deprotection reaction of the Fmoc group was set to one cycle, and this cycle was repeated to elongate a peptide on the resin surface. After elongation of the last amino acid, the Fmoc deprotection step was not carried out, and the resin was washed four times with DCM (1.0 mL per column), dried, and then used for subsequent studies. Using this method as a standard condition, the following solid phase-supported peptide compounds (compounds 1-2-5 to 1-2-12) were prepared.

While the peptide compounds were prepared using 100 mg of each of compounds 1-2-1 to 1-2-3 per column as described above, the peptide compounds were similarly prepared with a plurality of columns arranged per sequence.

Fmoc-Ile-MeAla-Aze(2)-MeCha-MeGly-Asp(O-Trt(2-Cl)-resin)-pyrro (SEQ ID NO: 1) (compound 1-2-5)

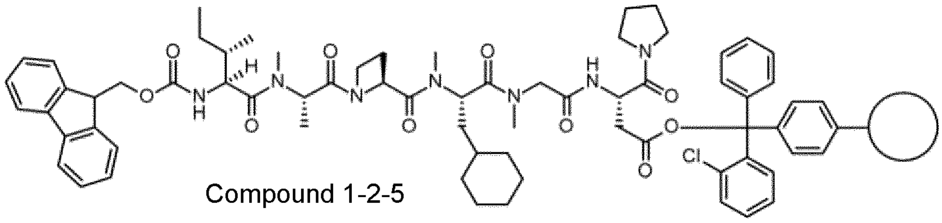

Compound 1-2-5

The compound was prepared from Fmoc-Asp(O-Trt(2-Cl)-resin)-pyrro (compound 1-2-1, 0.552 mmol/g).

Fmoc-Ile-MeGly-Aze(2)-MeCha-MeGly-Asp(O-Trt(2-Cl)-resin)-pip (SEQ ID NO: 2) (compound 1-2-6)

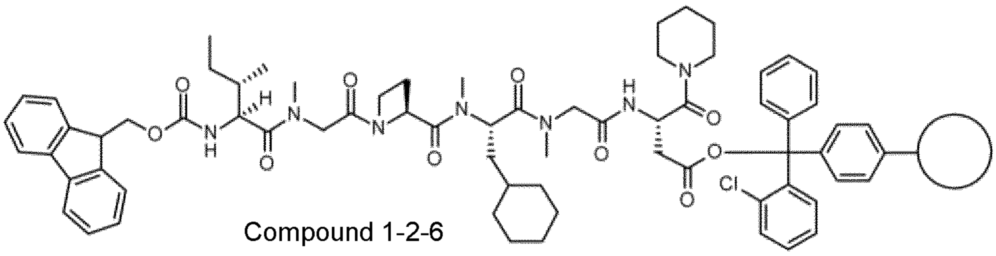

Compound 1-2-6

The compound was prepared from Fmoc-Asp(O-Trt(2-Cl)-resin)-pip (compound 1-2-2, 0.452 mmol/g).

Fmoc-Thr(tBu)-MeAla-Cys(StBu)-Phe-Asp(O-Trt(2-Cl)-resin)-pip (SEQ ID NO: 3) (compound 1-2-7)

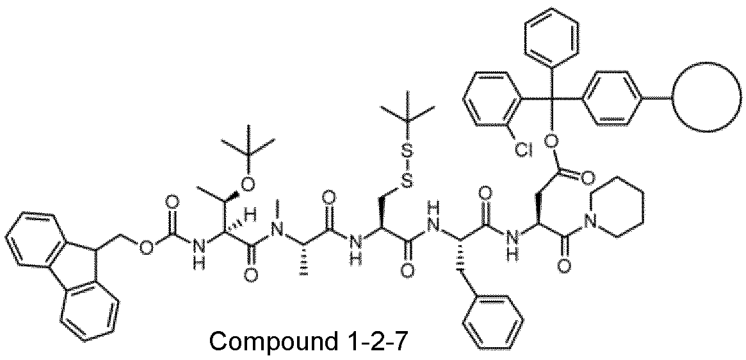

Compound 1-2-7

The compound was prepared from Fmoc-Asp(O-Trt(2-Cl)-resin)-pip (compound 1-2-2, 0.452 mmol/g).

Fmoc-Nle-MeAla(3-Pyr)-Ser(iPen)-Asp-(O-Trt(2-Cl)-resin)-pip (SEQ ID NO: 4) (compound 1-2-8)

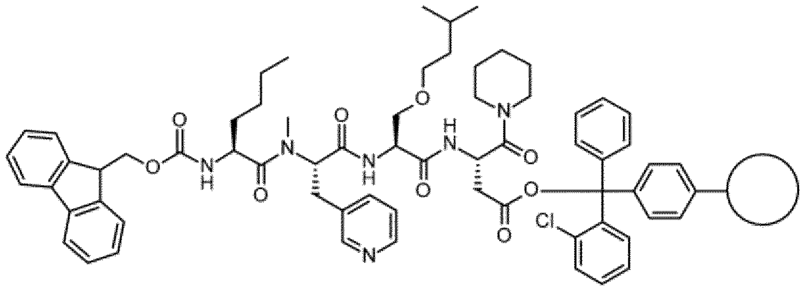

Compound 1-2-8

The compound was prepared from Fmoc-Asp(O-Trt(2-Cl)-resin)-pip (compound 1-2-2, 0.452 mmol/g).

Fmoc-Nle-MePhe(3-Cl)-Ser(iPen)-Asp-(O-Trt(2-Cl)-resin)-pip (SEQ ID NO: 5) (compound 1-2-9)

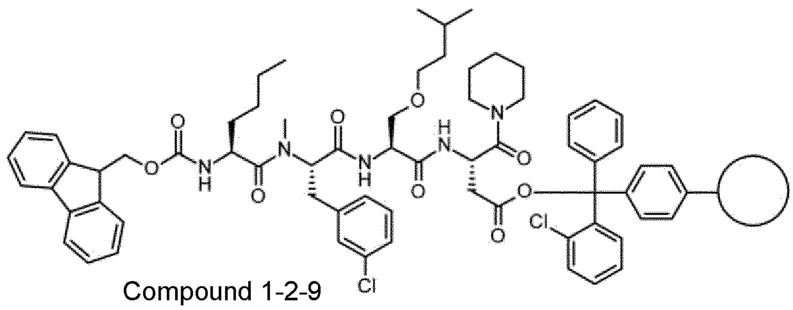

Compound 1-2-9

The compound was prepared from Fmoc-Asp(O-Trt(2-Cl)-resin)-pip (compound 1-2-2, 0.452 mmol/g).

Fmoc-Ser(nPr)-MeAla-Phe-Asp(O-Trt(2-Cl)-resin)-pip (SEQ ID NO: 6) (compound 1-2-10)

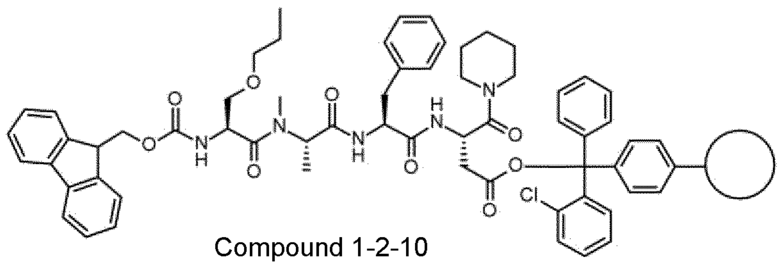

Compound 1-2-10

The compound was prepared from Fmoc-Asp(O-Trt(2-Cl)-resin)-pip (compound 1-2-2, 0.452 mmol/g).

Fmoc-Aib-Pro-D-3-Abu-O-Trt(2-Cl)-resin (compound 1-2-11)

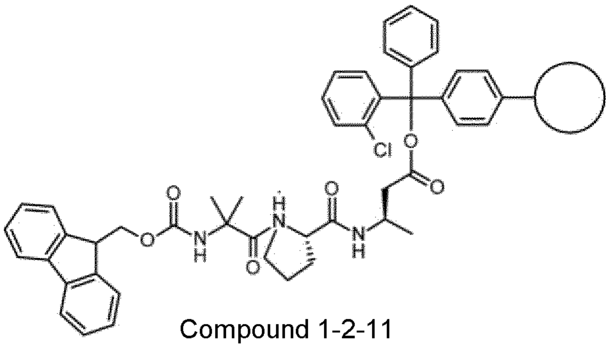

Compound 1-2-11

The compound was prepared from Fmoc-D-3-Abu-O-Trt(2-Cl)-resin (compound 1-2-3, 0.519 mmol/g).

Fmoc-cLeu-MeVal-D-3-Abu-O-Trt(2-Cl)-resin (compound 1-2-12)

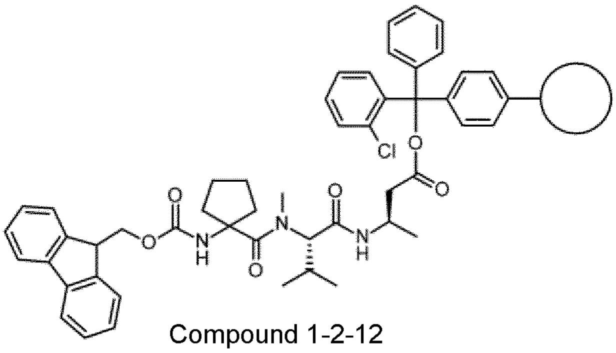

Compound 1-2-12

The compound was prepared from Fmoc-D-3-Abu-O-Trt(2-Cl)-resin (compound 1-2-3, 0.519 mmol/g).

Fmoc-Ala-MeAla-O-Trt(2-Cl)-resin (compound 1-2-13)

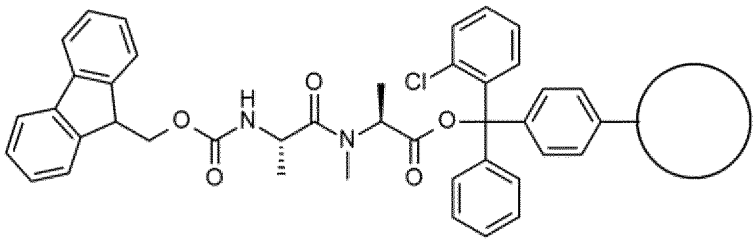

Compound 1-2-13

Compound 1-2-13 (Fmoc-Ala-MeAla-O-Trt(2-Cl)-resin, 0.408 mmol/g) was prepared by the same method as that for compounds 1-2-5 to 1-2-12 except that 1 g of Fmoc-MeAla-O-Trt(2-Cl)-resin (compound 1-2-4, 0.514 mmol/g) added in a 20 mL column with a filter, instead of an automated peptide synthesizer, to carry out a solid-phase reaction, and the resin was washed four times with DMF (7 mL) during the Fmoc deprotection step.

As an example, for the purpose of confirming that compound 1-2-5 was obtained, a peptide was isolated from a part of the obtained resin with a TFE/DCM solution (1/1 (v/v)) containing DIPEA (0.045 mol/L). The isolating solution was analyzed by LCMS, and the result showed that a target peptide (compound 1-2-5*) was formed. For compounds 1-2-6 to 1-2-12, formation of target peptides (compounds 1-2-6* to 1-2-12*) was confirmed by the same method as that for compound 1-2-5. In this Example, * added to a compound number indicates a compound examined with a peptide

isolated from the resin for checking the reaction. Compound 1-2-5* indicates a peptide compound in which a bond between a carboxylic acid of a peptide contained in compound 1-2-5 and a 2-chlorotrityl group of the resin is cleaved.

Fmoc-Ile-MeAla-Aze(2)-MeCha-MeGly-Asp-pyrro (SEQ ID NO: 9) (compound 1-2-5*)

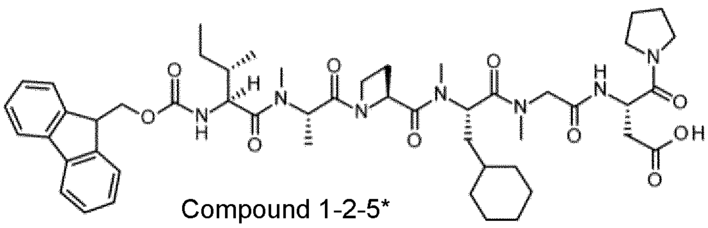

Compound 1-2-5*

LCMS (ESI) m/z = 928.79 (M+H)+
Retention time: 0.88 minutes (analysis condition SQDFA05)

Fmoc-Ile-MeGly-Aze(2)-MeCha-MeGly-Asp-pip (SEQ ID NO: 10) (compound 1-2-6*)

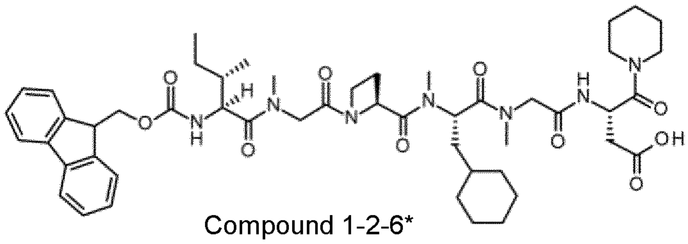

Compound 1-2-6*

LCMS (ESI) m/z = 928.84 (M+H)+
Retention time: 0.92 minutes (analysis condition SQDFA05)

Fmoc-Thr(tBu)-MeAla-Cys(StBu)-Phe-Asp-pip (SEQ ID NO: 11) (compound 1-2-7*)

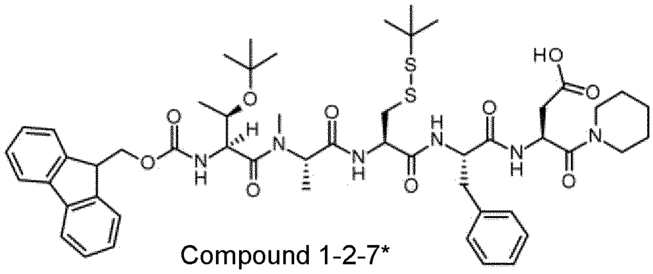

Compound 1-2-7*

LCMS (ESI) m/z = 1003.82 (M+H)+
Retention time: 1.11 minutes (analysis condition SQDFA05)

Fmoc-Nle-MeAla(3-Pyr)-Ser(iPen)-Asp-pip (SEQ ID NO: 12) (compound 1-2-8*)

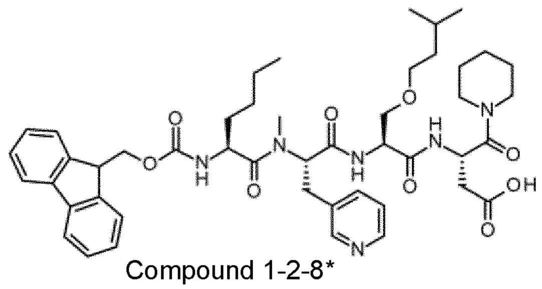

Compound 1-2-8*

LCMS (ESI) m/z = 855.83 (M+H)+
Retention time: 0.80 minutes (analytical condition SQDFA05)

Fmoc-Nle-MePhe(3-Cl)-Ser(iPen)-Asp-pip (SEQ ID NO: 13) (compound 1-2-9*)

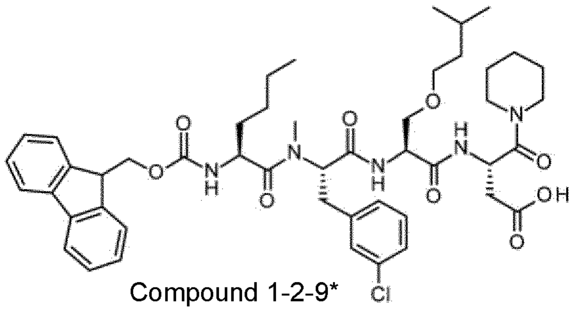

Compound 1-2-9*

LCMS (ESI) m/z = 888.78 (M+H)+
Retention time: 1.10 minutes (analysis condition SQDFA05)

Fmoc-Ser(nPr)-MeAla-Phe-Asp-pip (SEQ ID NO: 14) (compound 1-2-10*)

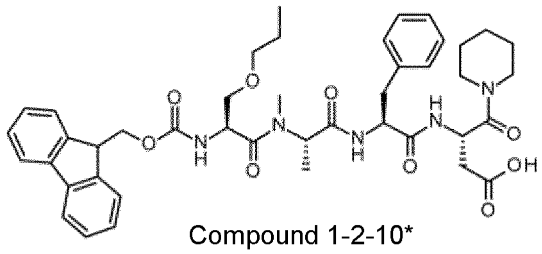

Compound 1-2-10*

LCMS (ESI) m/z = 784.75 (M+H)+
Retention time: 0.91 minutes (analytical condition SQDFA05)

Fmoc-Aib-Pro-D-3-Abu-OH (compound 1-2-11*)

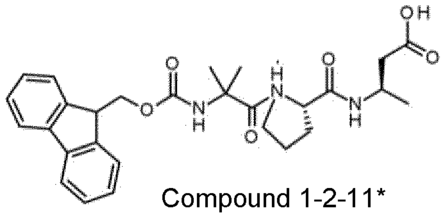

Compound 1-2-11*

LCMS (ESI) m/z = 508.60 (M+H)+
Retention time: 0.73 minutes (analysis condition SQDFA05)

Fmoc-cLeu-MeVal-D-3-Abu-OH (compound 1-2-12*)

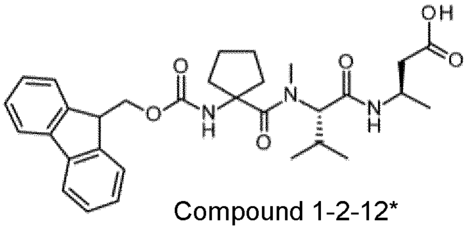

Compound 1-2-12*

LCMS (ESI) m/z = 326.56 (M-H)-
Detected as MS of fragment freed of Fmoc protection ((M-H-Fmoc)-)
Retention time: 0.85 minutes (analysis condition SQDFA05)

Fmoc-Ala-MeAla-OH (compound 1-2-13*)

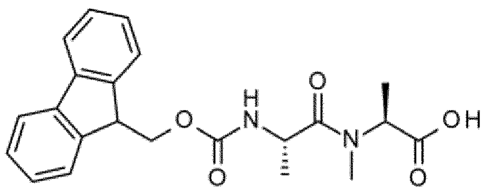

Compound 1-2-13*

LCMS(ESI) m/z = 397 (M+H)+
Retention time: 0.69 minutes (analytical condition SQDFA05)

Example 2: Experiment for examining effects of reagents and solvents on Fmoc deprotection step, resin washing step and elongation step in solid-phase synthesis of peptide

In solid-phase synthesis of a peptide, amino acid elongation is performed subsequently to Fmoc deprotection and resin washing. In this Example, compound 1-2-5 was used as a peptide-supporting solid phase resin, reagents and solvents in each of the steps were changed, and the degrees of reduction of impurities formed by peptide synthesis (diketopiperazine elimination compound, 6-membered cyclic amidine skeleton compound and excessive elongation compound) were compared to determine a range of appropriate conditions.

Basic operation A (Table 5, runs 1 to 24)

100 mg of the peptide-supporting solid-phase resin prepared in Example 1-2-5 (compound 1-2-5) was put in a solid-phase reaction vessel, DCM (1.0 mL) was added inside a glove box purged with nitrogen, and the mixture was

left standing for 10 minutes to swell the resin. Thereafter, the solution was discharged from a frit, and subsequently, the resin was washed twice with a solvent (0.7 mL per column) to be used during Fmoc deprotection. An Fmoc deprotection solution (0.7 mL per column) was added, and the mixture was reacted at room temperature for 10 minutes to perform deprotection of the Fmoc group. The Fmoc deprotection solution was prepared by dissolving a base shown in Table 5 in the Fmoc deprotection solvent at a volume percent concentration (v/v%) shown in Table 5. The solution was discharged from the frit, and the resin was then washed six times with a washing solvent (0.7 mL per column).

To the resin obtained as described above, a solution obtained by mixing a solution of Fmoc-MeLeu-OH (0.6 mol/L) and Oxyma (0.375 mol/L) in an elongation solvent (0.3 mL per column) and a solution of DIC (10 v/v%) in an elongation solvent (0.36 mL per column) was added, and the mixture was shaken at 40°C for 2.5 hours. When DCM was used as a solvent, the mixture was shaken at room temperature for 2.5 hours. The solution was discharged from the frit, and the resin was then washed four times with DMF (0.7 mL per column) and four times with DCM (0.7 mL per column), and dried.

For example, in run 3 in Table 5, the following procedure was carried out in accordance with basic operation A.

100 mg of the peptide-supporting solid-phase resin prepared in Example 1-2-5 (compound 1-2-5 prepared in Example 1-2-5 from compound 1-2-1 at 0.497 mmol/g) was put in a solid-phase reaction vessel, DCM (1.0 mL) was added inside a glove box purged with nitrogen, and the mixture was left standing for 10 minutes to swell the resin. Thereafter, the solution was discharged from a frit, and subsequently, the resin was washed twice with DMF (0.7 mL per column). A solution of 1,8-diazabicyclo[5.4.0]-7-undecene (DBU) in DMF (2 v/v%, 0.7 mL per column) was added, and the mixture was reacted at room temperature for 10 minutes to perform deprotection of an Fmoc group. The solution was discharged from the frit, and the resin was then washed six times with DMF (0.7 mL per column).

To the resin obtained as described above, a solution obtained by mixing a solution of Fmoc-MeLeu-OH (0.6 mol/L) and Oxyma (0.375 mol/L) in DMF (0.3 mL) and a solution of DIC (10 v/v%) in DMF (0.36 mL) was added, and the mixture was shaken at 40°C for 2.5 hours. The solution was discharged from the frit, and the resin was then washed four times with DMF (0.7 mL per column) and four times with DCM (0.7 mL per column), and dried.

For checking the progress of the reaction, a part of the obtained resin was taken out, swollen with DCM, and washed twice with DMF, a solution of 1,8-diazabicyclo[5.4.0]-7-undecene (DBU) in DMF (2 v/v%) was then added, and the mixture was reacted at room temperature for 10 minutes to perform deprotection of an Fmoc group. The solution was discharged from the frit, the resin was washed four times with DMF, and four times with DCM, a peptide was then isolated with a TFE/DCM solution (1/1 (v/v)) containing DIPEA (0.045 mol/L). The isolating solution was analyzed by LCMS (SQDAA05 long) to confirm formation of a target peptide compound (TM) (2-1*), a diketopiperazine (DKP) elimination compound (compound 2-2*), a 6-membered cyclic amidine skeleton compound (compound 2-3*) and a MeLeu excessive elongation compound (compound 2-4*).

Target peptide compound (TM), H-MeLeu-Ile-MeAla-Aze(2)-MeCha-MeGly-Asp-pyrro (SEQ ID NO: 15) (compound 2-1*)

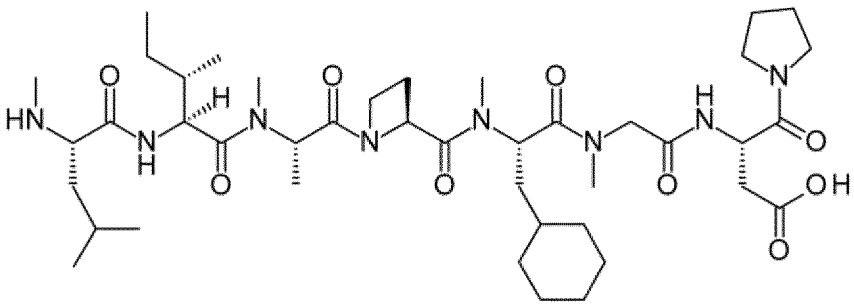

Compound 2-1*

LCMS (ESI) m/z = 833.79 (M+H)+
Retention time: 2.64 minutes (analysis condition SQDAA05 long)

Diketopiperazine elimination compound, H-MeLeu-Aze(2)-MeCha-MeGly-Asp-pyrro (SEQ ID NO: 16) (compound 2-2*)

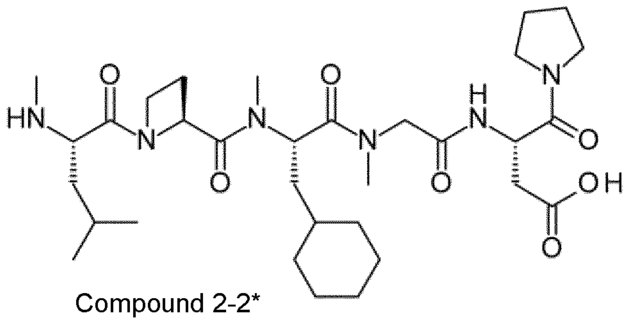

Compound 2-2*

LCMS (ESI) m/z = 635.58 (M+H)+
Retention time: 2.12 minutes (analysis condition SQDAA05 long)

6-membered cyclic amidine skeleton compound (compound 2-3*)

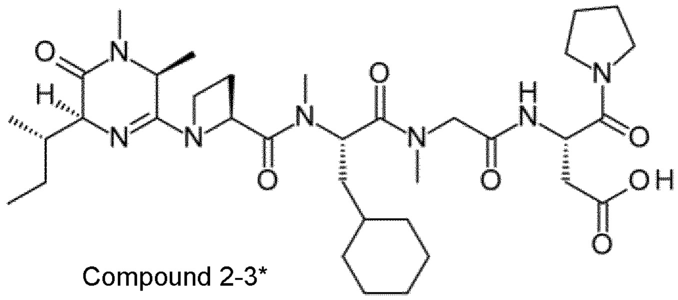

Compound 2-3*

LCMS (ESI) m/z = 688.65 (M+H)+
Retention time: 2.49 minutes (analysis condition SQDAA05 long)

MeLeu excessive elongation compound, H-MeLeu-MeLeu-Ile-MeAla-Aze(2)-MeCha-MeGly-Asp-pyrro (SEQ ID NO: 17) (compound 2-4*)

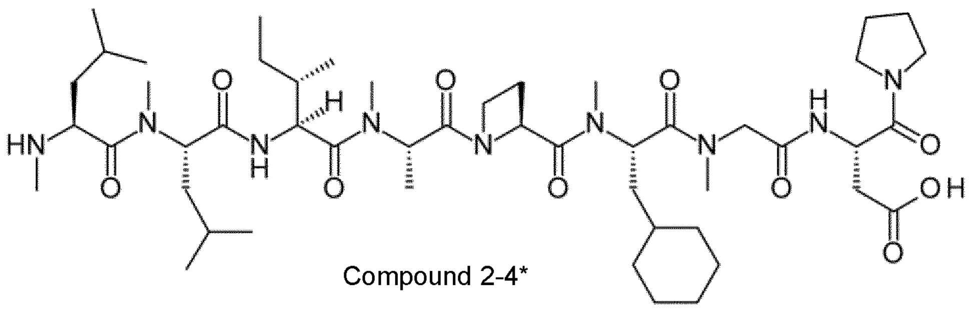

Compound 2-4*

LCMS (ESI) m/z = 960.90 (M+H)+
Retention time: 3.04 minutes (analysis condition SQDAA05 long)

Basic operation B (Table 5, runs 25 to 54)

100 mg of the peptide-supporting solid-phase resin prepared in Example 1-2-5 (compound 1-2-5) was put in a solid-phase reaction vessel, DCM (1.0 mL) was added inside a glove box purged with nitrogen, and the mixture was left standing for 10 minutes to swell the resin. Thereafter, the solution was discharged from a frit, and subsequently, the resin was washed twice with a solvent (0.7 mL per column) to be used during Fmoc deprotection. An Fmoc deprotection

solution (0.7 mL per column) was added, and the mixture was reacted at room temperature for 10 minutes to perform deprotection of the Fmoc group. The Fmoc deprotection solution was prepared by dissolving a base shown in Table 5 in the Fmoc deprotection solvent at a volume percent concentration (v/v%) shown in Table 5. The solution was discharged from the frit, and the resin was then washed six times with a washing solvent (0.7 mL per column).

To the resin obtained as described above, a solution obtained by mixing a solution of Fmoc-MeLeu-OH (4 equivalents) in an elongation solvent (0.25 mL), a solution of an uronium-type condensation agent or a phosphonium-type condensation agent (4 equivalents) in an elongation solvent (0.25 mL), and DIPEA (6 equivalents) and leaving the mixture to stand for about 1 to 2 minutes was added, and the mixture was shaken at room temperature for 4 hours. The solution was discharged from the frit, and the resin was then washed four times with DMF (0.7 mL per column) and four times with DCM (0.7 mL per column), and dried.

For example, in run 26 in Table 5, the following procedure was carried out in accordance with basic operation B.

100 mg of the peptide-supporting solid-phase resin prepared in Example 1-2-5 (compound 1-2-5 prepared in Example 1-2-5 from compound 1-2-1 at 0.552 mmol/g) was put in a solid-phase reaction vessel, DCM (1.0 mL) was added inside a glove box purged with nitrogen, and the mixture was left standing for 10 minutes to swell the resin. Thereafter, the solution was discharged from a frit, and subsequently, the resin was washed twice with toluene (0.7 mL per column). A solution of 1,8-diazabicyclo[5.4.0]-7-undecene (DBU) in toluene (2 v/v%, 0.7 mL per column) was added, and the mixture was reacted at room temperature for 10 minutes to perform deprotection of an Fmoc group. The solution was discharged from the frit, and the resin was then washed six times with toluene (0.7 mL per column).

To the resin obtained as described above, a solution obtained by mixing a solution of Fmoc-MeLeu-OH (0.221 mmol, 4 equivalents) in DMF (0.25 mL), a solution of [ethylcyano(hydroxyimino)acetato-O2)tri 1-pyrrolidinylphosphonium hexafluorophosphate (PyOxim) (0.221 mmol, 4 equivalents) in DMF (0.25 mL), and DIPEA (0.057 mL, 0.331 mmol, 6 equivalents) and leaving the mixture to stand for about 1 to 2 minutes was added, and the mixture was shaken at room temperature for 4 hours. The solution was discharged from the frit, and the resin was then washed four times with DMF (0.7 mL per column) and four times with DCM (0.7 mL per column), and dried.

For checking the progress of the reaction, a part of the obtained resin was taken out, swollen with DCM, and washed twice with DMF, a solution of 1,8-diazabicyclo[5.4.0]-7-undecene (DBU) in DMF (2 v/v%) was then added, and the mixture was reacted at room temperature for 10 minutes to perform deprotection of an Fmoc group. The solution was discharged from the frit, the resin was washed four times with DMF, and four times with DCM, a peptide was then isolated with a TFE/DCM solution (1/1 (v/v)) containing DIPEA (0.045 mol/L). The isolating solution was analyzed by LCMS (SQDAA05 long).

<u>Basic operation C (Table 5, run 55)</u>

100 mg of the peptide-supporting solid-phase resin prepared in Example 1-2-5 (compound 1-2-5 prepared in Example 1-2-5 from compound 1-2-1 at 0.552 mmol/g) was put in a solid-phase reaction vessel, DCM (1.0 mL) was added inside a glove box purged with nitrogen, and the mixture was left standing for 10 minutes to swell the resin. Thereafter, the solution was discharged from a frit, and subsequently, the resin was washed twice with toluene (0.7 mL per column). A solution of 1,8-diazabicyclo[5.4.0]-7-undecene (DBU) in toluene (2 %v/v, 0.7 mL per column) was added, and the mixture was reacted at room temperature for 10 minutes to perform deprotection of an Fmoc group. The solution was discharged from the frit, and the resin was then washed six times with toluene (0.7 mL per column).

To the resin obtained as described above, a mixture of a solution of Fmoc-MeLeu-Cl (described later) (0.221 mmol, 4 equivalents) in NMP (0.7 mL) and 2,4,6-trimethylpyridine (0.55 mmol, 10 equivalents) was added, and the mixture was shaken at 40°C for 1.5 hours. The solution was discharged from the frit, and the resin was then washed four times with DMF (0.7 mL per column) and four times with DCM (0.7 mL per column), and dried. Fmoc-MeLeu-Cl was prepared in the following manner. (2S)-2-[9H-fluoren-9-ylmethoxycarbonyl(methyl)amino]-4-methylpentanoic acid (Fmoc-MeLeu-OH) (80.8 mg, 0.22 mmol) was dissolved in dichloromethane (0.44 mL), and DMF (1.70 μL, 0.22 mmol) was added dropwise while the solution was cooled in an ice bath. Next, thionyl chloride (24.1 μL, 0.33 mmol) was added. The obtained reaction mixture was stirred at room temperature for 30 minutes, and then concentrated under reduced pressure. To the obtained residue was added toluene (0.81 mL, 10 v/w), and the mixture was concentrated twice under reduced pressure. The obtained crude product was dissolved in NMP (0.7 mL), and the solution was used for the above-described reaction.

For checking the progress of the reaction, a part of the obtained resin was taken out, swollen with DCM, and washed twice with DMF, a solution of 1,8-diazabicyclo[5.4.0]-7-undecene (DBU) in DMF (2 v/v%) was then added, and the mixture was reacted at room temperature for 10 minutes to perform deprotection of an Fmoc group. The solution was discharged from the frit, the resin was washed four times with DMF, and four times with DCM, a peptide was then isolated with a TFE/DCM solution (1/1 (v/v)) containing DIPEA (0.045 mol/L). The isolating solution was analyzed by LCMS (SQDAA05 long).

Formation of a diketopiperazine elimination compound is a side reaction that is commonly known in peptide

synthesis. Formation of a 6-membered cyclic amidine skeleton compound has not been reported previously, and is a new peptide synthesis problem found by the inventors of the present application. Without being bound by a specific theory, formation of a diketopiperazine elimination compound proceeds as the amino group at the N-terminal nucleophilically attacks a carbonyl group forming the second amide bond from the N-terminal so that a 6-membered ring is formed, followed by elimination of the original amino group forming the amide bond. On the other hand, formation of a 6-membered cyclic amidine skeleton compound proceeds as a 6-membered ring is similarly formed, followed by elimination of a carbonyl group-derived hydroxy group formed by nucleophilic attack. That is, formation of a diketopiperazine elimination compound and formation a 6-membered amidine skeleton compound are peptide synthesis problems caused by formation of a 6-membered ring in common.

Table 5 below shows the obtained results (the relative ratios of UV areas in LCMS for compound 2-1*, compound 2-2*, compound 2-3* and compound 2-4* under respective conditions are expressed as a percentage).

[Table 5]

| | Reaction condition | | | | | Area % | | | |
|---|---|---|---|---|---|---|---|---|---|
| | Fmoc deprotection | | Washing | Elongation | | | | | |
| run | Base | Solvent | Solvent | Condensation agent | Solvent | TM-DKP (Compound 2-2*) | Amidine form (Compound 2-3*) | TM (Compound 2-1*) | MeLeu Excessive elongation (Compound 24*) |
| 1 | 20% pip | DMF | DMF x 6 | DIC/oxyma | DMF | 7.56 | 8.05 | 84.39 | 0 |
| 2 | 4% TMG | DMF | DMF x 6 | DIC/oxyma | DMF | 1.28 | 3.26 | 95.46 | 0 |
| 3 | 2% DBU | DMF | DMF x 6 | DIC/oxyma | DMF | 2.42 | 5.41 | 90.18 | 2.00 |
| 4 | 4% MTBD | DMF | DMF x 6 | DIC/oxyma | DMF | 2.46 | 5.57 | 91.97 | 0 |
| 5 | 4% HP1 (dma) | DMF | DMF x 6 | DIC/oxyma | DMF | 0.92 | 2.04 | 97.04 | 0 |
| 6 | 2% DBU | DCM | DCM x 6 | DIC/oxyma | DCM | 0.36 | 1.05 | 94.66 | 3.93 |
| 7 | 2% DBU | toluene | toluene x 6 | DIC/oxyma | toluene | 0.34 | 1.26 | 81.12 | 17.28 |
| 8 | 2% DBU | cumene | cumene x 6 | DIC/oxyma | cumene | 0.21 | 0.79 | 87.30 | 11.70 |
| 9 | 2% DBU | THF | THF x 6 | DIC/oxyma | THF | 0.94 | 2.24 | 81.56 | 15.26 |
| 10 | 2% DBU | DME | DME x 6 | DIC/oxyma | DME | 0.59 | 1.31 | 89.06 | 9.04 |
| 11 | 2% DBU | 1,3-dioxolane | 1,3-dioxolane x 6 | DIC/oxyma | 1,3-dioxolane | 0.42 | 1.25 | 93.62 | 4.71 |
| 12 | 2% DBU | 2-MeTHF | 2-MeTHF x 6 | DIC/oxyma | 2-MeTHF | 0.91 | 2.43 | 90.02 | 6.64 |
| 13 | 2% DBU | tributyl phosphate | tributyl phosphate x 6 | DIC/oxyma | tributyl phosphate | 0.71 | 1.53 | 75.88 | 21.88 |
| 14 | 2% DBU | Methyl Propionate | Methyl Propionate x 6 | DIC/oxyma | Methyl Propionate | 0.39 | 1.55 | 89.05 | 9.01 |
| 15 | 2% DBU | butyl acetate | butyl acetate x 6 | DIC/oxyma | butyl acetate | 0.24 | 0.48 | 75.05 | 24.23 |

(continued)

| | Reaction condition | | | | | Area % | | | |
|---|---|---|---|---|---|---|---|---|---|
| | Fmoc deprotection | | Washing | Elongation | | | | | |
| run | Base | Solvent | Solvent | Condensation agent | Solvent | TM-DKP (Compound 2-2*) | Amidine form (Compound 2-3*) | TM (Compound 2-1*) | MeLeu Excessive elongation (Compound 24*) |
| 16 | 2% DBU | 3-Pentanone | 3-Pentanone x 6 | DIC/oxyma | 3-Pentanone | 0.61 | 2.49 | 88.60 | 8.30 |
| 17 | 2% DBU | MEK | MEK x 6 | DIC/oxyma | MEK | 0.32 | 1.17 | 86.09 | 12.42 |
| 18 | 2% DBU | dimethyl carbonate | dimethyl carbonatex 6 | DIC/oxyma | dimethyl carbonate | 0.27 | 1.12 | 84.25 | 14.36 |
| 19 | 2% DBU | DMA | DMA x 6 | DIC/oxyma | DMA | 1.63 | 3.06 | 90.63 | 4.68 |
| 20 | 2% DBU | NMP | NMP x 6 | DIC/oxyma | NMP | 1.93 | 4.27 | 86.43 | 7.37 |
| 21 | 2% DBU | DMI | DMI x 6 | DIC/oxyma | DMI | 1.59 | 2.95 | 95.07 | 0.39 |
| 22 | 2% DBU | toluene | toluene x 6 | DIC/oxyma | EtOAc | 0.36 | 1.01 | 82.56 | 16.07 |
| 23 | 2% DBU | toluene | toluene x 6 | DIC/oxyma | MEK | 0.39 | 1.16 | 81.99 | 16.46 |
| 24 | 2% DBU | toluene | toluene x 6 | DIC/oxyma | 2-MeTHF | 0.67 | 2.00 | 95.50 | 1.83 |
| 25 | 2% DBU | DCM | DCM x 6 | PyOxim/DIPEA | DCM | 0.52 | 0.38 | 99.10 | 0 |
| 26 | 2% DBU | toluene | toluene x 6 | PyOxim/DIPEA | DMF | 0.52 | 0.68 | 98.45 | 0.34 |
| 27 | 2% DBU | toluene | toluene x 6 | TATU/DIPEA | DMF | 0.42 | 1.29 | 97.36 | 0.94 |
| 28 | 2% DBU | toluene | toluene x 6 | HATU/DIPEA | DMF | 0.51 | 3.88 | 93.38 | 2.22 |
| 29 | 2% DBU | toluene | toluene x 6 | TOTU/DIPEA | DMF | 0.54 | 1.01 | 97.97 | 0.47 |
| 30 | 2% DBU | toluene | toluene x 6 | HOTU/DIPEA | DMF | 0.54 | 1.18 | 97.90 | 0.38 |
| 31 | 2% DBU | toluene | toluene x 6 | PyAOP/DIPEA | DMF | 0.64 | 0.40 | 98.28 | 0.68 |
| 32 | 2% DBU | toluene | toluene x 6 | PyBOP/DIPEA | DMF | 0.42 | 0.79 | 98.61 | 0.18 |
| 33 | 2% DBU | toluene | toluene x 6 | COMU/DIPEA | DMF | 0.45 | 0.82 | 98.19 | 0.54 |
| 34 | 2% DBU | toluene | toluene x 6 | HBTU/DIPEA | DMF | 0.65 | 1.49 | 96.78 | 1.08 |

(continued)

| | Reaction condition | | | | | Area % | | | |
|---|---|---|---|---|---|---|---|---|---|
| | Fmoc deprotection | | Washing | Elongation | | | | | |
| run | Base | Solvent | Solvent | Condensation agent | Solvent | TM-DKP (Compound 2-2*) | Amidine form (Compound 2-3*) | TM (Compound 2-1*) | MeLeu Excessive elongation (Compound 24*) |
| 35 | 2% DBU | toluene | toluene x 6 | HCTU/DIPEA | DMF | 0.46 | 1.32 | 97.72 | 0.50 |
| 36 | 2% DBU | toluene | toluene x 6 | TDBTU/DIPEA | DMF | 0.48 | 0.73 | 98.03 | 0.76 |
| 37 | 2% DBU | toluene | toluene x 6 | TBTU/DIPEA | DMF | 0.81 | 2.56 | 96.00 | 0.63 |
| 38 | 2% DBU | toluene | toluene x 6 | TCTU/DIPEA | DMF | 0.76 | 1.43 | 97.28 | 0.53 |
| 39 | 2% DBU | toluene | toluene x 6 | PyOxim/DIPEA | DMSO | 0.48 | 1.00 | 98.52 | 0 |
| 40 | 2% DBU | toluene | toluene x 6 | PyOxim/DIPEA | DMI | 0.62 | 1.61 | 97.42 | 0.35 |
| 41 | 2% DBU | toluene | toluene x 6 | PyOxim/DIPEA | NMP | 0.69 | 1.63 | 97.43 | 0.25 |
| 42 | 2% DBU | THF | THF x 6 | PyOxim/DIPEA | DMF | 0.89 | 0.79 | 98.32 | 0 |
| 43 | 2% DBU | THF | THF x 6 | TOTU/DIPEA | DMF | 0.98 | 0.88 | 98.14 | 0 |
| 44 | 20% pip | DCM | DCM x 6 | PyOxim/DIPEA | DCM | 0.68 | 7.25 | 92.06 | 0 |
| 45 | 4% HPI (dma) | DCM | DCM x 6 | PyOxim/DIPEA | DCM | 0.36 | 0.33 | 99.31 | 0 |
| 46 | 20% pip | THF | THF x 6 | PyOxim/DIPEA | DCM | 9.43 | 7.93 | 82.64 | 0 |
| 47 | 4% TMG | THF | THF x 6 | PyOxim/DIPEA | DCM | 1.06 | 2.13 | 96.81 | 0 |
| 48 | 4% HP1 (dma) | THF | THF x 6 | PyOxim/DIPEA | DCM | 0.30 | 0.82 | 98.88 | 0 |
| 49 | 20% pip | toluene | toluene x 6 | PyOxim/DIPEA | DCM | 3.45 | 8.26 | 88.29 | 0 |
| 50 | 4% HPI (dma) | toluene | toluene x 6 | PyOxim/DIPEA | DCM | 0.29 | 0.89 | 98.82 | 0 |
| 51 | 2% DBU/ 2% pip | DCM | DCM x 6 | PyOxim/DIPEA | DCM | 0.58 | 0.32 | 99.10 | 0 |

(continued)

| | Reaction condition | | | | | Area % | | | |
|---|---|---|---|---|---|---|---|---|---|
| | Fmoc deprotection | | Washing | Elongation | | | | | |
| run | Base | Solvent | Solvent | Condensation agent | Solvent | TM-DKP (Compound 2-2*) | Amidine form (Compound 2-3*) | TM (Compound 2-1*) | MeLeu Excessive elongation (Compound 24*) |
| 52 | 2% DBU/ 4% HP1 (dma) | DCM | DCM x 6 | PyOxim/DIPEA | DCM | 0.38 | 0.50 | 99.12 | 0 |
| 53 | 2% DBU/ 4% P1-tBu | DCM | DCM x 6 | PyOxim/DIPEA | DCM | 0.61 | 0.54 | 98.86 | 0 |
| 54 | 2% DBU/ 4% P2-Et | DCM | DCM x 6 | PyOxim/DIPEA | DCM | 0.43 | 0.49 | 99.09 | 0 |
| 55 | 2% DBU | toluene | toluene x 6 | Fmoc-MeLeu-Cl/ 2,4,6-Trimethyl pyridine | NMP | 0.87 | 1.03 | 98.10 | 0 |

Run 1 includes Fmoc deprotection step, a resin washing step and an elongation step whitch are generally carried out in common peptide synthesis (comparative experiment). For the base in Fmoc deprotection, piperidine (pip) (pKa: 19.35 (in acetonitrile), reference: Eur. J. Org. Chem. 2019, 6735-6748) is used. It was confirmed that under this condition, a diketopiperazine (DKP) elimination compound lacking two residues and a 6-membered cyclic amidine skeleton compound were each formed at about 8%. On the other hand, it was confirmed that use of a stronger base having a pKa of 23 or more in a conjugate acid enabled suppression of formation of a diketopiperazine elimination compound and a 6-membered cyclic amidine skeleton compound as in runs 2 to 5.

Next, the base in the Fmoc deprotection step was fixed as DBU, and studies were conducted on solvents in the Fmoc deprotection step, the resin washing step and the elongation step. As a result, it was confirmed that as in runs 6 to 18, it was possible to suppress formation of a diketopiperazine elimination compound and a 6-membered cyclic amidine skeleton compound in comparison with run 3 by changing the solvent used from DMF to a halogen solvent (DCM), an aromatic hydrocarbon solvent (toluene or cumene), an ether solvent (THF, DME, 1,3-dioxolane or 2-methyltetrahydrofuran), a phosphoric acid ester solvent (tributyl phosphate), an ester solvent (methyl propionate or butyl acetate), a ketone solvent (diethyl ketone or methyl ethyl ketone), or a carbonate solvent (dimethyl carbonate). This result is a result contrasting to the fact that in runs 19 to 21, there was no suppressing effect on formation of a diketopiperazine elimination compound and an amidine form when an amide solvent other than DMF (DMA or NMP) or DMI was used. A group of solvents confirmed to suppress formation of a diketopiperazine elimination compound and a 6-membered amidine skeleton compound have a DN (donor number) value of 26 or less in common. On the other hand, a group of amide solvents and urea solvents having no formation suppressing effect have a DN (donor number) value of more than 26 in common. Without being bound by a specific theory, the result of performing calculation of energy until diketopiperazine elimination through a 6-membered ring intermediate shows that the higher the hydrogen bond accepting capacity of a solvent, the greater the stabilization by coordination, and the donor number (DN) value as an index of the unshared electron pair donating property of the solvent may be an important parameter. For discussing the results of the studies on solvents on the basis of the above-mentioned premises, it is considered that a reason why formation of a diketopiperazine elimination compound and a 6-membered amidine skeleton compound was suppressed with a solvent having a DN value of 26 or less is that a reaction through a 6-membered ring intermediate was suppressed, and it is thought that a wide range of solvents having a DN value of 26 or less as well as the above-described group of solvents can be applied to the present invention. It was confirmed that it was possible to suppress formation of a diketopiperazine elimination compound and an amidine form even when as in runs 22 to 24, solvents in the Fmoc deprotection step and the resin washing step were fixed as toluene, and the elongation solvent was changed to ethyl acetate, methyl ethyl ketone or 2-methyltetrahydrofuran. From the results of a series of studies, it was confirmed that formation of an excessive elongation compound could be another problem of decreasing the purity of a peptide product.

The results of carrying out the elongation step under conditions found by the inventors of the present application, in addition to the Fmoc deprotection step and the resin washing step, are shown in runs 25 to 43, 45, 47, 48 and 50. Under these conditions, a suppressing effect on formation of an excessive elongation compound in addition to a diketopiperazine elimination compound and a 6-membered cyclic amidine skeleton compound was observed, and it was confirmed that a target peptide was obtained with a high purity.

As in the results shown in runs 44, 46 and 49, it was once again confirmed that even though the resin washing step, and the elongation step under conditions found by the inventors of the present application were carried out using a solvent during Fmoc deprotection which had been found by the inventors of the present application, it was not possible to suppress formation of a diketopiperazine elimination compound and a 6-membered cyclic amidine skeleton compound when piperidine that is most commonly used was used as a base during Fmoc deprotection.

In the present invention, two or more bases may coexist at a time in the Fmoc deprotection step. In a case in which two bases coexist, for example, even when piperidine generally known to give an adduct to dibenzofulvene formed in the Fmoc deprotection step coexists, formation of a diketopiperazine elimination compound and a 6-membered cyclic amidine skeleton compound can be suppressed as long as an optimum base (here DBU) is used as one of the bases as in run 51.

When HP1 (dma), P1tBu and P2Et having stronger basicity over DBU coexisted, isomerization (epimerization) of a target peptide slightly proceeded, but it was confirmed that it was possible to suppress formation of a diketopiperazine elimination compound and a 6-membered cyclic amidine skeleton compound (runs 52 to 54).

In the present invention, an acid chloride may be used in the elongation step. It was confirmed that for example, as in run 55, it was possible to suppress formation of a diketopiperazine elimination compound and a 6-membered cyclic amidine skeleton compound even when a separately prepared acid chloride was applied in NMP in the presence of 2,4,6-trimethylpyridine after the resin washing step was carried out using a solvent during Fmoc deprotection which had been found by the inventors of the present application.

Thus, from the results of Example 2, outlined conditions for each of the steps (Fmoc deprotection step, resin washing step and elongation step) which enable suppression of formation of a diketopiperazine elimination compound and a 6-membered cyclic amidine skeleton compound were determined. In suppression of formation of a diketopiperazine

elimination compound and a 6-membered cyclic amidine skeleton compound, the combination of reaction conditions is not limited to that shown in Example 2.

Example 3: Experiment of peptide synthesis with neutralization step (using base) added in Fmoc deprotection step in solid-phase synthesis of peptide

Some documents describe a case in which a base used in the Fmoc deprotection step is neutralized in the Fmoc deprotection step or the subsequent resin washing step.

For example, in solid-phase peptide synthesis, a case is known in which for the purpose of suppressing diketopiperazine elimination, an Fmoc deprotection reaction is quickly carried out in DBU/DMF, followed by addition of HOBt to perform neutralization (Org. Lett., 2008, 10, 3857-3860.).

In peptide synthesis using an anchor group, a case has been reported in which for carrying out an Fmoc deprotection reaction and subsequent elongation on one pot, neutralization is performed using hydrochloric acid or the like after Fmoc deprotection using DBU (WO 2012165546 A1).

An experiment was conducted for examining an effect of such a neutralization step on formation of a diketopiperazine elimination compound.

Basic operation (Table 6)

100 mg of the peptide-supporting solid-phase resin prepared in Example 1-2-5 (compound 1-2-5 prepared in Example 1-2-5 from compound 1-2-1 at 0.552 mmol/g) was put in a solid-phase reaction vessel, DCM (1.0 mL) was added inside a glove box purged with nitrogen, and the mixture was left standing for 10 minutes to swell the resin. Thereafter, the solution was discharged from a frit, and subsequently, the resin was washed twice with a solvent (0.7 mL per column) to be used during Fmoc deprotection, which is shown in Table 6. An Fmoc deprotection solution (0.7 mL per column) was added, and the mixture was left standing at room temperature for 10 minutes to perform deprotection of the Fmoc group. The Fmoc deprotection solution was prepared by dissolving a base shown in Table 6 in the Fmoc deprotection solvent at a volume percent concentration (v/v%) shown in Table 6. Subsequently, as a neutralization step, 0.4 mL of a solution of HOAt (0.25 mol/L or 0.5 mol/L) in DMF (about 2 equivalents or 4 equivalents to a solid phase-supported peptide) was added, and the mixture was left standing for 5 minutes. As a comparative experiment, an experiment was also conducted in parallel in which the Fmoc deprotection step was carried out for 10 minutes without carrying out the neutralization step, DMF (0.4 mL) was then added, and the mixture was left standing for 5 minutes. The solution was discharged from the frit, and the resin was then washed six times with a washing solvent shown in Table 6 (0.7 mL per column).

To the resin obtained as described above, a solution obtained by mixing a solution of Fmoc-MeLeu-OH (0.221 mmol, 4 equivalents) in DCM (0.3 mL), a solution of [ethylcyano(hydroxyimino)acetato-O2]tri 1-pyrrolidinylphosphonium hexafluorophosphate (PyOxim) (0.221 mmol, 4 equivalents) in DCM (0.3 mL), and DIPEA (0.057 mL, 0.331 mmol, 6 equivalents) and leaving the mixture to stand for about 1 to 2 minutes was added, and the mixture was shaken at room temperature for 2 hours or 4 hours. The solution was discharged from the frit, and the resin was then washed four times with DMF (0.7 mL per column) and four times with DCM (0.7 mL per column), and dried.

For example, in run 2 in Table 6, the following procedure was carried out in accordance with the basic operation.

100 mg of the peptide-supporting solid-phase resin prepared in Example 1-2-5 (compound 1-2-5 prepared in Example 1-2-5 from compound 1-2-1 at 0.552 mmol/g) was put in a solid-phase reaction vessel, DCM (1.0 mL) was added inside a glove box purged with nitrogen, and the mixture was left standing for 10 minutes to swell the resin. Thereafter, the solution was discharged from the frit, and subsequently, a solution of 1,8-diazabicyclo[5.4.0]-7-undecene (DBU) in DCM (2 v/v%, 0.7 mL per column) was added. The mixture was left standing at room temperature for 10 minutes to perform Fmoc deprotection. Subsequently, as a neutralization step, 0.4 mL of a solution of HOAt (0.5 mol/L) in DMF (about 4 equivalents to a solid phase-supported peptide) was added, and the mixture was left standing for 5 minutes. The solution was discharged from the frit, and the resin was then washed six times with DCM (0.7 mL per column).

To the resin obtained as described above, a solution obtained by mixing a solution of Fmoc-MeLeu-OH (0.221 mmol, 4 equivalents) in DCM (0.3 mL), a solution of [ethylcyano(hydroxyimino)acetato-O2]tri 1-pyrrolidinylphosphonium hexafluorophosphate (PyOxim) (0.221 mmol, 4 equivalents) in DCM (0.3 mL), and DIPEA (0.057 mL, 0.331 mmol, 6 equivalents) and leaving the mixture to stand for about 1 to 2 minutes was added, and the mixture was shaken at room temperature for 4 hours. The solution was discharged from the frit, and the resin was then washed four times with DMF (0.7 mL per column) and four times with DCM (0.7 mL per column), and dried.

For checking the progress of the reaction, a part of the obtained resin was taken out, swollen with DCM, and washed twice with DMF, a solution of 1,8-diazabicyclo[5.4.0]-7-undecene (DBU) in DMF (2 v/v%) was then added, and the mixture was reacted at room temperature for 10 minutes to perform deprotection of an Fmoc group. The solution was discharged from the frit, the resin was washed four times with DMF, and four times with DCM, a peptide was then

isolated with a TFE/DCM solution (1/1 (v/v)) containing DIPEA (0.045 mol/L). The isolating solution was analyzed by LCMS (SQDAA05 long), and the results shown in Table 6 were obtained.

[Table 6]

| | Reaction conditions | | | | | Area % | | | |
|---|---|---|---|---|---|---|---|---|---|
| | Fmoc deprotection | | Washing | Elongation | | | | | |
| run | Base | Solvent | Solvent | Condensation agent | Solvent | TM-DKP (Compound 2-2*) | Amidine form (Compound 2-3*) | TM (Compound 2-1*) | MeLeu **Excessive** elongation (Compound 2-4*) |
| 1 | 2% DBU | DCM (中和工程なし) | DCM x 6 | PyOxim/DIPEA | DCM | 0.36 | 0.19 | 99.45 | 0 |
| 2 | 2% DBU | DCM (HOAt 4 eqで中和) | DCM x 6 | PyOxim/DIPEA | DCM | 0.79 | 0.76 | 98.45 | 0 |
| 3 | 2% DBU | THF (中和工程なし) | THF x 6 | PyOxim/DIPEA | DCM | 0.59 | 0.86 | 98.56 | 0 |
| 4 | 2% DBU | THF (HOAt 2 eqで中和) | THF x 6 | PyOxim/DIPEA | DCM | 1.42 | 1.52 | 97.06 | 0 |
| 5 | 2% DBU | THF (HOAt 4 eqで中和) | THF x 6 | PyOxim/DIPEA | DCM | 5.15 | 25.08 | 69.77 | 0 |

Thus, from the results of Example 3, it was confirmed that when the neutralization step was carried out, the purity of a target peptide decreased as the formation of a diketopiperazine elimination compound and a 6-membered cyclic amidine skeleton compound increased in comparison with a case where the neutralization step was not carried out.

Example 4: Experiment for examining universality of reducing effect on diketopiperazine elimination during peptide synthesis under found conditions for peptide sequence

In this Example, the amino acid described in Core 1 was elongated by a common conventional method (condition-1) and the method of the present invention (condition-2) for various peptide sequences prepared in Example 1-2-5 (peptide sequences elongated to Core 2 in Table 7, compounds 1-2-6 to 1-2-12), and the amounts of the diketopiperazine elimination compound formed were compared.

Condition-1 (conventional method)

100 mg of the peptide-supporting solid-phase resin prepared in Example 1-2-5 (compounds 1-2-6 to 1-2-12 (corresponding to runs 1 to 7, respectively, in Table 7) was put in a solid-phase reaction vessel, DCM (1.0 mL) was added inside a glove box purged with nitrogen, and the mixture was left standing for 10 minutes to swell the resin. Thereafter, the solution was discharged from a frit, and subsequently, the resin was washed twice with DMF (0.7 mL per column). A solution of piperidine in DMF (20 v/v%, 0.7 mL per column) was added, and the mixture was reacted at room temperature for 10 minutes to perform deprotection of the Fmoc group. The solution was discharged from the frit, and the resin was then washed eight times with DMF (0.7 mL per column).

To the resin obtained as described above, a solution obtained by mixing a solution of an Fmoc-protected amino acid (0.6 mol/L) and HOAt (0.375 mol/L) in DMF (0.3 mL) and a solution of DIC (10 v/v%) in DMF (0.36 mL) was added, and the mixture was shaken at 40°C for 4 hours. The solution was discharged from the frit, and the resin was then washed four times with DMF (0.7 mL per column) and four times with DCM (0.7 mL per column), and dried.

Condition-2 (method of the present invention)

100 mg of the peptide-supporting solid-phase resin prepared in Example 1-2-5 (compounds 1-2-6 to 1-2-12 (corresponding to runs 1 to 7, respectively, in Table 7) was put in a solid-phase reaction vessel, DCM (1.0 mL) was added inside a glove box purged with nitrogen, and the mixture was left standing for 10 minutes to swell the resin. Thereafter, the solution was discharged from a frit, and subsequently, the resin was washed twice with toluene (0.7 mL per column). A solution of 1,8-diazabicyclo[5.4.0]-7-undecene (DBU) in toluene (2 v/v%, 0.7 mL per column) was added, and the mixture was reacted at room temperature for 10 minutes to perform deprotection of an Fmoc group. The solution was discharged from the frit, and the resin was then washed twice with toluene (0.7 mL per column) and twice with DCM (0.7 mL per column).

To the resin obtained as described above, a solution obtained by mixing a solution of an Fmoc-protected amino acid (4 equivalents) in DCM (0.25 mL), a solution of [ethylcyano(hydroxyimino)acetato-O2)tri 1-pyrrolidinylphosphonium hexafluorophosphate (PyOxim) (4 equivalents) in DCM (0.25 mL), and DIPEA (6 equivalents) and leaving the mixture to stand for about 1 to 2 minutes was added, and the mixture was shaken at room temperature for 4 hours. The solution was discharged from the frit, and the resin was then washed four times with DMF (0.7 mL per column) and four times with DCM (0.7 mL per column), and dried.

For checking the progress of reaction, a part of each of the obtained resins was taken out, and swollen with DCM, and a peptide was then isolated with a TFE/DCM solution (1/1 (v/v)) containing DIPEA (0.045 mol/L). The isolating solution was analyzed by LCMS (SQDFA05 long) to confirm formation of a target peptide (TM) (compounds 4-1-1* to 4-1-7*) and a diketopiperazine elimination compound (compounds 4-2-1* to 4-2-7*).

Table 7 shows the amount of the diketopiperazine (DKP) elimination compound formed for each sequence and each condition. The amount of the diketopiperazine (DKP) elimination compound formed shown in Table 7 is a UV area percent of the diketopiperazine (DKP) elimination compound when the total UV area percent of the target peptide (TM) and the diketopiperazine (DKP) elimination compound in LCMS is 100 percent.

Target peptide compound (TM), Fmoc-MeLeu-Ile-MeGly-Aze(2)-MeCha-MeGly-Asp-pip (SEQ ID NO: 18) (run 1) (compound 4-1-1*)

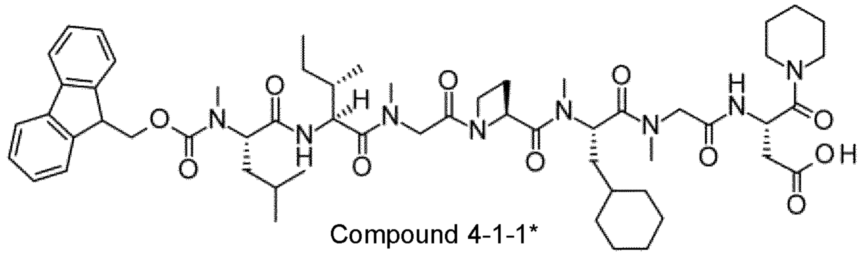

Compound 4-1-1*

LCMS (ESI) m/z = 1056.10 (M+H)+
Retention time: 3.11 minutes (analysis condition SQDFA05 long)

Diketopiperazine elimination compound, Fmoc-MeLeu-Aze(2)-MeCha-MeGly-Asp-pip (SEQ ID NO: 19) (run 1) (compound 4-2-1*)

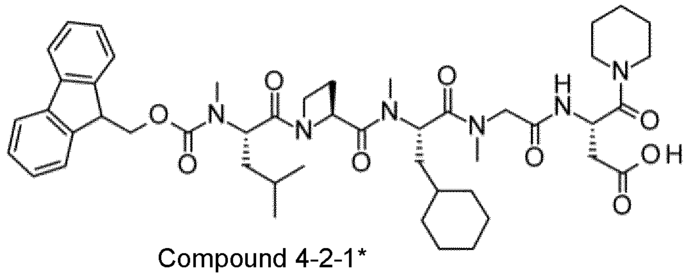

Compound 4-2-1*

LCMS (ESI) m/z = 871.94 (M+H)+
Retention time: 2.99 minutes (analysis condition SQDA05 long)

Target peptide compound (TM), Fmoc-Thr(tBu)-Thr(tBu)-MeAla-Cys(StBu)-Phe-Asp-pip (SEQ ID NO: 20) (run 2) (compound 4-1-2*)

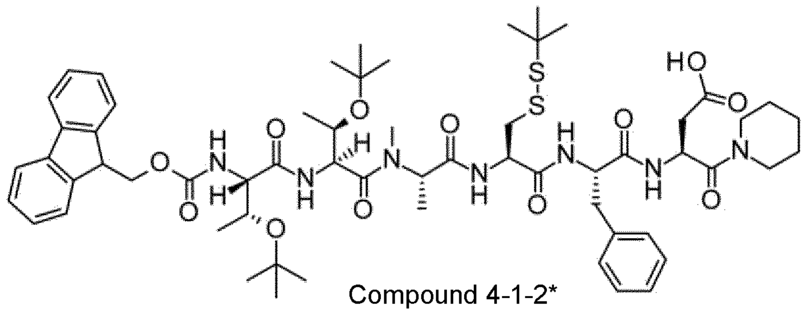

Compound 4-1-2*

LCMS (ESI) m/z = 1160.90 (M+H)+
Retention time: 3.81 minutes (analysis condition SQDA05 long)

Diketopiperazine elimination compound, Fmoc-Thr(tBu)-Cys(StBu)-Phe-Asp-pip (SEQ ID NO: 21) (run 2) (compound 4-2-2*)

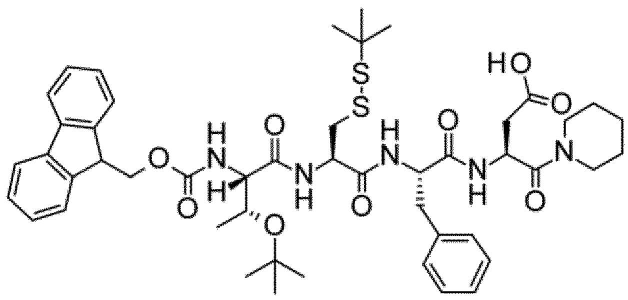

Compound 4-2-2*

LCMS (ESI) m/z = 918.77 (M+H)+
Retention time: 3.47 minutes (analysis condition SQDA05 long)

Target peptide compound (TM), Fmoc-Nle-MeAla(3-Pyr)-Ser(iPen)-Asp-pip (SEQ ID NO: 22) (run 3) (compound 4-1-3*)

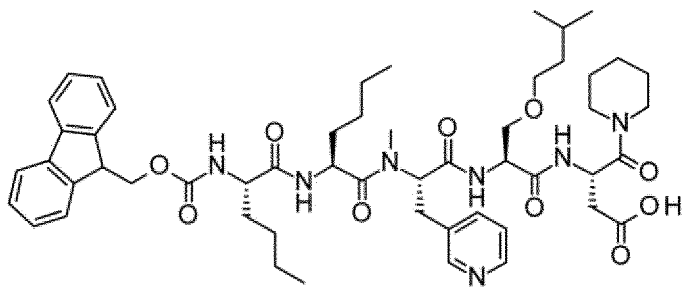

Compound 4-1-3*

LCMS (ESI) m/z = 968.94 (M+H)+
Retention time: 2.49 minutes (analysis condition SQDA05 long)

Diketopiperazine elimination compound, Fmoc-Nle-Ser(iPen)-Asp-pip (run 3) (compound 4-2-3*)

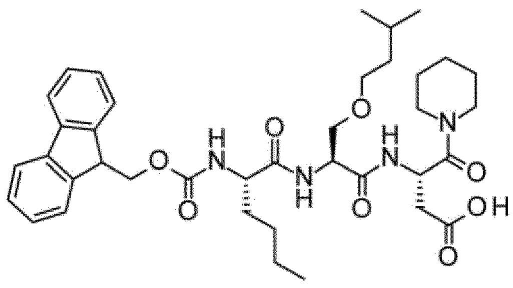

Compound 4-2-3*

LCMS (ESI) m/z = 693.72 (M+H)+
Retention time: 2.88 minutes (analysis condition SQDA05 long)

Target peptide compound (TM), Fmoc-Nle-MePhe(3-Cl)-Ser(iPen)-Asp-pip (SEQ ID NO: 23) (run 4) (compound 4-1-4*)

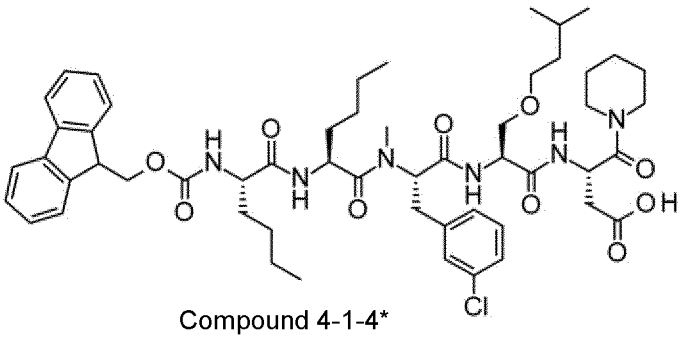

Compound 4-1-4*

LCMS (ESI) m/z = 1001.86 (M+H)+
Retention time: 3.55 minutes (analysis condition SQDA05 long)

Diketopiperazine elimination compound, Fmoc-Nle-Ser(iPen)-Asp-pip (run 4) (compound 4-2-4*)

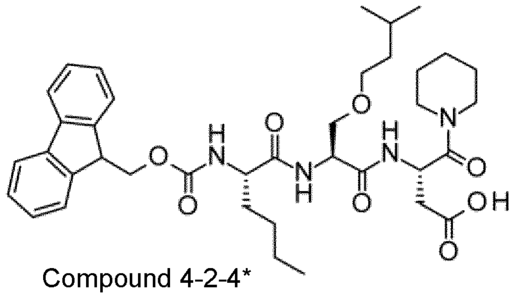

Compound 4-2-4*

LCMS (ESI) m/z = 693.72 (M+H)+
Retention time: 2.88 minutes (analysis condition SQDA05 long)

Target peptide compound (TM), Fmoc-Pro-Ser (nPr)-MeAla-Phe-Asp-pip (SEQ ID NO: 24) (run 5) (compound 4-1-5*)

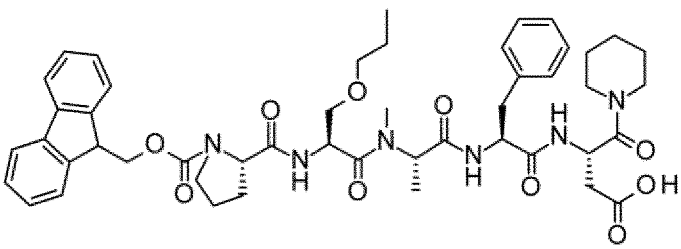

Compound 4-1-5*

LCMS (ESI) m/z = 881.78 (M+H)+
Retention time: 2.61 minutes (analysis condition SQDA05 long)

Diketopiperazine elimination compound, Fmoc-Pro-Phe-Asp-pip (run 5) (compound 4-2-5*)

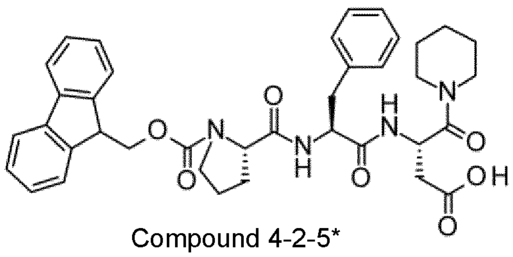

Compound 4-2-5*

LCMS (ESI) m/z = 667.64 (M+H)+
Retention time: 2.44 minutes (analysis condition SQDA05 long)

Target peptide compound (TM), Fmoc-MeIle-Aib-Pro-D-3-Abu-OH (SEQ ID NO: 25) (run 6) (compound 4-1-6*)

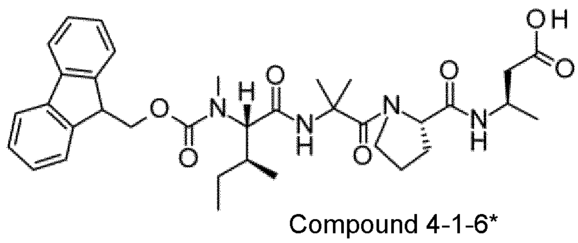

Compound 4-1-6*

LCMS (ESI) m/z = 635.71 (M+H)+
Retention time: 2.35 minutes (analysis condition SQDA05 long)

Diketopiperazine elimination compound, Fmoc-MeIle-D-3-Abu-OH (run 6) (compound 4-2-6*)

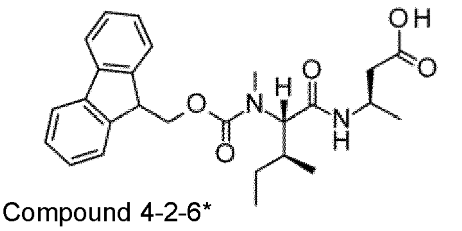

Compound 4-2-6*

LCMS (ESI) m/z = 453.55 (M+H)+
Retention time: 2.46 minutes (analysis condition SQDA05 long)

Target peptide compound (TM), Fmoc-Pro-cLeu-MeVal-D-3-Abu-OH (SEQ ID NO: 26) (run 7) (compound 4-1-7*)

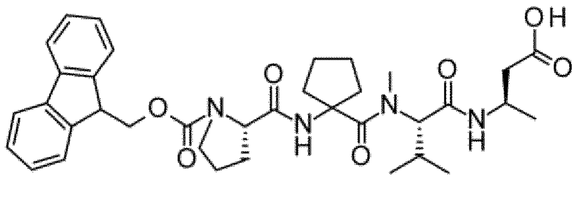

Compound 4-1-7*

LCMS (ESI) m/z = 645.79 (M-H)-
Retention time: 2.33 minutes (analysis condition SQDA05 long)

Diketopiperazine elimination compound, Fmoc-Pro-D-3-Abu-OH (run 7) (compound 4-2-7*)

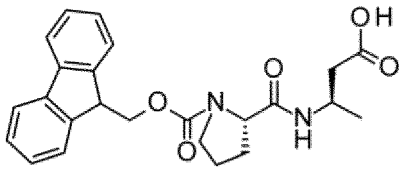

Compound 4-2-7*

LCMS (ESI) m/z = 423.53 (M+H)+
Retention time: 1.87 minutes (analysis condition SQDA05 long)

[Table 7]

| run | Solid-phase-supported peptide compound as raw material before elongation of amino acid in core 01 | TM | DKP elimination compound | core 01 | core 02 | core 03 | core 04 | core 05 | core 46 | core 07 | core cterm | SEQ ID NO: | Condition | Condition-1 Conventional method | Condition-2 Method of invention |
|---|---|---|---|---|---|---|---|---|---|---|---|---|---|---|---|
| 1 | Compound 1-2-6 | Compound 4-1-1* | Compound 4-2-1* | MeLeu | Ile | MeGly | Aze(2) | MeCha | MeGly | Asp | pip | 27 | Amount of formation of DKP elimination compound | 10.44% | 0.64% |
| 2 | Compound 1-2-7 | Compound 4-1-2* | Compound 4-2-2* | Thr(tBu) | Thr (tBu) | MeAla | Cys (StBu) | Phe | Asp | | pip | 28 | | 6.24% | 0.34% |
| 3 | Compound 1-2-8 | Compound 4-1-3* | Compound 4-2-3* | Nle | Nle | MeAla (3-Pyr) | Ser (iPen) | Asp | | | pip | 29 | | 0.69% | 0% |
| 4 | Compound 1-2-9 | Compound 4-1-4* | Compound 4-2-4* | Nle | Nle | MePhe (3-Cl) | Ser (iPen) | Asp | | | pip | 30 | | 1.14% | 0% |
| 5 | Compound 1-2-10 | Compound 4-1-5* | Compound 4-2-5* | Pro | Ser (nPr) | MeAla | Phe | Asp | | | pip | 31 | | 4.91% | 1.60% |
| 6 | Compound 1-2-11 | Compound 4-1-6* | Compound 4-2-6* | MeIle | Aib | Pro | D. 3.Abu | | | | none | 32 | | 34.08% | 3.94% |
| 7 | Compound 1-2-12 | Compound 4-1-7* | Compound 4-2-7* | Pro | cLeu | MeVal | β-3-Abu | | | | none | 33 | | 0.93% | 0% |

Thus, from the results of Example 4, it was confirmed that in synthesis of various peptides as well as specific peptide sequences, a diketopiperazine elimination compound suppressing effect was exhibited when elongation was performed by the method of the present invention in comparison with a common conventional method.

Example 5: Experiment for examining universality of reducing effect on diketopiperazine elimination during peptide synthesis under found conditions for substrate of carboxylic acid to be elongated

In this Example, various amino acids described in Core 1 were elongated by the method of the present invention (condition-3) for the peptide sequence prepared in Example 1-2-5 (compound 1-2-5), and the amounts of the diketopiperazine elimination compound and the 6-membered cyclic amidine skeleton compound formed were determined.

Condition-3 (method of the present invention)

100 mg of the peptide-supporting solid-phase resin prepared in Example 1-2-5 (compound 1-2-5 prepared in Example 1-2-5 from compound 1-2-1 at 0.552 mmol/g) was put in a solid-phase reaction vessel, DCM (1.0 mL) was added inside a glove box purged with nitrogen, and the mixture was left standing for 10 minutes to swell the resin. Thereafter, the solution was discharged from a frit, and subsequently, the resin was washed twice with toluene (0.7 mL per column). A solution of 1,8-diazabicyclo[5.4.0]-7-undecene (DBU) in toluene (2 v/v%, 0.7 mL per column) was added, and the mixture was reacted at room temperature for 10 minutes to perform deprotection of an Fmoc group. The solution was discharged from the frit, and the resin was then washed six times with toluene (0.7 mL per column).

To the resin obtained as described above, a solution obtained by mixing a solution of a carboxylic acid to be elongated (0.221 mmol, 4 equivalents), which is described in Table 8, in DMF (0.25 mL), a solution of [ethylcyano(hydroxyimino)acetato-O2)tri 1-pyrrolidinylphosphonium hexafluorophosphate (PyOxim) (0.221 mmol, 4 equivalents) in DMF (0.25 mL), and DIPEA (0.057 mL, 0.331 mmol, 6 equivalents) and leaving the mixture to stand for about 1 to 2 minutes was added, and the mixture was shaken at room temperature for 2 hours. The solution was discharged from the frit, and the resin was then washed four times with DMF (0.7 mL per column) and four times with DCM (0.7 mL per column), and dried.

For checking the progress of reaction, a part of each of the obtained resins was taken out, and swollen with DCM, and a peptide was then isolated with a TFE/DCM solution (1/1 (v/v)) containing DIPEA (0.045 mol/L). The isolating solution was analyzed by LCMS (SQDFA05 long or SQDAA05 long) to confirm formation of a target peptide (TM) (compounds 5-1-1* to 5-1-6*), a diketopiperazine elimination compound (compounds 5-2-1* to 5-2-6*) and a 6-membered cyclic amidine skeleton compound (compound 2-3*).

Table 8 shows the amount of each of the diketopiperazine elimination compound (TM-DKP) and the 6-membered cyclic amidine skeleton compound formed for each sequence. The amount of each of the diketopiperazine (DKP) elimination compound and the 6-membered cyclic amidine skeleton compound formed shown in Table 8 is a UV area percent of each of the diketopiperazine elimination compound (TM-DKP) and the 6-membered cyclic amidine skeleton compound when the total UV area percent of the target peptide (TM), the diketopiperazine elimination compound (TM-DKP) and the 6-membered cyclic amidine skeleton compound in LCMS is 100 percent.

Target peptide compound (TM), Teoc-Phe(4-CF3)-Ile-MeAla-Aze(2)-MeCha-MeGly-Asp-pyrro (SEQ ID NO: 34) (run 1) (compound 5-1-1*)

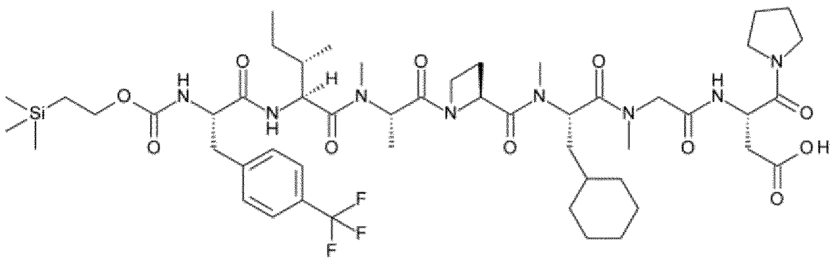

Compound 5-1-1*

LCMS (ESI) m/z = 1065.7 (M+H)+
Retention time: 2.90 minutes (analysis condition SQDA05 long)

Diketopiperazine elimination compound, Teoc-Phe(4-C-F3)-Aze(2)-MeCha-MeGly-Asp-pyrro (SEQ ID NO: 35) (run 1) (compound 5-2-1 *)

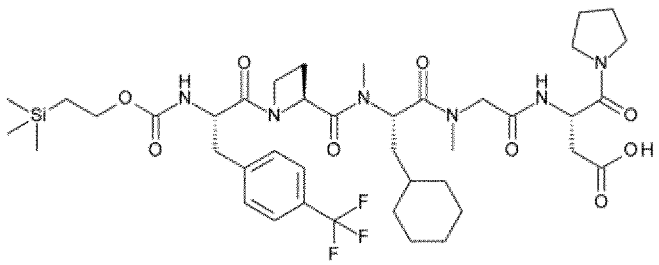

Compound 5-2-1*

LCMS (ESI) m/z = 867.5 (M+H)+
Retention time: 2.85 minutes (analysis condition SQDA05 long)

Target peptide compound (TM), Boc-MeAla-Ile-MeAla-Aze(2)-MeCha-MeGly-Asp-pyrro (SEQ ID NO: 36) (run 2) (compound 5-1-2*)

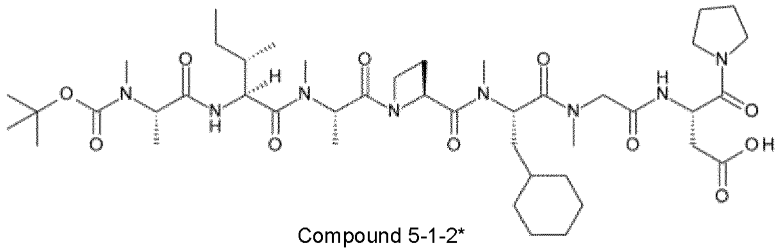

Compound 5-1-2*

LCMS (ESI) m/z = 891.7 (M+H)+
Retention time: 3.07 minutes (analysis condition SQDAA05 long)

Diketopiperazine elimination compound, Boc-MeAla-Aze(2)-MeCha-MeGly-Asp-pyrro (SEQ ID NO: 37) (run 2) (compound 5-2-2*)

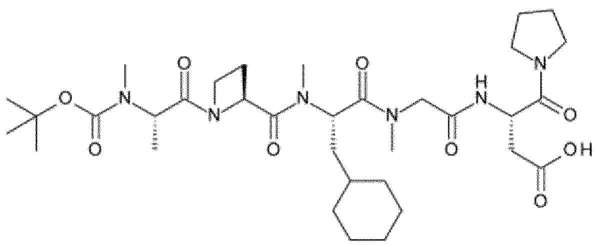

Compound 5-2-2*

LCMS (ESI) m/z = 691.6 (M-H)-
Retention time: 2.77 minutes (analysis condition SQDAA05 long)

Target peptide compound (TM), Alloc-Phe-Ile-MeAla-Aze(2)-MeCha-MeGly-Asp-pyrro (SEQ ID NO: 38) (run 3) (compound 5-1-3*)

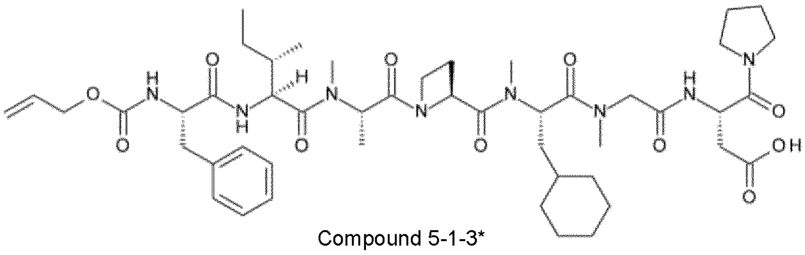

Compound 5-1-3*

LCMS (ESI) m/z = 937.6 (M+H)+
Retention time: 3.02 minutes (analysis condition SQDAA05 long)

Diketopiperazine elimination compound, Alloc-Phe-Aze(2)-MeCha-MeGly-Asp-pyrro (SEQ ID NO: 39) (run 3) (compound 5-2-3*)

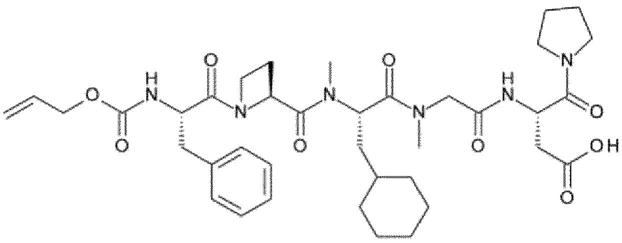

Compound 5-2-3*

LCMS (ESI) m/z = 739.5 (M+H)+
Retention time: 2.90 minutes (analysis condition SQDAA05 long)

Target peptide compound (TM), Ac-Ile-MeAla-Aze(2)-MeCha-MeGly-Asp-pyrro (SEQ ID NO: 40) (run 4) (compound 5-1-4*)

The expression of Ac means an acetyl group obtained by elongating acetic acid.

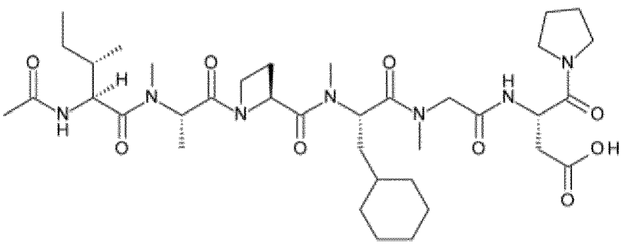

Compound 5-1-4*

LCMS (ESI) m/z = 748.6 (M+H)+
Retention time: 1.53 minutes (analysis condition SQDA05 long)

Diketopiperazine elimination compound, Ac-Aze(2)-MeCha-MeGly-Asp-pyrro (SEQ ID NO: 41) (run 4) (compound 5-2-4*)

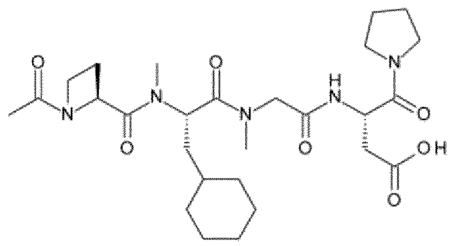

Compound 5-2-4*

LCMS (ESI) m/z = 548.5 (M-H)-
Retention time: 1.22 minutes (analysis condition SQDA05 long)

Target peptide compound (TM), Tfa-Aib-Ile-MeAla-Aze(2)-MeCha-MeGly-Asp-pyrro (SEQ ID NO: 42) (run 5) (compound 5-1-5*)

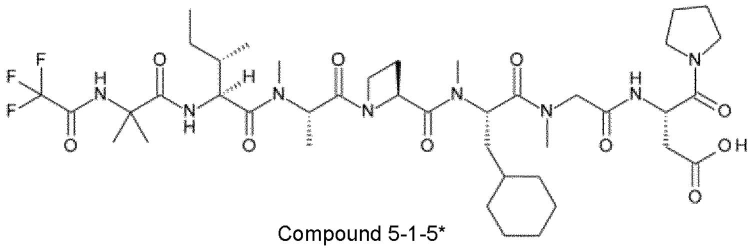

Compound 5-1-5*

LCMS (ESI) m/z = 887.6 (M+H)+
Retention time: 2.75 minutes (analysis condition SQDAA05 long)

Diketopiperazine elimination compound, Tfa-Aib-Aze(2)-MeCha-MeGly-Asp-pyrro (SEQ ID NO: 43) (run 5) (compound 5-2-5*)

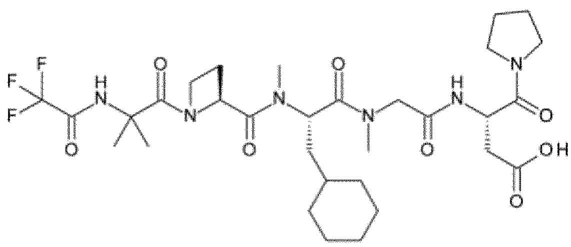

Compound 5-2-5*

LCMS (ESI) m/z = 687.6 (M-H)-
Retention time: 2.42 minutes (analysis condition SQDAA05 long)

Target peptide compound (TM), Ns-Aib-Ile-MeAla-Aze(2)-MeCha-MeGly-Asp-pyrro (SEQ ID NO: 44) (run 6) (compound 5-1-6*)

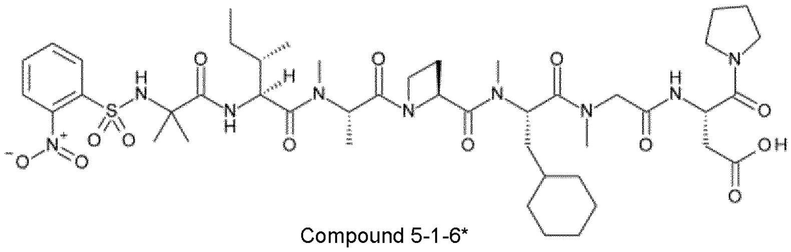

Compound 5-1-6*

LCMS (ESI) m/z = 976.6 (M+H)+
Retention time: 2.08 minutes (analysis condition SQDFA05 long)

Diketopiperazine elimination compound, Ns-Aib-Aze(2)-MeCha-MeGly-Asp-pyrro (SEQ ID NO: 45) (run 6) (compound 5-2-6*)

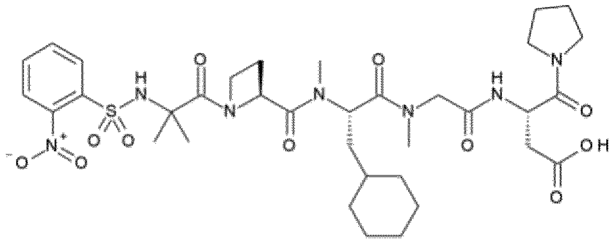

Compound 5-2-6*

LCMS (ESI) m/z = 776.5 (M-H)-
Retention time: 1.85 minutes (analysis condition SQDFA05 long)

[Table 8]

| | | | | Area % | | |
|---|---|---|---|---|---|---|
| **run** | Carboxylic acid to be elongated | **TM** | **TM-DKP** | **TM-DKP** | **Amidine form (Compound 2-3)** | **TM** |
| 1 | Teoc-Phe(4-CF3)-OH | **Compound 5-1-1*** | **Compound 5-2-1*** | 0.48 | 1.18 | 98.34 |
| 2 | Boc-MeAla-OH | **Compound 5-1-2*** | **Compound 5-2-2*** | 0.65 | 0.72 | 98.63 |
| 3 | Alloc-Phe-OH | **Compound 5-1-3*** | **Compound 5-2-3*** | 0.60 | 0.68 | 98.72 |
| 4 | AcOH | **Compound 5-1-4*** | **Compound 5-2-4*** | 2.58 | 1.39 | 96.03 |
| 5 | Tfa-Aib-OH | **Compound 5-1-5*** | **Compound 5-2-5*** | 4.54 | 1.22 | 94.24 |
| 6 | Ns-Aib-OH | **Compound 5-1-6*** | **Compound 5-2-6*** | 0.80 | 0.83 | 98.37 |

Thus, from the results of Example 5, it was confirmed that in elongation of various carboxylic acids as well as specific Fmoc amino acids, it was possible to suppress formation of a diketopiperazine elimination compound and a 6-membered cyclic amidine skeleton compound to synthesize a target peptide when elongation is performed by the method of the present invention.

Example 6: Examination of effect on diketopiperazine formation of dipeptide supported on solid phase through ester bond under found conditions

In general, diketopiperazine elimination becomes a problem when diketopiperazine is formed by cleavage of the ester bond of a dipeptide supported on a solid phase.

In this Example, the dipeptide supported on a solid phase through an ester bond, which had been prepared in Example 1-2-4, was subjected to elongation of Fmoc-MeLeu-OH was elongated by a common conventional method (condition-4) and the method of the present invention (condition-5), and an experiment was conducted for examining an effect by using the method of the present invention on diketopiperazine formation.

Condition-4 (conventional method) (run 1)

100 mg of the dipeptide-supporting solid-phase resin prepared in Example 1-2-5 (compound 1-2-13) was put in a solid-phase reaction vessel, DCM (1.0 mL) was added, and the mixture was left standing for 10 minutes to swell the resin. Thereafter, the solution was discharged from a frit, and subsequently, the resin was washed twice with DMF (0.7 mL per column). A solution of piperidine in DMF (20 v/v%, 0.7 mL per column) was added, and the mixture was reacted at room temperature for 10 minutes to perform deprotection of the Fmoc group. The solution was discharged from the frit, and the resin was then washed six times with DMF (0.7 mL per column).

To the resin obtained as described above, a solution obtained by mixing a solution of Fmoc-MeLeu-OH (0.6 mol/L) and Oxyma (0.375 mol/L) in DMF (0.3 mL) and a solution of DIC (10 v/v%) in DMF (0.36 mL) was added, and the mixture was shaken at 40°C for 2.5 hours. The solution was discharged from the frit, and the resin was then washed four times with DMF (0.7 mL per column) and four times with DCM (0.7 mL per column), and dried.

Condition-5 (method of the present invention) (run 2 and run 3)

100 mg of the peptide-supporting solid-phase resin prepared in Example 1-2-5 (compound 1-2-13) was put in a solid-phase reaction vessel, DCM (1.0 mL) was added inside a glove box purged with nitrogen, and the mixture was left standing for 10 minutes to swell the resin. Thereafter, the solution was discharged from a frit, and subsequently, the resin was washed twice with toluene (0.7 mL per column). A solution of 1,8-diazabicyclo[5.4.0]-7-undecene (DBU) in toluene (2 v/v%, 0.7 mL per column) was added, and the mixture was reacted at room temperature for 10 minutes to perform deprotection of an Fmoc group. The solution was discharged from the frit, and the resin was then washed six times with toluene (0.7 mL per column).

To the resin obtained as described above, a solution obtained by mixing a solution of Fmoc-MeLeu-OH (0.163 mmol, 4 equivalents) in DMF or DCM (0.25 mL), a solution of [ethylcyano(hydroxyimino)acetato-O2)tri 1-pyrrolidinylphosphonium hexafluorophosphate (PyOxim) (0.163 mmol, 4 equivalents) in DMF or DCM (0.25 mL), and DIPEA (0.040 mL, 0.245 mmol, 6 equivalents) and leaving the mixture to stand for about 1 to 2 minutes was added, and the mixture was shaken at room temperature for 2 hours. The solution was discharged from the frit, and the resin was then washed four times with DMF (0.7 mL per column) and four times with DCM (0.7 mL per column), and dried.

For checking the progress of reaction, a part of each of the obtained resins was taken out, and swollen with DCM, and a peptide was then isolated with a TFE/DCM solution (1/1 (v/v)) containing DIPEA (0.045 mol/L). The isolating solution was analyzed by LCMS (SQDFA05 long) to confirm formation of a target peptide (compound 6-1*).

Target peptide compound (TM), Fmoc-MeLeu-Ala-MeAla-OH (compound 6-1*)

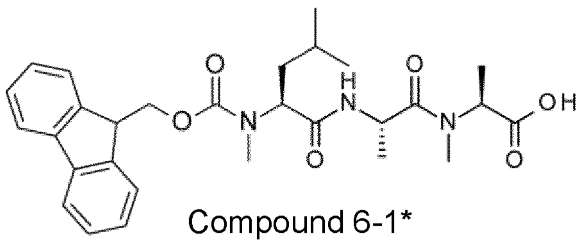

Compound 6-1*

LCMS (ESI) m/z = 524.5 (M+H)+

Retention time: 2.48 minutes (analysis condition SQDFA05 long)

Fmoc quantitation method

For determination of the loading rate, the obtained resin (run 1, 10.64 mg) was put in the reaction vessel, DMF (4.0 mL) was added, and the mixture was shaken at room temperature for 30 minutes. Thereafter, DBU (40 μL) was added, and the mixture was shaken at 30°C for 15 minutes. Thereafter, DMF was added so that the amount of the reaction mixed solution was 10.0 mL, and 80 μL of the resulting solution was diluted with DMF (920 μL). The obtained diluted solution was analyzed by LCMS (injection volume: 5 μL), and the loading rate of the obtained resin was calculated to be 0.021 mmol/g from the UVarea value of dibenzofulvene (294 nm: 88.61, 304 nm: 72.01). (A calibration curve prepared on the basis of UVarea values of dibenzofulvene at wavelengths of 294 nm and 304 nm every measurement day using a mixed solution of Fmoc-Gly-OH having a known concentration (purchased from a commercial supplier) and DBU as a standard substance were used to calculate amounts of support at respective wavelengths, and the average value of the amounts of support was taken as a loading rate of resin.)

In run 2, the loading rate was calculated by the same Fmoc quantitation method to be 0.321 mmol/g. (UVarea value at 294 nm: 1432.15 and UVarea value at 304 nm: 1217.70)

In run 3, the loading rate was calculated by the same Fmoc quantitation method to be 0.306 mmol/g. (UVarea value at 294 nm: 1364.67 and UVarea value at 304 nm: 1162.58)

Recovery ratio calculation method

In Example 6, the recovery ratio was defined as follows, and the ratio of suppression of diketopiperazine elimination from the resin during solid-phase reaction was evaluated.

Recovery ratio = loading rate of reaction product-supporting resin (mmol/g) ÷ loading rate of resin (mmol/g) at a target product yield of 100% (formula 1)

The loading rate of resin (mmol/g) at a target product yield of 100% is calculated as below.

Amount of support on resin (mmol/g) at a target product yield of 100% = loading rate of raw material resin (mmol/g) × weight of raw material resin (g) ÷ weight of resin (g) at a target product yield of 100% (formula 2)

The weight of resin (g) at a target product yield of 100% is calculated as below.

Weight of resin (g) at a target product yield of 100% = weight of raw material resin (g) - weight of peptide component (g) on raw material resin + weight of peptide component (g) on resin at a target product yield of 100% (formula 3)

The weight of the peptide component (g) on raw material resin is calculated as below.

Weight of peptide component (g) on raw material resin = weight of raw material resin (g) × loading rate of raw material resin (mmol/g) × molecular weight of peptide component (g/mol) on raw material resin × 0.001 (mol/mmol) (formula 4)

The weight of the peptide component (g) on resin at a target product yield of 100% is calculated as below.

Weight of peptide component (g) on resin at a target product yield of 100% = weight of raw material resin (g) × loading rate of raw material resin (mmol/g) × molecular weight of peptide component of target product (g/mol) × 0.001 (mol/mmol) (formula 5)

Substitution of formulae 3, 4 and 5 into formula 2 gives the following formula.

Amount of support on resin (mmol/g) at a target product yield of 100% = loading rate of raw material resin (mmol/g) ÷ (1 - loading rate of raw material resin (mmol/g) × molecular weight of peptide component (g/mmol) on raw material resin × 0.001 (mol/mmol) + loading rate of raw material resin (mmol/g) × molecular weight of peptide component of target product (g/mol) × 0.001 (mol/mmol)) = 1 ÷ (1 ÷ loading rate of raw material resin (mmol/g) - molecular wight of peptide component (g/mol) on raw material resin × 0.001 (mol/mmol) + molecular weight of peptide component of target product (g/mol) × 0.001 (mol/mmol)) (formula 6)

Substitution of formula 6 into formula 1 gives the following formula, from which the recovery ratio is calculated.

Recovery ratio = loading rate of on reaction product-supporting resin (mmol/g) × (1 ÷ amount of support on raw material resin (mmol/g) - molecular weight of peptide component (g/mol) on raw material resin × 0.001 (mol/mmol) + molecular weight of peptide component of target product (g/mol) × 0.001 (mol/mmol)

Elongation was performed by the conventional method (condition-4) and the method of the present invention (condition-5). For resins obtained by the respective methods, the loading rate calculated by the Fmoc quantitation method and recovery ratios calculated by the recovery ratio calculation method are shown in Table 9.

[Table 9]

| | | Reaction condition | | | | | | |
|---|---|---|---|---|---|---|---|---|
| | | Fmoc deprotection | | Washing | Elongation | | | |
| run | Condition | Base | Solvent | Solvent | **Condensation agent** | **Solvent** | **Amount of support** (mmol/g) | **Recovery ratio** (%) |
| 1 | **Condition-4 (conventional method)** | 20% pip | DMF | DMF × 6 | DIC/Oxyma | DMF | 0.021 | 5.4 |
| 2 | **Condition-5 (method of invention)** | 2% DBU | toluene | toluene × 6 | PyOxim/ DIPEA | DMF | 0.321 | 83 |
| 3 | **Condition-5 (method of invention)** | 2% DBU | toluene | toluene × 6 | PyOxim/ DIPEA | DCM | 0.306 | 79 |

Thus, from the results of Example 6, it was confirmed that a dipeptide supported on a solid phase through an ester bond was subjected to elongation by the method of the present invention to suppress diketopiperazine formation, so that it was possible to efficiently synthesize a target peptide.

<u>Example 7: Examination of presence of carbamic acid salt in Fmoc deprotection step and examination of effect on diketopiperazine elimination</u>

The table below shows the compounds described in Examples.

[Table 10]

| Compound No. | Abbreviation | Structural formula | Name |
|---|---|---|---|
| 1 - 3 - 1 | Fmoc - Phe - NMe2 | 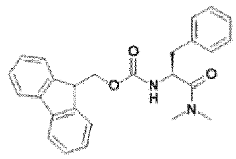 | 9H-fluoren-9-ylmethyl N-[(1S)-1-benzyl-2-(dimethylamino)-2-oxoethyl]carbamate |
| 1 - 3 - 2 | H-Phe-OH | 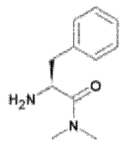 | (2S)-2-amino-N,N-dimethyl-3-phenyl-propanamide |
| 1 - 3 -3a | DBU · HOCO-Phe-NMe2 | 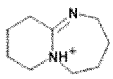 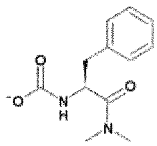 | N-[(1S)-1-benzyl-2-(dimethylamino)-2-oxoethyl]carbamate DBU salt |
| 1 - 3 - 3b | Piperidne · HOCO - Phe - NMe2 | 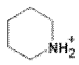 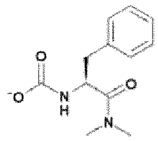 | N-[(1S)-1-benzyl-2-(dimethylamino)-2-oxoethyl]carbamate piperidinium salt |
| 1 - 3 - 3c | TMG · HOCO -Phe - NMe2 | 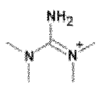 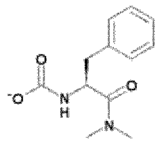 | N-[(1S)-1-benzyl-2-(dimethylamino)-2-oxoethyl]carbamate TMG salt |
| 1 - 3 - 3d | HP1(dma) · HOCO-Phe - NMe2 | 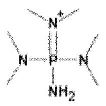 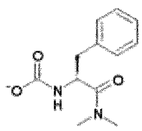 | N-[(1S)-1-benzyl-2-(dimethylamino)-2-oxoethyl]carbamate HP1 (dma) salt |

In Examples, the following abbreviations were used.

NMR: nuclear magnetic resonance

ROESY: Rotating frame Overhauser effect spectroscopy

NOESY: Nuclear Overhauser effect spectroscopy

HSQC: Heteronuclear single quantum correlation

HMBC: Heteronuclear multiple bond correlation

cpd.: Compound

The NMR measurement was performed at 298 K using AVANCE III 600 Cryo-TCI or AVANCE III HD 600 BBFO manufactured by Bruker.

When a base containing a plurality of nitrogen atoms is protonated into a cation to form a salt with an anion in the system, a cation in which any of the nitrogen atoms is protonated can give a cation source. For example, in the case of 1,8-diazabicyclo[5.4.0]-7-undecene (DBU), the three cations shown below may give a cation source, and in the present

specification, salts formed by any of the cations with a corresponding anion are not discriminated, and are each called a DBU salt (Figure 1). The same applies to other bases. For example, salts similarly formed by 1,1,3,3-tetramethylguanidine (TMG) or iminotris(dimethylamino)phosphoran (HP1 (dma)) are each called a TMG salt or HP1 (dma) salt.

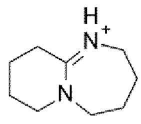 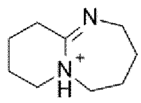 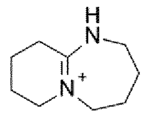

Synthesis of compound 1-3-1: 9H-fluoren-9-ylmethyl N-[(1S)-1-benzyl-2-(dimethylamino)-2-oxo-ethyllcarbamate (Fmoc-Phe-$NMe_2$)

To a mixed solution of commercially available N-(9-fluorenylmethoxycarbonyl)-L-phenylalanine (Fmoc-Phe-OH, CAS: 35661-40-6, compound aa2-12) (2.0 g, 5.16 mmol) in N,N-dimethylformamide (DMF) (14 mL) and dichloromethane (DCM) (4 mL), 1-ethyl-3-(3-dimethylaminopropyl)carbodiimide hydrochloride (EDCI·HCl) (1.19 g, 6.19 mmol) and 1-hydroxybenzotriazole (HOBt) (0.767 g, 5.68 mmol) were added, a solution of dimethylamine in tetrahydrofuran (THF) (2.0 M, 2.84 mL, 5.68 mmol) was then further added, and the mixture was stirred for 15 minutes. To the solution was added isopropyl acetate (20 mL), and the organic phase was washed twice with 1 M hydrochloric acid (20 mL), once with water (50 mL), twice with a half saturated aqueous sodium bicarbonate solution (20 mL), and twice with a half saturated aqueous sodium chloride solution (20 mL). The obtained organic phase was dried over sodium sulfate, and then filtered, and the solvent of the obtained mother liquid was distilled off under reduced pressure. The obtained crude product was azeotoropically distilled with toluene, and then allowed to cool at 5°C to obtain a white crystal of compound 1-3-1 (1.88 g, 88%).

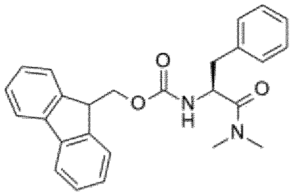

Compound 1-3-1

$^{1}$H-NMR (600 M Hz, toluene-d8, 298 K) $\delta$ 7.51 (d, 2H, J=7.2 Hz), 7.43 (d, 2H, J=7.3 Hz), 7.21-7.16 (m, 2H), 7.14-7.07 (m, 2H), 7.05-7.00 (m, 4H), 7.00-6.95 (m, 1H), 6.00 (d, 1H, J=8.4 Hz, NH), 4.88 (ddd, 1H, J=8.4, 8.4, 6.0 Hz), 4.32 (dd, 1H, J=10.5, 7.4 Hz), 4.23 (dd, 1H, J=10.5, 7.4 Hz), 4.03 (t, 1H, J=7.4 Hz), 2.93 (dd, 1H, J=13.0, 8.4 Hz), 2.86 (dd, 1H, J=13.0, 6.0 Hz), 2.49 (s, 3H), 2.02 (s, 3H).

$^{13}$C-NMR (151 MHz, toluene-d8, 298 K) $\delta$ 171.1, 155.9, 144.6, 141.8, 129.9, 128.5, 127.8 (overlapped with solvent peak, detected by CH-HSQC), 127.3, 127.0, 125.6, 125.4, 120.2, 67.2, 52.3, 47.7, 40.2, 36.0, 35.0.

LCMS (ESI) m/z = 415.44 (M+H)+
Retention time: 0.89 minutes (analysis condition SQDFA05)

Example 7-1: Examination of presence of DBU·HOCO-Phe-$NMe_2$ when compound 1-3-1 is treated with DBU

Example 7-1-1: Product obtained by treating compound 1-3-1 with DBU in solution of N,N-dimethylformamide-d7 (DMF-d7)

$^{1}$H-NMR of a solution of compound 1-3-1 (12.9 mg, 0.031 mmol) in N,N-dimethylformamide-d7 (DMF-d7) (0.5 mL) was measured (Figure 1-b)). Thereafter, to the relevant solution was added 1,8-diazabicyclo[5.4.0]-7-undecene (DBU) (5.11 $\mu$L, 0.034 mmol), and the mixture was vigorously stirred, followed by measurement of $^{1}$H-NMR (Figure 1-a) and 1D-ROESY ((Figure 2-a)-2) and (Figure 2-a)-3)). As a result, it was confirmed from the NMR spectrum that mainly three compounds were formed. That is, it was confirmed that three compounds: dibenzofulvene, compound 1-3-2 (H-Phe-$NMe_2$) and N-[(1S)-1-benzyl-2-(dimethylamino)-2-oxo-ethyl]carbamate DBU salt (compound 1-3-3a and DBU·HOCO-Phe-$NMe_2$) were present in the relevant solution, and compound 1-3-2 and compound 1-3-3a underwent chemical exchange. Identification of compound 1-3-2 (H-Phe-$NMe_2$) and identification of compound 1-3-3a in the relevant solution are described in detail in this Example (Example 7-1-1) and Example 7-1-3, respectively.

Confirmation of chemical exchange of compound 1-3-2 (H-Phe-$NMe_2$) and compound 1-3-3a formed by applying DBU to compound 1-3-1 in DMF-d7, and ratio thereof

1D-ROESY of the relevant solution was measured ($^1$H signals at 4.84 ppm and 3.95 ppm were each selectively excited, Figure 2-a)-2 and Figure 2-a)-3), and the result showed that the $^1$H signal of compound 1-3-3a at 4.84 ppm and the $^1$H signal at 3.95 ppm corresponding to a proton of $\alpha$-methine of compound 1-3-2 underwent chemical exchange in the system ((Figure 2-a)-2) and (Figure 2-a)-3)). That is, from the $^1$H signal at 3.95 ppm when the $^1$H signal at 4.84 ppm was selectively excited (Figure 2-a)-2) and from the $^1$H signal at 4.85 ppm when the $^1$H signal at 3.95 ppm was selectively excited (Figure 2-a)-3), ROESY signal in the same phase as that of the selectively excited signal was observed, and therefore these signals were confirmed to undergo chemical exchange (Figure 2-a)). Further, from the integral ratio of $^1$H signals in $^1$H-NMR, the abundance ratio of compound 1-3-3a and compound 1-3-2 was confirmed to be 82:18 (Figure 2-b)).

Examination of presence of compound 1-3-2 (H-Phe-$NMe_2$) in relevant solution

Three (3) $\mu$L of a separately provided standard product of compound 1-3-2 was mixed with the relevant solution, followed by measurement of $^1$H-NMR (Figure 3-a)-3 and Figure 3-b)) and 1D-NOSEY (Figure 3-a)-4 and Figure 3-a)-5).

1) $^1$H-NMR of a solution obtained by adding 3 $\mu$L of the standard product of compound 1-3-2 to the relevant solution was measured, and the result showed that the $^1$H signal at 3.95 ppm observed in $^1$H-NMR of the relevant solution (Figure 3-a)-2) and the $^1$H signal at 3.94 ppm observed in the standard product of compound 1-3-2 (Figure 3-a)-1) were observed as a triplet $^1$H signal at 3.95 ppm, with both the signals completely coinciding with each other without separation (Figure 3-a)-3).
2) 1D-NOESY of the solution obtained by adding 3 $\mu$L of the standard product of compound 1-3-2 to the relevant solution was measured ($^1$H signals at 4.84 ppm and 3.95 ppm were each selectively excited), and the result showed that from the $^1$H signal at 3.95 ppm when the $^1$H signal at 4.84 ppm was selectively excited (Figure 3-a)-4) and from the $^1$H signal at 4.85 ppm when the $^1$H signal at 3.95 ppm was selectively excited (Figure 3-a)-5), NOESY signal in the same phase as that of the selectively excited signal and with the same shape as that of the original signal (4.85 ppm: double triplet and 3.95 ppm: triplet) was observed.
3) Addition of 3 $\mu$L of the standard product of compound 1-3-2 to the relevant solution changed the integral ratio of the $^1$H signal of the compound 1-3-3a at 4.84 ppm and the $^1$H signal of compound 1-3-2 at 3.95 ppm from 82 : 18 (integral ratio before addition of compound 1-3-2 [Figure 2-b]) to 61 : 39 (Figure 3-b)).

From the above three points of 1) to 3), it was confirmed that compound 1-3-2 was present in the relevant solution.

Example 7-1-2: Identification of compound 1-3-3a formed by applying DBU to compound 1-3-2 by spraying $^{13}CO_2$

The following experimental operations in Example 7-1-2 were all carried out in a glove box (under dehydrated conditions in a nitrogen atmosphere). N,N-dimethylformamide-d7 (DMF-d7) dried for 12 hours or more by using MS4A heated to a high temperature with a microwave and then allowed to cool in vacuum ($\times$2) to be activated was used as a solvent, and a sample collected into a low pressure/vacuum tube for NMR with a valve in the glove box and completely prevented from contacting the outside air was used to measure NMR.

Preparation of solution A (Sol A)

A solution of compound 1-3-2 (4.64 mg, 0.024 mmol) in N,N-dimethylformamide-d7 (DMF-d7) (0.5 mL) was bubbled with $^{13}CO_2$ for 1 minute, followed by measurement of $^1$H-NMR (Figure 4-b)), $^{13}$C-NMR (Figure 5-a)) and 1D-NOESY ($^1$H signals at 4.77 ppm and 3.96 ppm were each selectively excited, (Figure 4-c) and (Figure 4-d)). Thereafter, 1,8-diazabicyclo[5.4.0]-7-undecene (DBU) (3.97 $\mu$L, 0.027 mmol) was added to prepare a solution A containing compound 1-3-3a (DBU·HOCO-Phe-$NMe_2$), and $^1$H-NMR (Figure 4-e)) and $^{13}$C-NMR (Figure 5-b)) were measured.

Identification of compound 1-3-3a in solution A

The structure of compound 1-3-3a can be explained without contradiction from the results of measurements of $^1$H-NMR, $^{13}$C-NMR and 1D-NOESY.

4) $^1$H-NMR of a solution obtained by bubbling the solution of compound 1-3-2 in N,N-dimethylformamide-d7 (DMF-d7) (0.5 mL) with $^{13}CO_2$ for 1 minute was measured, and the result showed the presence of a new $^1$H signal at 4.76 ppm which had not been observed in $^1$H-NMR of compound 1-3-2 as a raw material (Figure 4-a)) (Figure 4-b))

(formation of carbamate anion or carbamic acid, see Figure 5-a)).

5) The integral ratio of the $^1H$ signal at 4.76 ppm observed in $^1H$-NMR of a solution obtained by bubbling the solution of compound 1-3-2 in N,N-dimethylformamide-d7 (DMF-d7) (0.5 mL) with $^{13}CO_2$ for 1 minute and the $^1H$ signal at 3.96 ppm corresponding to α-methylene of compound 1-3-2 was measured, and the result showed that the integral ratio was 52 : 48 (Figure 4-f).

6) 1D-NOESY of a solution obtained by the solution of compound 1-3-2 in N,N-dimethylformamide-d7 (DMF-d7) (0.5 mL) with $^{13}CO_2$ for 1 minute was measured, and the result showed that from the $^1H$ signal at 3.96 ppm when the $^1H$ signal at 4.76 ppm was selectively excited (Figure 4-c)) and from the $^1H$ signal at 4.76 ppm when the $^1H$ signal at 3.96 ppm was selectively excited, NOESY signal in the same phase was observed (Figure 4-d)), so that both the signals were confirmed to undergo chemical exchange.

7) $^1H$-NMR of a solution obtained by bubbling the solution of compound 1-3-2 in N,N-dimethylformamide-d7 (DMF-d7) (0.5 mL) with $^{13}CO_2$ for 1 minute and then adding DBU was measured, and the result showed that the $^1H$ signal at 4.76 ppm observed before addition of DBU was shifted under high magnetic field to 4.83 ppm when DBU was added (Figure 4-e)) (confirmation of formation of compound 1-3-3a).

8) $^1H$-NMR of a solution obtained by bubbling the solution of compound 1-3-2 in N,N-dimethylformamide-d7 (DMF-d7) (0.5 mL) with $^{13}CO_2$ for 1 minute and then adding DBU was measured, and the result showed that there was $^3J_{CH}$ coupling at 2.7 Hz between a $^1H$ signal of α-methine at 4.83 ppm and a $^{13}C$ signal of $^{13}C$-labeled carbonyl at 161.5 ppm (Figure 5-b)) (Figure 4-g)).

9) $^{13}C$-NMR of a solution obtained by bubbling the solution of compound 1-3-2 in N,N-dimethylformamide-d7 (DMF-d7) (0.5 mL) with $^{13}CO_2$ for 1 minute was measured (Figure 5-a)), $^{13}C$-NMR of a solution obtained by adding DBU to the foregoing solution was measured (Figure 5-b)), the result of the former measurement was compared with the result of the latter measurement, and the result showed that the $^{13}C$ signal corresponding to $^{13}C$-labeled carbonyl was shifted under high magnetic field from 157.1 ppm to 161.5 ppm by adding DBU (confirmation of formation of compound 1-3-3a).

From the six points of 4) to 9), it was confirmed that in preparation of the solution A (Sol A), the primary amine of compound 1-3-2 reacted with $^{13}CO_2$ to form carbamate or carbamic acid, and to this, 1,8-diazabicyclo[5.4.0]-7-undecene (DBU) was applied to form compound 1-3-3a (DBU·HOCO-Phe-$NMe_2$). From the above-described experiment, it is considered without contradiction that compound 1-3-3a (DBU·HOCO-Phe-$NMe_2$) was formed. For this structure, more detailed identification was performed in Example 7-1-3.

Example 7-1-3: Demonstration of structure of compound 1-3-3a present in solution A + solution B (Sol A + Sol B)

The following experimental operations described in Example 7-1-3 were all carried out in a glove box (under dehydrated conditions in a nitrogen atmosphere). N,N-dimethylformamide-d7 (DMF-d7) dried for 12 hours or more by using MS4A heated to a high temperature with a microwave and then allowed to cool in vacuum (×2) to be activated was used as a solvent, and a sample collected into a low pressure/vacuum tube for NMR with a valve in the glove box and completely prevented from contacting the outside air was used to measure NMR.

Preparation of solution B (Sol B)

To a solution of compound 1-3-1 (11.5 mg, 0.028 mmol) in N,N-dimethylformamide-d7 (DMF-d7) (0.5 mL), 1,8-diazabicyclo[5.4.0]-7-undecene (DBU) (4.56 μL, 0.031 mmol) was added to prepare a solution B, and $^1H$-NMR was measured (Sol B, Figure 6-a)).

Preparation of solution A + solution B (Sol A + Sol B)

The solution B (Sol B) (0.15 mL, Figure 6-a), 0.0084 mmol) was mixed with the solution A (Sol A) (0.35 mL, Sol A, Figure 6-c), 0.0168 mmol), and $^1H$-NMR (Figure 6-b)), $^{13}C$-NMR, CH-HMBC (Figure 7-c)), CH-HSQC, NH-HMBC (Figure 7-b)) and NH-HSQC (Figure 7-a)) were measured.

Examination of presence of compound 1-3-3a in solution B (Sol B)

It was confirmed that the $^1H$ signal at 4.83 ppm corresponding to α-methine of compound 1-3-3a, which had been observed in $^1H$-NMR of the solution A (Figure 6-c)) and the $^1H$ signal at 4.85 ppm observed in $^1H$-NMR of the solution B (Figure 6-a)) completely coincided with each other while each shifting to the $^1H$ signal at 4.84 ppm (Figure 6-b)). That is, it was confirmed that when the solution B was mixed with the solution A, a low magnetic field-side peak without coupling in the $^1H$ signal at 4.85 ppm observed in the solution B (a peak surrounded by a dotted line in Figure 6-a)) and a low magnetic field-side peak with recognizable coupling to $^{13}C$ (2.7 Hz) in the $^1H$ signal at 4.83 ppm observed

in the solution A (a peak surrounded by a circle in Figure 6-b)) were shifted together and converted into a middle peak having a broad shape (a peak surrounded by a circle in Figure 6-b)). These results showed the presence of compound 1-3-3a in (Sol B) with the $^1$H signal.

Demonstration of structure of compound 1-3-3a in solution A + solution B (Sol A + Sol B)

By using $^1$H-NMR (Figure 6-b)), $^{13}$C-NMR, CH-HMBC (Figure 7-c)), CH-HSQC, NH-HMBC (Figure 7-b)) and NH-HSQC (Figure 7-a)), it can be explained without contradiction that the structure of compound 1-3-3a in the solution A + the solution B (Sol A + Sol B) is a DBU salt of carbamic acid.

10) CH-HMBC of a mixed solution of the solution A (Sol A) + the solution B (Sol B) was measured, and the result showed that there was a CH-HMBC cross peak between the $^1$H signal of $\alpha$-methine at 4.84 ppm and $^{13}$C-labeled $^{13}$C signal at 161.5 ppm which is higher than the $^{13}$C signal of the solvent (Figure 7-c)). That is, because of the CH-HMBC correlation and the value of the $^{13}$C signal, and since $^{13}$C is derived from bubbling $^{13}CO_2$ experimentally, the $^{13}$C signal at 161.5 ppm is identified as carbonyl of carbamate without contradiction.

11) NH-HSQC of a mixed solution of the solution A (Sol A) + the solution B (Sol B) was measured, and the result showed that there was a NH-HSQC correlation between the $^1$H signal of NH and the identified $^{15}$N signals at 5.30 ppm and 91.4 ppm, so that the $^{15}$N signal at 91.4 ppm was identified as a nitrogen atom of carbamate (Figure 7-a)). It was also confirmed that there was a NH-HMBC correlation between the $^1$H signal of benzylmethylene at 2.91/2.83 ppm and the $^{15}$N signal at 91.4 ppm corresponding to the nitrogen atom of carbamate (Figure 7-b)), and from the value of the $^{15}$N signal, it can be explained without contradiction that the $^{15}$N signal at 91.4 ppm is the nitrogen atom of carbamate.

From the two points of 10) and 11), it was confirmed that compound 1-3-3a was present in the system when the solution A + the solution B (Sol A + Sol B) were prepared. As described below, the NMR signals of carbamate parts of compound 1-3-3a can be all attributed (Figure 8), and the values of all NMR signals can account for the structure of compound 1-3-3a without contradiction.

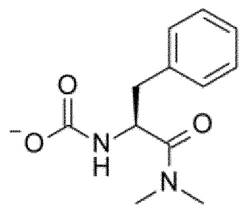

Compound 1-3-3

$^1$H-NMR (600 M Hz, DMF-d7, 298 K) $\delta$ 7.29-7.23 (m, 4H), 7.22-7.11 (m, 1H), 5.30 (brs, 1H, NH), 4.90 (ddd, 1H, J=6.8, 6.2 Hz, $^3J_{CH}$= 2.7 Hz), 2.91 (dd, 1H, J=13.3, 6.8 Hz), 2.87 (s, 3H), 2.83 (dd, 1H, J=13.3, 6.2 Hz), 2.78 (s, 3H).

$^{13}$C-NMR (151 MHz, DMF-d7, 298 K) $\delta$ 173.6 (qC), 161.5 (qC), 139.4 (qC), 129.7 (CH x2), 128.3 (CH x2), 126.3 (CH), 53.0 (CH), 40.0 (CH2), 36.4 (CH3), 34.8 (CH3).

$^{15}$N-NMR (61 M Hz, DMF-d7, 298 K, assigned by NH-HSQC and NH-HMBC) $\delta$ 96, 91.

The above results showed that when Fmoc-protected amino group was treated with DBU, the amino group was not fully exposed with even decarboxylation proceeding subsequently to elimination of dibenzofulvene, but most part thereof was present as DBU·HOCO-Phe-$NMe_2$. It was also shown that it was possible to examine the presence and the abundance ratio thereof by $^1$H-NMR.

Example 7-2: Examination of presence of carbamic acid salt when compound 1-3-1 is treated with DBU or piperidine in various solvents

Basic operation

A solution of compound 1-3-1 (about 25 mg) in a heavy solvent (1.25 mL, tetrahydrofuran-d8 (THF-d8) [Entry 1], toluene-d8 [Entry 2], dicyclomethane-d2 (DCM-d2) [Entry 3], or N,N-dimethylformamide-d7 (DMF-d7) [Entry 4]) was prepared, and 1.0 mL of the relevant solution was then divided into two parts (0.5 mL). 1,8-diazabicyclo[5.4.0]-7-undecene (DBU) (6.8 $\mu$L) was applied to one part, and piperidine (67.5 $\mu$L) was applied to the other part. NMR of each part was measured, and the ratio of compound 1-3-3a to compound 1-3-2 was determined from the integral ratio of protons in $^1$H-NMR (Table 11). The amount of compound 1-3-1 used in this experiment was 25.1 mg for entry 1, 24.8 mg for entry 2, 25.0 mg for entry 3, and 25.3 mg for entry 4.

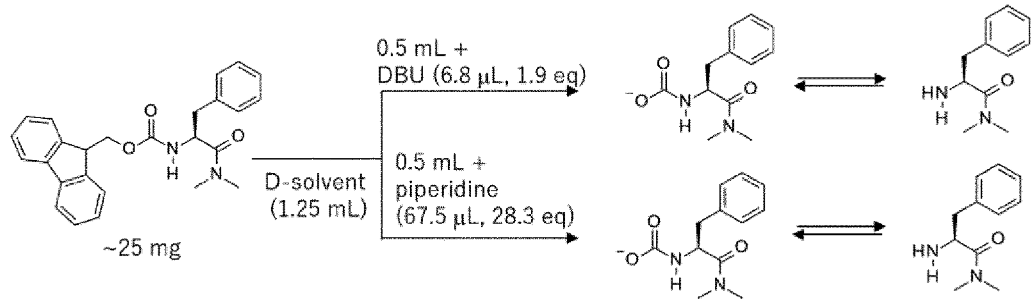


[Table 11]

| entry | Solvent | Base | |
|---|---|---|---|
| | | DBU (1.9 eq) | Piperidine (28.3 eq) |
| | | Compound 1-3-3a : Compound 1-3-2 | |
| 1 | THF-d8 | 53:47 | 0:100 |
| 2 | Toluene-d8 | 65:35 | 0:100 |
| 3 | DCM-d2 | 76:24 | 0:100 |
| 4 | DMF-d7 | 84:16 | 0:100 |

From the above results, it was confirmed that when the Fmoc-protected amino group was treated with DBU, the proportion of the carbamic acid salt present was 50% or more in any of the solvents. On the other hand, it was confirmed that when the treatment was performed with pyridine, the carbamic acid salt was not detected in any of the solvents, and even decarboxylation proceeded, so that the amino group was fully exposed.

For discussing the results of experiments on a solid face on the basis of this finding, without being bound by a specific theory, it can be explained that as in the following scheme, formation of a 6-membered ring which causes diketopiperazine elimination or the like is prevented due to the presence of the carbamic acid salt, resulting in the reducing effect.

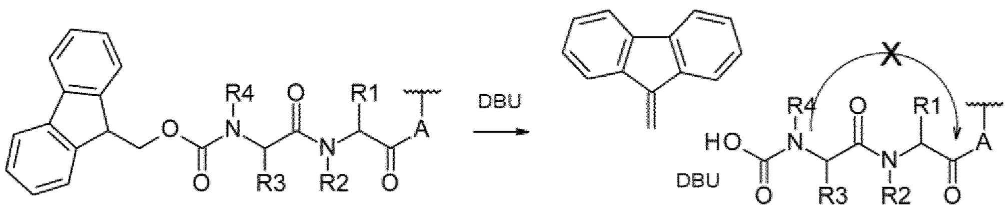


For example, deprotection with DBU in which formation of the carbamic acid salt had been confirmed and deprotection with piperidine in which formation of the carbamic acid salt had not been confirmed in NMR were compared in a solid-phase experiment, and an effect of the formation of the carbamic acid salt was examined. Using reagents and solvents shown in runs 1 and 2 in Table 12, a reaction is carried out by the same method as in the basic operation B described in Example 2. Table 12 below shows the obtained results (the relative ratios of UV areas in LCMS for compound 2-1*, compound 2-2*, compound 2-3* and compound 2-4* under respective conditions are expressed as a percentage).

[Table 12]

| | Reaction condition | | | | | Area % | | | |
|---|---|---|---|---|---|---|---|---|---|
| | Fmoc deprotection | | Washing | Elongation | | | | | |
| run | Base | Solvent | Solvent | Condensation agent | Solvent | TM-DKP (Compound 2-2') | Amidine form (Compound 2-3*) | TM (Compound 2-1*) | MeLeu Excessive elongation (Compound 2-4*) |
| 1 | 2% DBU | THF | THF × 6 | PyOxim/ DIPEA | DCM | 0.59 | 0.91 | 98.50 | 0 |
| 2 | 20% pip | THF | THF × 6 | PyOxim/ DIPEA | DCM | 9.27 | 8.06 | 82.67 | 0 |

Without being bound by a specific theory, from comparison between run 1 (2% DBU) and run 2 (20% piperidine) in Table 12, it can be explained without contradiction that the reason why formation of a diketopiperazine elimination compound and a 6-membered cyclic amidine skeleton compound can be significantly suppressed when the treatment is performed with DBU is that formation of a 6-membered ring which causes diketopiperazine elimination is prevented due to the presence of a carbamic acid salt.

Example 7-3: Examination of presence of compound 1-3-3 when compound 1-3-1 is treated with various bases in THF

Basic operation

Four solutions of compound 1-3-1 (about 10 mg, 0.24 mmol) in tetrahydrofuran-d8 (THF-d8) (0.5 mL) were prepared, and the four bases of piperidine (67.5 μL, Entry 1), 1.1.3.3-tetramethylguanidine (TMG) (13.5 μL, Entry 2), 1,8-diazabicyclo[5.4.0]-7-undecene (DBU) (6.8 μL, Entry 3) and iminotris(dimethylamino)phosphoran (HP1 (dma)) (13.5 μL, Entry 4) were then applied, respectively, to the solutions. After 30 minutes, NMR of each of the solutions was measured, and the ratio of compound 1-3-3a to compound 1-3-2 was determined from the integral ratio of protons in $^1$H-NMR (Table 13). The amount of compound 1-3-1 used in this experiment was 10.0 mg for entry 1, 10.0 mg for entry 2, 10.0 mg for entry 3, and 10.1 mg for entry 4.

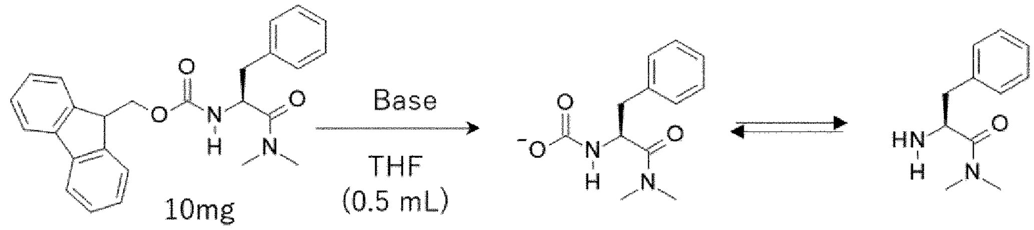


[Table 13]

| entry | Base | pKa* | Equiv | Compound 1-3-3 : Compound 1-3-2 | time/min |
|---|---|---|---|---|---|
| 1 | piperidine | 19.35 | 28.3 (20%) | 0:100 | 30 |
| 2 | TMG | 23. 35 | 4.46 (4%) | 51:49 | 30 |
| 3 | DBU | 24.31 | 1.88 (2%) | 54:46 | 30 |
| 4 | HP1 (dma) | 25.85 | 3.08 (4%) | 87:13 | 30 |

From the above results, it was confirmed that when the Fmoc-protected amino group was treated with a base having a pKa of 23 or more in a conjugate acid, the proportion of the carbamic acid salt present was 50% or more.

For discussing the results of experiments on a solid face on the basis of this finding, without being limited to a specific theory, the reason why it was possible to reduce diketopiperazine elimination or the like in runs 47 and 48 in Example 2 is that formation of a 6-membered ring which causes diketopiperazine elimination or the like was prevented due to the presence of a carbamic acid salt as described above.

Example 7-4: Examination of behavior when acid (HOAt) is added after compound 1-3-1 is treated with DBU

To a solution of compound 1-3-1 (about 25 mg) in a deuterated solvent (1.25 mL, tetrahydrofuran-d8 (THF-d8) [Entry 1], toluene-d8 [Entry 2], dicyclomethane-d2 (DCM-d2) [Entry 3], or N,N-dimethylformamide-d7 (DMF-d7) [Entry 4]), 1,8-diazabicyclo[5.4.0]-7-undecene (DBU) (9.0 μL) was added, and 1.0 mL of the relevant solution was then divided into two parts (0.5 mL). 1-Hydroxybenzotriazole (HOAt) (3.3 mg, with HOAt) was added to one part, and nothing was added to the other part (without HOAt). NMR of each part was measured, and the ratio of compound 1-3-3a to compound 1-3-2 was determined from the integral ratio of protons in $^1$H-NMR (Table 14). The amount of compound 1-3-1 used in this experiment was 24.9 mg for entry 1, 25.3 mg for entry 2, 25.2 mg for entry 3, and 24.9 mg for entry 4.

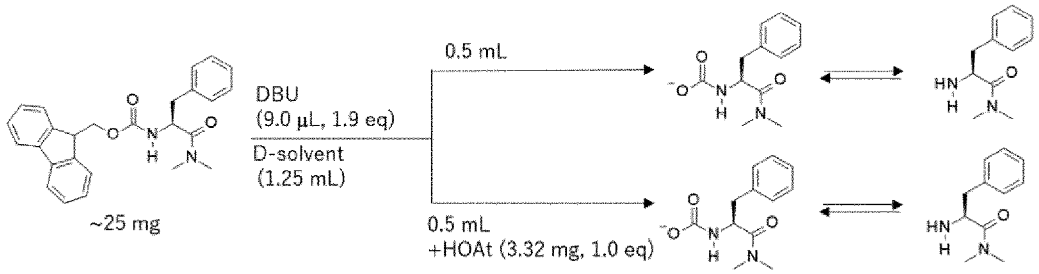


[Table 14]

| entry | solvent | Compound 1-3-3a : Compound 1-3-2 | |
|---|---|---|---|
| | | with HOAt | without HOAt |
| 1 | THF-d8 | 0:100 | 41:59 |
| 2 | Toluene-d8 | 5:95 | 58:42 |
| 3 | DCM-d2 | 2:98 | 63:37 |
| 4 | DMF-d7 | 20:80 | 82:18 |

From the above results, it was confirmed that when HOAt as an acid (pKa in water: 3.28) was added after the Fmoc-protected amino group was treated with DBU, the abundance ratio of the carbamic acid salt significantly decreased.

For discussing the results of experiments on a solid face on the basis of this finding, without being limited to a specific theory, it can be explained without contradiction that a reason why the ratio of a diketopiperazine elimination compound and an amidine form increased when HOAt was added for neutralization in Example 3 is that the abundance ratio of the carbamic acid salt decreased.

Thus, without being limited to a specific theory, diketopiperazine elimination of the like can be reduced due to the presence of a N-terminal amino group as a carbamic acid salt when Fmoc protection is treated with a base. From the results of Example 7-3, the range of bases which can be present as a carbamic acid salt was determined.

Example 7-5: Experiment of peptide synthesis using solvent bubbled with $CO_2$ in Fmoc deprotection step in solid-phase synthesis of peptide

From Examples 7-1 to 7-4, it can be explained that formation of a 6-membered ring which causes diketopiperazine elimination or the like is prevented due to the presence of the carbamic acid salt, resulting in suppression diketopiperazine formation. Thus, an experiment was conducted for examining an effect on formation of a diketopiperazine elimination compound by using a solvent bubbled with $CO_2$ as a carbonate source for stabilization of the carbamic acid salt during Fmoc deprotection reaction.

Condition A (Table 15, run 1, condition without $CO_2$ bubbling)

100 mg of the peptide-supporting solid-phase resin prepared in Example 1-2-5 (compound 1-2-11 prepared in Example 1-2-5 from compound 1-2-3 at 0.567 mmol/g) was put in a solid-phase reaction vessel, DCM (1.0 mL) was added inside a glove box purged with nitrogen, and the mixture was left standing for 10 minutes to swell the resin. Thereafter, the solution was discharged from a frit, and subsequently, the resin was washed twice with DMF (0.7 mL per column). A solution of 1,8-diazabicyclo[5.4.0]-7-undecene (DBU) in DMF (2 v/v%, 0.7 mL per column) was added, and the mixture was reacted at room temperature for 10 minutes to perform deprotection of an Fmoc group. The solution was discharged from the frit, and the resin was then washed six times with DMF (0.7 mL per column).

To the resin obtained as described above, a solution obtained by mixing a solution of Fmoc-MeIle-OH (0.227 mmol, 4 equivalents) in DMF (0.25 mL), a solution of [ethylcyano(hydroxyimino)acetato-O2]tri 1-pyrrolidinylphosphonium hexafluorophosphate (PyOxim) (0.227 mmol, 4 equivalents) in DMF (0.25 mL), and DIPEA (0.056 mL, 0.340 mmol, 6 equivalents) and leaving the mixture to stand for about 1 to 2 minutes was added, and the mixture was shaken at room temperature for 2 hours. The solution was discharged from the frit, and the resin was then washed four times with DMF (0.7 mL per column) and four times with DCM (0.7 mL per column), and dried.

Condition B (Table 15, run 2, condition with solvent bubbled with $CO_2$)

100 mg of the peptide-supporting solid-phase resin prepared in Example 1-2-5 (compound 1-2-11 prepared in Example 1-2-5 from compound 1-2-3 at 0.567 mmol/g) was put in a solid-phase reaction vessel, DCM (1.0 mL) was added inside a glove box purged with nitrogen, and the mixture was left standing for 10 minutes to swell the resin. Thereafter, the solution was discharged from a frit, and subsequently, the resin was washed twice with DMF (0.7 mL per column). A solution of 1,8-diazabicyclo[5.4.0]-7-undecene (DBU) in $CO_2$-DMF (2 v/v%, 0.7 mL per column) was added, and the mixture was reacted at room temperature for 10 minutes to perform deprotection of an Fmoc group. The $CO_2$-DMF solvent was prepared by bubbling 50 mL of DMF in a 100 mL glass bottle with $CO_2$ gas for 5 minutes. The solution was discharged from the frit, and the resin was then washed six times with DMF (0.7 mL per column).

To the resin obtained as described above, a solution obtained by mixing a solution of Fmoc-MeIle-OH (0.227 mmol, 4 equivalents) in DMF (0.25 mL), a solution of [ethylcyano(hydroxyimino)acetato-O2)tri 1-pyrrolidinylphosphonium hexafluorophosphate (PyOxim) (0.227 mmol, 4 equivalents) in DMF (0.25 mL), and DIPEA (0.056 mL, 0.340 mmol, 6 equivalents) and leaving the mixture to stand for about 1 to 2 minutes was added, and the mixture was shaken at room temperature for 2 hours. The solution was discharged from the frit, and the resin was then washed four times with DMF (0.7 mL per column) and four times with DCM (0.7 mL per column), and dried.

For checking the progress of reaction, a part of each of the obtained resins was taken out, and swollen with DCM, and a peptide was then isolated with a TFE/DCM solution (1/1 (v/v)) containing DIPEA (0.045 mol/L). The isolating solution was analyzed by LCMS (SQDFA05 long) to confirm formation of a target peptide (TM) (compound 4-1-6*), a diketopiperazine elimination compound (compound 4-2-6*) and a MeIle excessive elongation compound (compound 7-5-1*).

MeIle excessive elongation compound, Fmoc-MeIle-MeIle-Aib-Pro-D-3-Abu-OH (compound 7-5-1*)

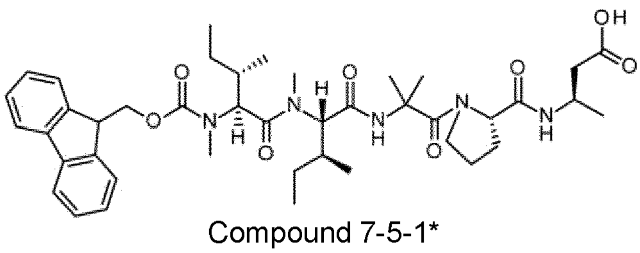

Compound 7-5-1*

LCMS (ESI) m/z = 538.7 (M-H)-
Detected as MS of fragment freed of Fmoc protection ((M-H-Fmoc)-)
Retention time: 2.63 minutes (analysis condition SQDA05 long)

Table 15 below shows the obtained results (the relative ratios of UV areas in LCMS for compound 4-1-6*, compound 4-2-6* and compound 7-5-1* under respective conditions are expressed as a percentage).

[Table 15]

| run | Reaction condition | | | | | Area % | | |
|---|---|---|---|---|---|---|---|---|
| | De-Fmoc | | Washing | Elongation | | | | |
| | Base | Solvent | Solvent | Condensation agent | Solvent | TM-DKP (Compound 4-2-6*) | TM(Compound 4-1-6*) | MelleMelle excessive elongation (Compound 7-5-1*) |
| 1 | 2% DBU | DMF | DMF × 6 | PyOxim/ DIPEA | DMF | 5.96 | 93.23 | 0.81 |
| 2 | 2% DBU | $CO_2$-DMF | DMF × 6 | PyOxim/ DIPEA | DMF | 2.14 | 96.60 | 1.26 |

Thus, from the results of Example 7-5, it was confirmed that when a solvent bubbled with $CO_2$ during Fmoc deprotection reaction was used (run 2), it was possible to suppress formation of a diketopiperazine elimination compound in comparison with a case where $CO_2$ bubbling was not performed (run 1).

[Industrial Applicability]

It has been found that according to the present invention, formation of side products such as a diketopiperazine and a 6-membered cyclic amidine skeleton compound can be suppressed to efficiently elongate a peptide chain in production of a peptide using a solid-phase method. The present invention is useful in the field of peptide synthesis by a solid-phase method.

[Sequence Listing]

C 1-A2025Psq.txt

## Claims

1. A method for producing a peptide by a solid-phase method, comprising the steps of:

   (1) providing a first peptide having a protective group containing an Fmoc skeleton and supported on a solid-phase synthesis resin;
   (2) treating the first peptide with one or more bases including at least a base having a pKa of 23 or more in acetonitrile as a conjugate acid in a solvent containing at least one selected from the group consisting of an aromatic hydrocarbon solvent, a halogen solvent, an ether solvent, an ester solvent, a ketone solvent, a carbonate solvent and a phosphoric acid ester solvent after the step (1); and
   (3) condensing the first peptide with a carboxylic acid or a carboxylic acid analog in a solvent in the presence or absence of a condensation agent to obtain a third peptide after the step (2).

2. The method according to claim 1, which does not comprise the step of treating the first peptide with piperidine as a single base before the step (2).

3. The method according to any one claim 1 or 2, which does not comprise the step of neutralizing the residual base by adding an acid between the step (2) and the step (3).

4. The method according to any one of claims 1 to 3, wherein at least a part of the first peptide obtained from the step (2) is in the form of a carbamic acid salt.

5. The method according to any one of claims 1 to 4, wherein the solvent in the step (2) contains at least one selected from the group consisting of an aromatic hydrocarbon solvent, a halogen solvent, an ether solvent, an ester solvent, a ketone solvent, a carbonate solvent and a phosphoric acid ester solvent at 25 v/v% or more.

6. The method according to any one of claims 1 to 5, wherein the aromatic hydrocarbon solvent is one or more selected from the group consisting of toluene, benzene, xylene, chlorobenzene, 1,2-dichlorobenzene, bromobenzene, anisole, ethylbenzene, nitrobenzene and cumene,

   the halogen solvent is one or more selected from the group consisting of dichloromethane, chloroform, 1,2-dichloroethane and carbon tetrachloride,
   the ether solvent is one or more selected from the group consisting of tetrahydrofuran, diethyl ether, 2-methyltetrahydrofuran, 1,4-dioxane, 1,2-dimethoxyethane, 1,3-dioxolane, diisopropyl ether, cyclopentyl methyl ether, t-butyl methyl ether, 4-methyltetrahydropyran, diglyme, triglyme and tetraglyme,
   the ester solvent is one or more selected from the group consisting of methyl acetate, ethyl acetate, butyl acetate, methyl propionate, propyl acetate, isopropyl acetate, isobutyl acetate, pentyl acetate and $\gamma$-valerolactone,
   the ketone solvent is one or more selected from the group consisting of acetone, methyl ethyl ketone, methyl isobutyl ketone, cyclohexanone, cyclopentanone and diethyl ketone,
   the carbonate solvent is one or more selected from the group consisting of dimethyl carbonate, diethyl carbonate and dibutyl carbonate, and
   the phosphoric acid ester solvent is one or more selected from the group consisting of trimethyl phosphate,

triethyl phosphate and tributyl phosphate.

7. The method according to any one of claims 1 to 6, wherein the solvent in the step (2) has a donor number value of 26 or less.

8. The method according to any one of claims 1 to 7, wherein the base in the step (2) is at least one selected from the group consisting of an amidine, a guanidine and a phosphazene.

9. The method according to any one of claims 1 to 8, wherein the base in the step (2) is at least one selected from the group consisting of DBU, MTBD, TMG, P1tBu, P2Et and HP1 (dma).

10. The method according to any one of claims 1 to 9, wherein the step (2) further comprises the step of bringing the solvent into contact with $CO_2$.

11. The method according to any one of claims 1 to 10, wherein the carboxylic acid or carboxylic acid analog is an amino acid having a protective group, a second peptide having a protective group, a $C_1$-$C_8$ alkylcarboxylic acid, or a $C_6$-$C_{10}$ arylcarboxylic acid; or an active ester of an amino acid having a protective group, a second peptide having a protective group, a $C_1$-$C_8$ alkylcarboxylic acid, or a $C_6$-$C_{10}$ arylcarboxylic acid; or an acid halide of an amino acid having a protective group, a second peptide having a protective group, a $C_1$-$C_8$ alkylcarboxylic acid, or a $C_6$-$C_{10}$ arylcarboxylic acid; wherein the $C_1$-$C_8$ alkylcarboxylic acid and the $C_6$-$C_{10}$ arylcarboxylic acid are optionally substituted with one or more substituents independently selected from the group consisting of alkenyl, alkynyl, alkoxy, cycloalkyl, aryl, heteroaryl, heterocyclyl, arylalkyl, heteroarylalkyl, halogen, nitro, dialkylamino, cyano, alkoxycarbonyl and dialkylaminocarbonyl.

12. The method according to any one of claims 1 to 11, wherein the first peptide is a dipeptide.

13. The method according to any one of claims 1 to 12, wherein the carboxylic acid or carboxylic acid analog is an amino acid or a second peptide having a protective group containing an Fmoc skeleton; or an active ester of an amino acid or a second peptide having a protective group; or an acid halide of an amino acid or a second peptide having a protective group; wherein the first peptide and/or the second peptide having a protective group containing an Fmoc skeleton contains one or more N-substituted amino acids, and/or the amino acid having a protective group containing an Fmoc skeleton is a N-substituted amino acid.

14. The method according to any one of claims 1 to 13, wherein the amino acid at the second residue from the N-terminal of the first peptide is a N-substituted amino acid.

15. The method according to any one of claims 1 to 14, wherein the condensation agent in the step (3) is in the form of a salt, and the counter anion thereof is $PF_6^-$ or $BF_4^-$.

16. The method according to any one of claims 1 to 15, wherein the condensation agent in the step (3) contains at least one selected from the group consisting of PyOxim, PyAOP, PyBOP, COMU, HATU, HBTU, HCTU, TDBTU, HOTU, TATU, TBTU, TCTU and TOTU.

17. A method for reducing the amount of a diketopiperazine impurity and/or a 6-membered cyclic amidine skeleton compound impurity formed in production of a peptide by a solid-phase method, comprising the steps of:

(1) providing a first peptide having a protective group containing an Fmoc skeleton and supported on a solid-phase; and
(2) treating the first peptide with one or more bases including at least a base having a pKa of 23 or more in acetonitrile as a conjugate acid in a solvent containing at least one selected from the group consisting of an aromatic hydrocarbon solvent, a halogen solvent, an ether solvent, an ester solvent, a ketone solvent, a carbonate solvent and a phosphoric acid ester solvent after the step (1).

*Fig.1*

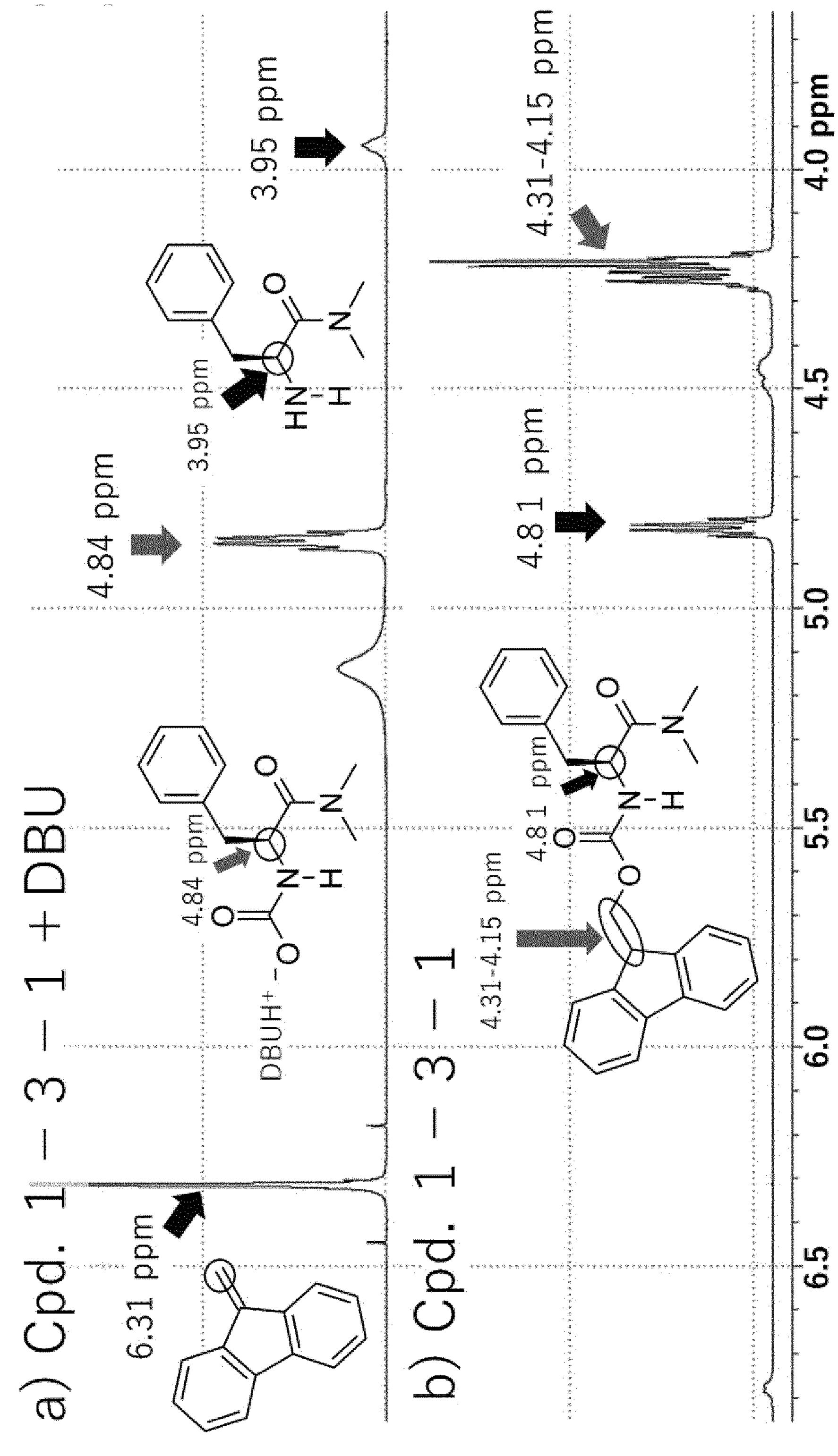
a) Cpd. 1 − 3 − 1 + DBU
6.31 ppm
4.84 ppm
3.95 ppm
b) Cpd. 1 − 3 − 1
4.31-4.15 ppm
4.81 ppm
6.5
6.0
5.5
5.0
4.5
4.0 ppm

# Fig.2

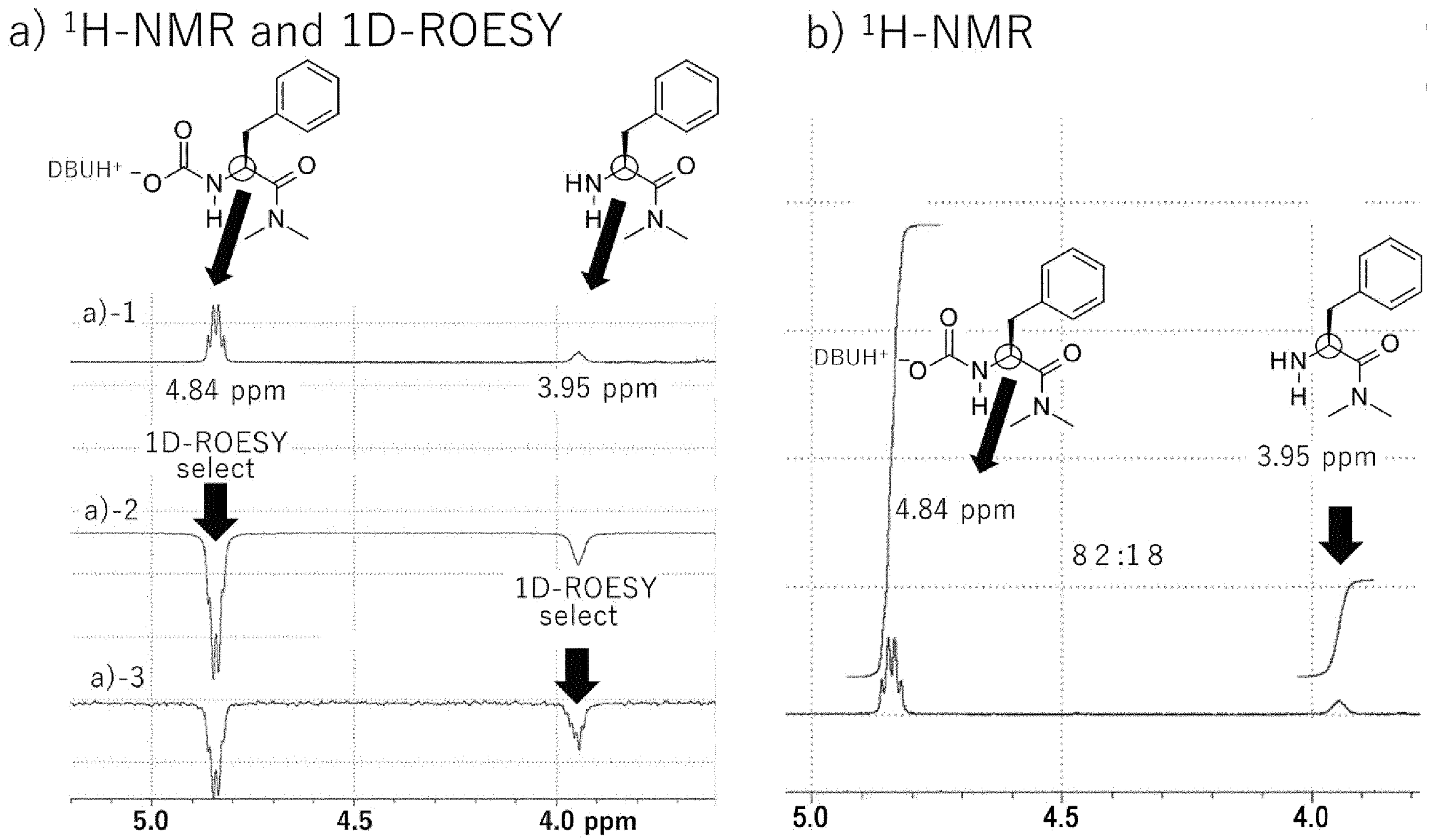
a) $^1$H-NMR and 1D-ROESY
a)-1
4.84 ppm
3.95 ppm
1D-ROESY select
a)-2
1D-ROESY select
a)-3
5.0
4.5
4.0 ppm
b) $^1$H-NMR
4.84 ppm
3.95 ppm
8 2 : 1 8
5.0
4.5
4.0

*Fig.3*

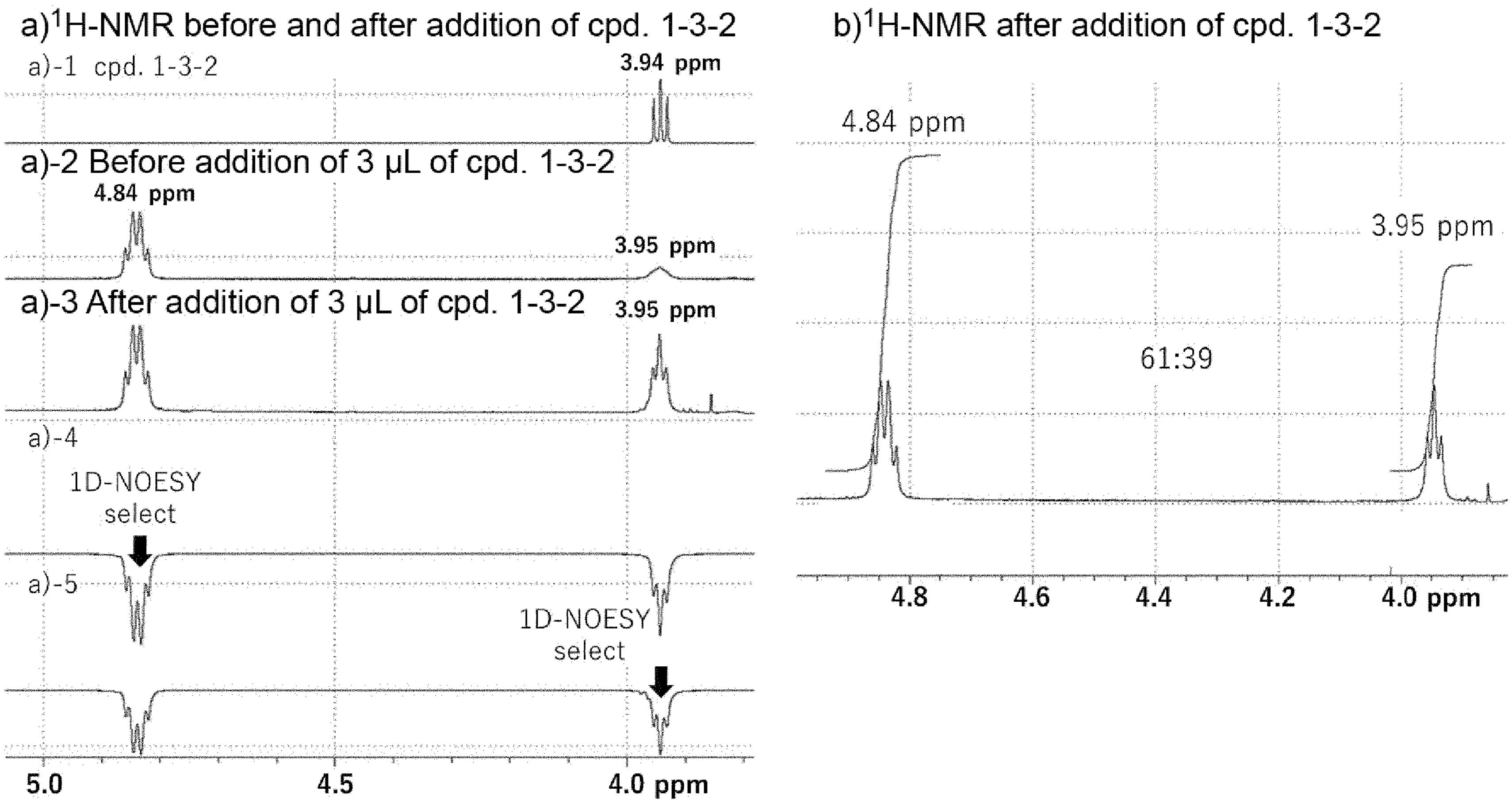
a)¹H-NMR before and after addition of cpd. 1-3-2
a)-1 cpd. 1-3-2
3.94 ppm
a)-2 Before addition of 3 μL of cpd. 1-3-2
4.84 ppm
3.95 ppm
a)-3 After addition of 3 μL of cpd. 1-3-2
3.95 ppm
a)-4
1D-NOESY select
a)-5
1D-NOESY select
5.0
4.5
4.0 ppm
b)¹H-NMR after addition of cpd. 1-3-2
4.84 ppm
3.95 ppm
61:39
4.8
4.6
4.4
4.2
4.0 ppm

*Fig.4*

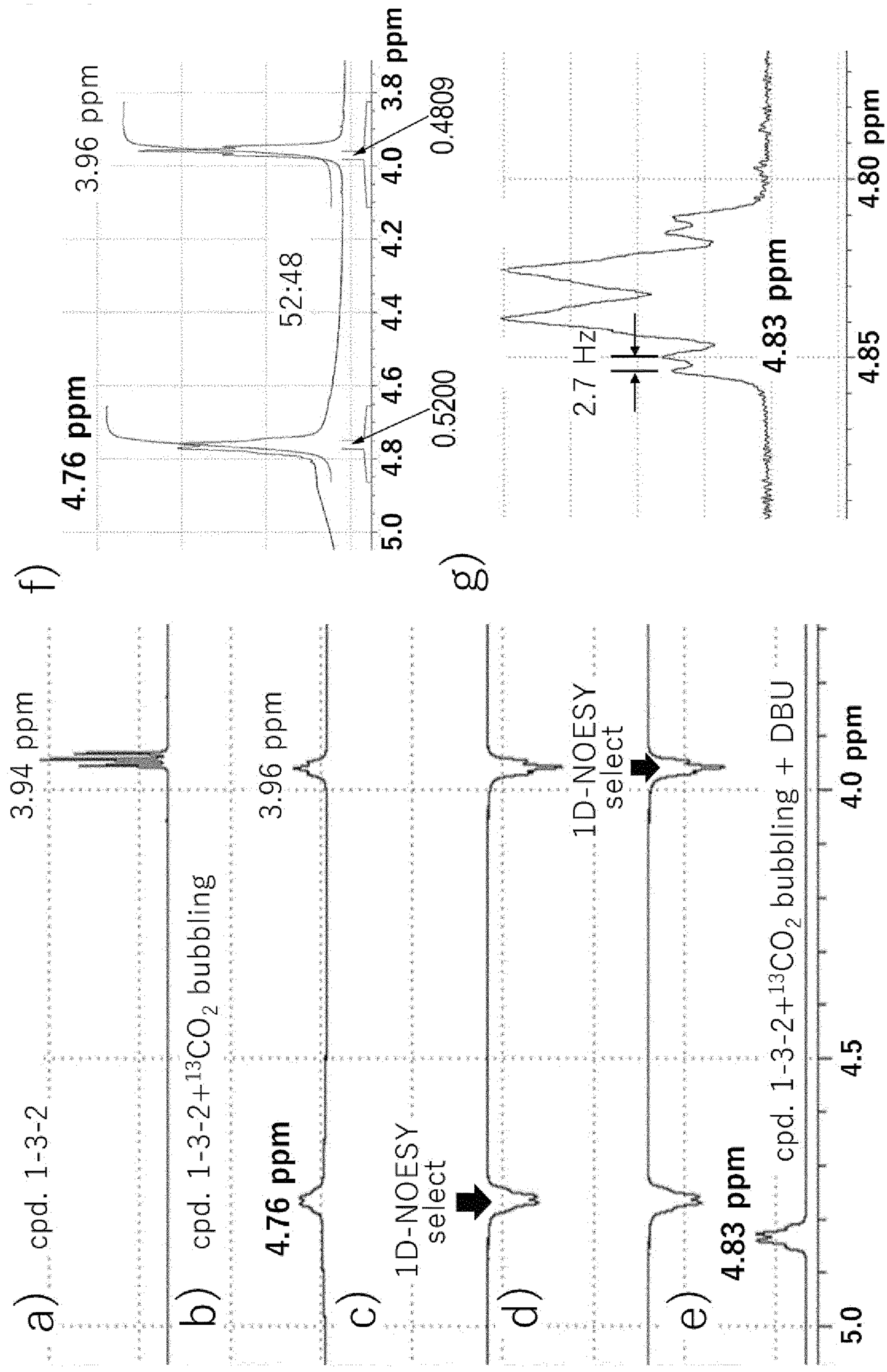
a) cpd. 1-3-2
3.94 ppm
b) cpd. 1-3-2+$^{13}CO_2$ bubbling
4.76 ppm
3.96 ppm
c)
1D-NOESY select
d)
1D-NOESY select
e)
4.83 ppm
cpd. 1-3-2+$^{13}CO_2$ bubbling + DBU
5.0
4.5
4.0 ppm
f)
4.76 ppm
3.96 ppm
52:48
0.5200
0.4809
5.0
4.8
4.6
4.4
4.2
4.0
3.8 ppm
g)
2.7 Hz
4.83 ppm
4.85
4.80 ppm

*Fig.5*

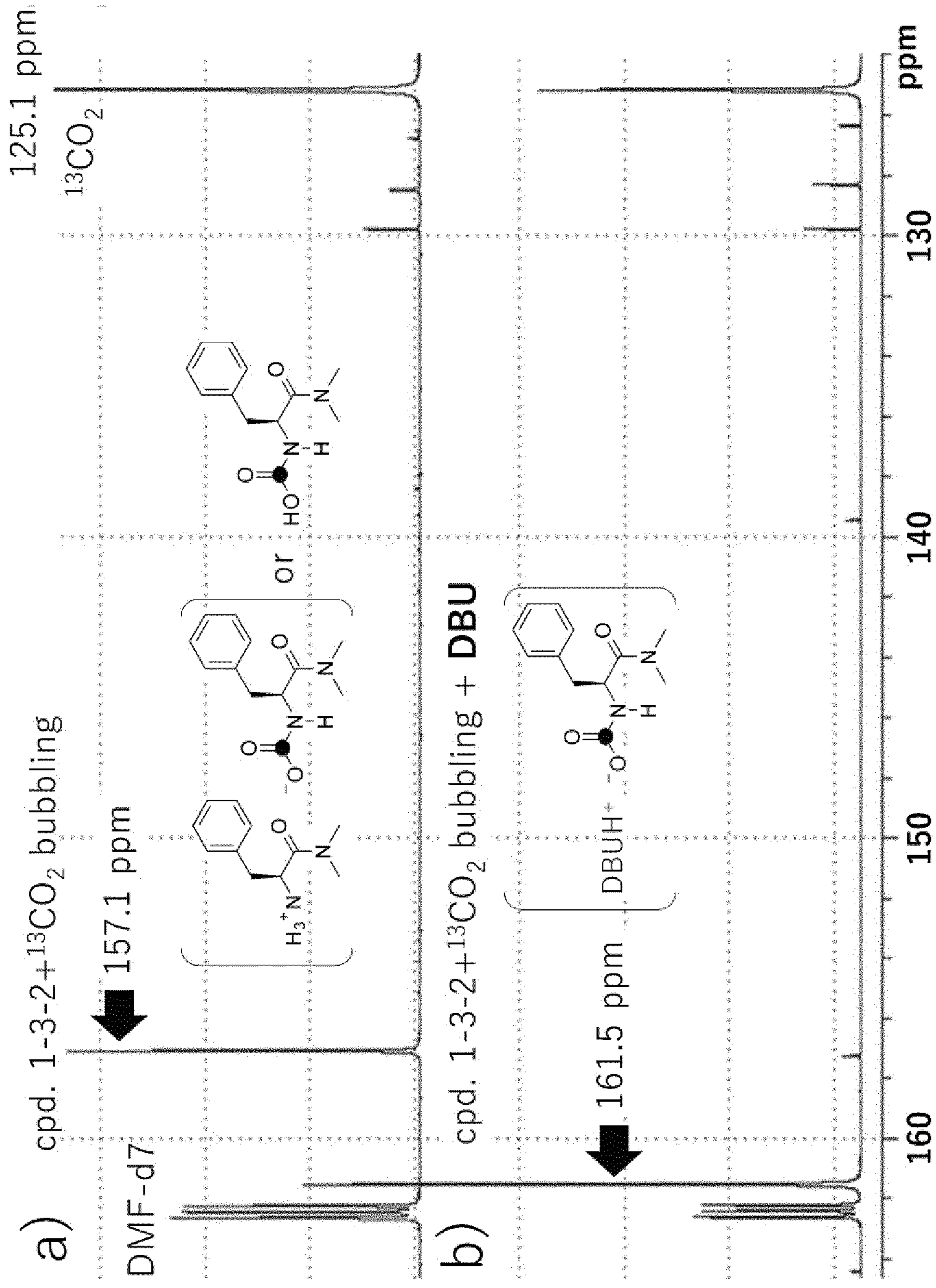
a) DMF-d7
cpd. 1-3-2+$^{13}CO_2$ bubbling
157.1 ppm
125.1 ppm
$^{13}CO_2$
or
b) cpd. 1-3-2+$^{13}CO_2$ bubbling + **DBU**
161.5 ppm
DBUH+
160
150
140
130
ppm

Fig.6

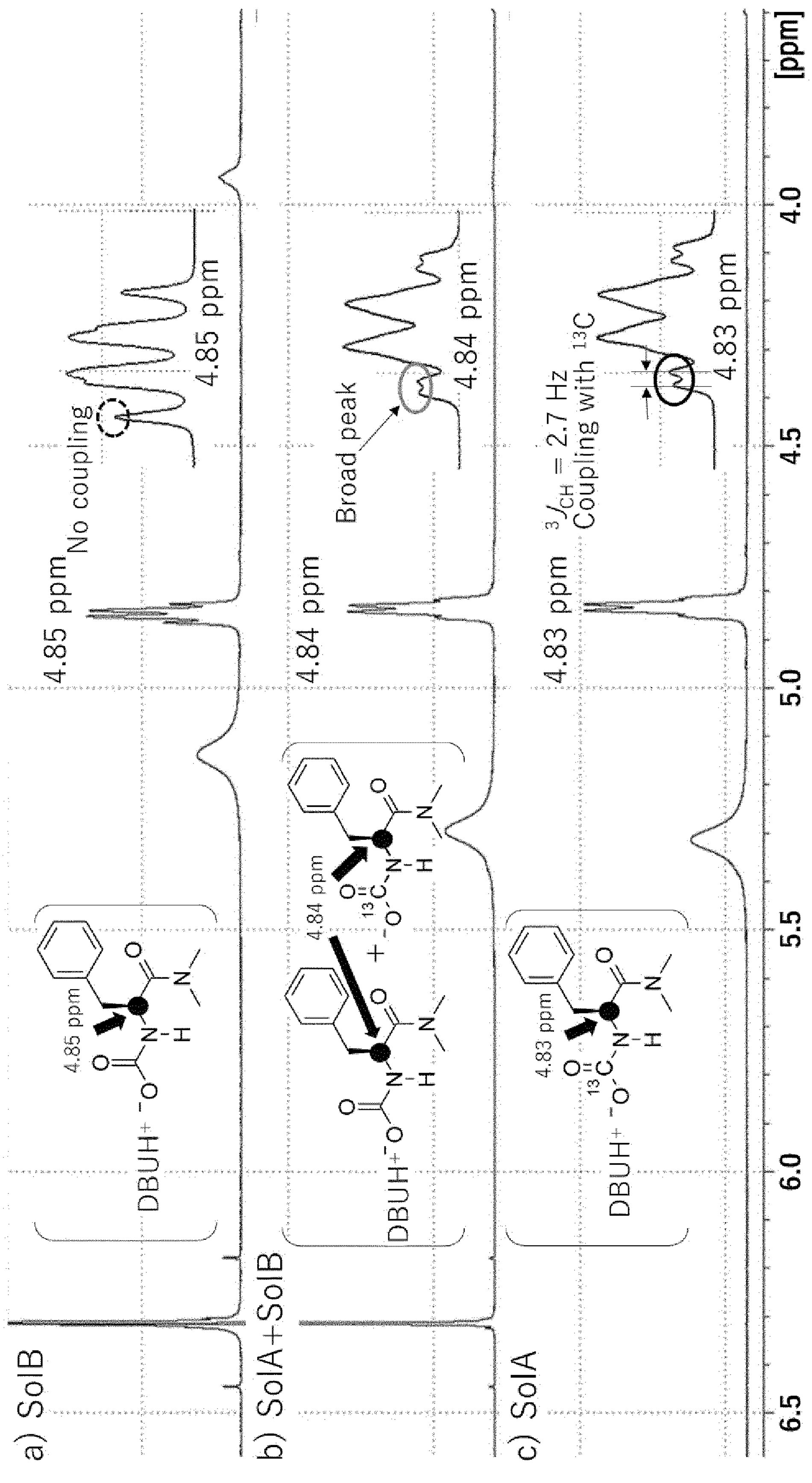
a) SolB
b) SolA+SolB
c) SolA
4.85 ppm
No coupling
4.84 ppm
Broad peak
4.83 ppm
$^3J_{CH}$ = 2.7 Hz
Coupling with $^{13}C$
DBUH+
6.5
6.0
5.5
5.0
4.5
4.0
[ppm]

*Fig.7*

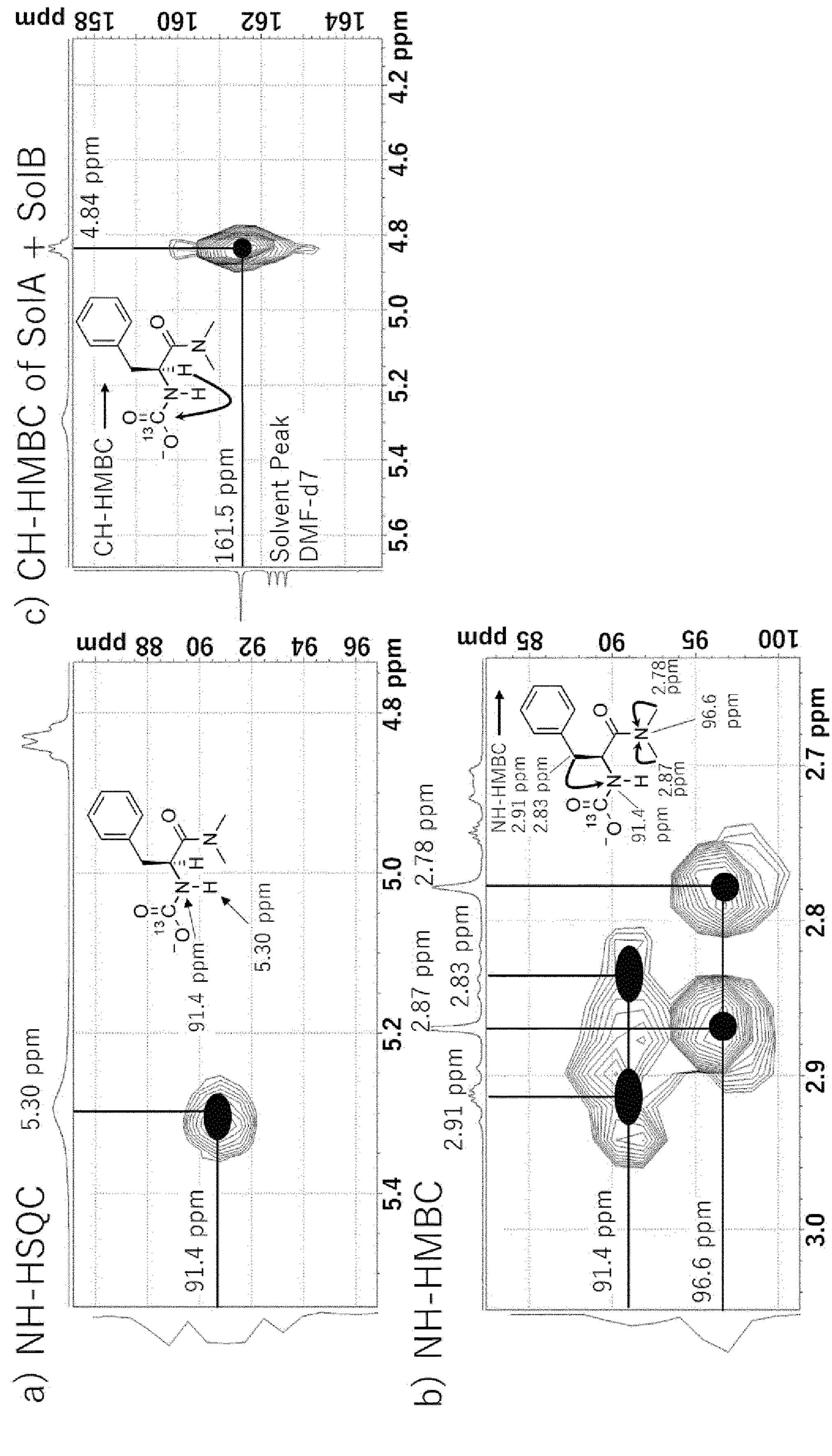
a) NH-HSQC
5.30 ppm
91.4 ppm
5.30 ppm
91.4 ppm
b) NH-HMBC
2.91 ppm
2.87 ppm
2.83 ppm
2.78 ppm
91.4 ppm
96.6 ppm
NH-HMBC
c) CH-HMBC of SolA + SolB
4.84 ppm
161.5 ppm
CH-HMBC
Solvent Peak
DMF-d7
ppm

# *Fig.8*

$^{1}$H NMR: Plain
$^{13}$C NMR: **Bold**
$^{15}$N NMR: *Under line, Italic*

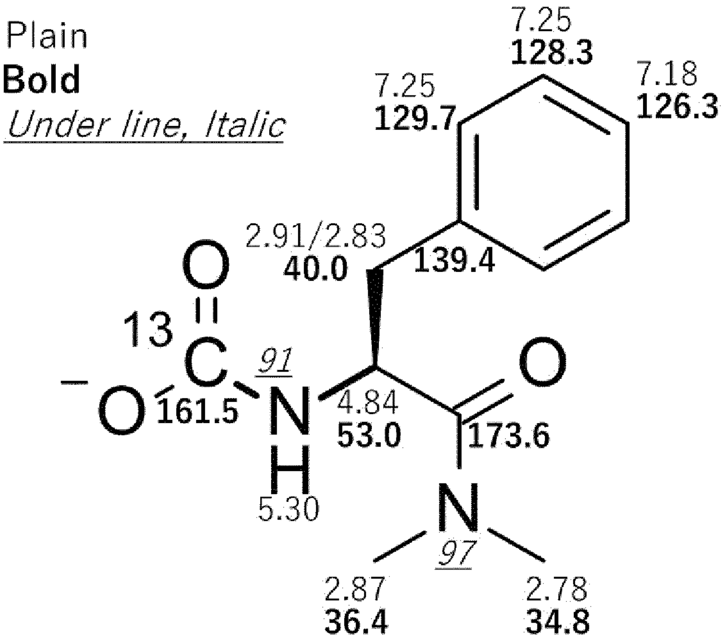

## INTERNATIONAL SEARCH REPORT

International application No.
**PCT/JP2021/039586**

**A. CLASSIFICATION OF SUBJECT MATTER**

***C07K 1/06***(2006.01)i; ***C40B 50/14***(2006.01)i
FI: C07K1/06 ZNA; C40B50/14

According to International Patent Classification (IPC) or to both national classification and IPC

**B. FIELDS SEARCHED**

Minimum documentation searched (classification system followed by classification symbols)

C07K1/06; C40B50/14

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2018-535258 A (IPROTEOS S. L.) 29 November 2018 (2018-11-29) example 12 | 1, 3, 4, 6-14, 17 |
| Y | example 12 | 1-17 |
| Y | WO 2012/165546 A1 (AJINOMOTO CO., INC.) 06 December 2012 (2012-12-06) claims, paragraphs [0072]-[0098], examples | 1-17 |
| Y | WO 2020/175473 A1 (FUJIFILM CORP.) 03 September 2020 (2020-09-03) synthesis examples | 1-17 |
| Y | COIN, Irene et al. Solid-Phase Synthesis of a Cyclodepsipeptide: Cotransin. ORGANIC LETTERS. 2008, vol. 10, no. 17, pages 3857-3860, ISSN: 1523-7060 page 3858, right column, third paragraph | 1-17 |
| P, X | WO 2021/090855 A1 (CHUGAI SEIYAKU K. K.) 14 May 2021 (2021-05-14) paragraphs [0884]-[0889] | 1-17 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 December 2021** | **21 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/039586**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2021/039586**

| Patent document cited in search report | Publication date (day/month/year) | Patent family member(s) | Publication date (day/month/year) |
|---|---|---|---|
| JP 2018-535258 A | 29 November 2018 | US 2018/0319839 A1<br>example 12<br>WO 2017/085034 A1<br>CA 3002892 A1<br>KR 10-2018-0071388 A<br>CN 108290925 A | |
| WO 2012/165546 A1 | 06 December 2012 | JP 2017-36322 A<br>US 2014/0088291 A1<br>claims, paragraphs [0129]-[0156], examples<br>WO 2012/165546 A1<br>EP 2716650 A1 | |
| WO 2020/175473 A1 | 03 September 2020 | (Family: none) | |
| WO 2021/090855 A1 | 14 May 2021 | JP 6880352 B1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018225851 A1
- WO 2013100132 A
- WO 2018225864 A
- JP 2010050089 A
- WO 2021090856 A
- WO 2013100132 A1
- WO 2012165546 A1

### Non-patent literature cited in the description


- *J. Peptide Res.,* 2005, vol. 65, 153-166
- *Org. Lett.,* 2012, vol. 14, 612-615
- Solid Phase Peptide Synthesis. Bachem
- *Org. Lett.,* 2008, vol. 10, 3857-3860
- **GREENE.** Protective Groups in Organic Synthesis. John Wiley & Sons, 2014
- *Eur. Chem. Bull.,* 2015, vol. 4 (2), 92-97
- *Encyclopedic Dictionary of Chemistry,* 1989, 1584-1585
- *Journal of the American Chemical Society,* 1972, vol. 94 (5), 1431-1434
- *Chemistry A European Journal,* 2012, vol. 18 (35), 10969-10982
- *Chemistryselect,* 2021, vol. 6 (4), 600-608
- *Tetrahedron Lett.,* 1998, vol. 39, 8951-8954
- *Org. Process Res Dev.,* 2018, vol. 22, 494-503
- *RSC Adv.,* 2020, vol. 10 (4), 2457-42492
- *Green Chem.,* 2017, vol. 19, 952-962
- *Green Chem.,* 2020, vol. 22, 996-1018
- *New J. Chem.,* 2009, vol. 33, 588
- *Eur. J. Org. Chem.,* 2019, 6735-6748
- *Eur. J. Org. Chem.,* vol. 2019, 6735-6748